# EUROPEAN PATENT APPLICATION

(11) **EP 4 627 943 A1**
(43) Date of publication of application: **08.10.2025**
(21) Application number: 24168583.3
(22) Date of filing: 04.04.2024
(51) Int. Cl.: A24B 15/10, A24B 15/24

(54) **COMPOSITIONS AND METHODS**

(71) Applicant: Nicoventures Trading Limited, London WC2R 3LA (GB)
(72) Inventor: de Souza Cruz, Priscila Brasil, Rio de Janeiro (BR); Kaiser, Samuel, Rio de Janeiro (BR); Flores, Douglas William Menezes, Rio de Janeiro (BR); Assis, Camila, Rio de Janeiro (BR); Nespeca, Maurilio Gustavo, Rio de Janeiro (BR); Porte, Liliane Medianeira Favero, Rio de Janeiro (BR); Jenske, Grace, Rio de Janeiro (BR); da Silva Pinheiro, Giovana, Rio de Janeiro (BR); de Azevedo, Julia Lilian Gauderth, Rio de Janeiro (BR); Bokowski, Liane Valadao Vieira, Rio de Janeiro (BR)
(74) Representative: Dehns

(57) **Abstract**

The present invention relates to a composition which produces a factory-made cigarette (FMC)-like experience and a method of forming the composition. The present invention also relates to a consumable for use in a non-combustible aerosol provision device, the consumable comprising the composition, and to a non-combustible aerosol provision system comprising the consumable and a non-combustible aerosol provision device.

## Description

### Technical Field

The present invention relates to a composition which produces a factory-made cigarette (FMC)-like experience and a method of forming the composition. The present invention also relates to a consumable for use in a non-combustible aerosol provision device, the consumable comprising the composition, and to a non-combustible aerosol provision system comprising the consumable and a non-combustible aerosol provision device.

### Background

Smoking articles such as cigarettes, cigars and the like burn tobacco during use to create tobacco smoke. Alternatives to these types of articles release an inhalable aerosol or vapour by releasing compounds from a substrate material by heating without burning. These may be referred to as non-combustible smoking articles, aerosol generating assemblies or non-combustible aerosol provision systems.

One example of such a product is a heating device which releases compounds by heating, but not burning, a solid aerosolisable material. This solid aerosolisable material may, in some cases, contain a tobacco material. The heating volatilises at least one component of the material, typically forming an inhalable aerosol. These products may be referred to as heat-not-burn devices, tobacco heating devices or tobacco heating products (THP). Various different arrangements for volatilising at least one component of the solid aerosolisable material are known.

As another example, there are e-cigarette / tobacco heating product hybrid devices, also known as electronic tobacco hybrid devices. These hybrid devices contain a liquid source (which may or may not contain nicotine) which is vaporised by heating to produce an inhalable vapour or aerosol. The device additionally contains a solid aerosolisable material (which may or may not contain a tobacco material) and components of this material are entrained in the inhalable vapour or aerosol to produce the inhaled medium.

A further alternative to cigarettes is vapour products which are handheld, battery-powered devices that heat a liquid (called an e-liquid) to produce an inhalable aerosol, commonly known as vapour.

The compositions used in tobacco heated products and vaping platforms can also be used in oral products which are ingested by the consumer.

### Summary

According to an aspect of the present disclosure, there is provided a composition comprising at least three of the following compounds (hereinafter "List 1"): nicotine; 2-hydroxy-4-methyl-2-cyclopenten-1-one; quinic acid; sucrose; N-(1-deoxy-1-fructosyl)proline; 3,4-dimethyl-2(3H)-furanone; lipid peroxidation inhibitor 1; pseudooxynicotine and/or oxynicotine; 2-ethyl-quinoxaline; 1,2,3,4,5,6-hexahydro-7H-cyclopenta[b]pyridin-7-one; 2-ethyl-pyridine; scopoletin; 2-ethyl-5-methyl-pyridine; rutin; retinol; 2-methyl-3-butyl-pyrazine; megastigmatrienone; 3,5-di(1,1-dimethylethyl)-4-hydroxy-benzaldehyde; L-alpha-amino-1H-pyrrole-1-hexanoic acid; and solanesol.

In embodiments, the composition comprises at least 4 of the compounds from List 1. In embodiments, the composition comprises at least 5 of the compounds from List 1. In embodiments, the composition comprises at least 6 of the compounds from List 1. In embodiments, the composition comprises at least 7 of the compounds from List 1. In embodiments, the composition comprises at least 8 of the compounds from List 1. In embodiments, the composition comprises at least 9 of the compounds from List 1. In embodiments, the composition comprises at least 10 of the compounds from List 1. In embodiments, the composition comprises at least 11 of the compounds from List 1. In embodiments, the composition comprises at least 12 of the compounds from List 1. In embodiments, the composition comprises at least 13 of the compounds from List 1. In embodiments, the composition comprises at least 14 of the compounds from List 1. n embodiments, the composition comprises at least 15 of the compounds from List 1. In embodiments, the composition comprises at least 16 of the compounds from List 1. In embodiments, the composition comprises at least 17 of the compounds from List 1. In embodiments, the composition comprises at least 18 of the compounds from List 1. In embodiments, the composition comprises at least 19 of the compounds from List 1. In embodiments, the composition comprises all of the compounds from List 1.

In embodiments, the composition comprises at least nicotine.

In embodiments, the composition comprises at least nicotine; 2-hydroxy-4-methyl-2-cyclopenten-1-one; and quinic acid.

In embodiments, the composition comprises at least 3,4-dimethyl-2(3H)-furanone; pseudooxynicotine and/or oxynicotine; 2-methyl-3-butyl-pyrazine; and megastigmatrienone.

In embodiments, the composition further comprises one or more of the following compounds (hereinafter "List 2"): nicotine; pseudooxynicotine and/or oxynicotine; lipid peroxidation inhibitor 1; scopoletin; N-(1-deoxy-1-fructosyl)proline; sucrose; Type I sucrose ester - GV - I0; Type I sucrose ester - GIV - I0; chlorogenic acid; 1,2,3,4,5,6-Hexahydro-7H-cyclopenta[b]pyridin-7-one; L-alpha-amino-1H-pyrrole-1-hexanoic acid; retinol; quinic acid; Type I sucrose ester - Gill - I0; rutin; megastigmatrienone; 2-ethyl-5-methyl-pyridine; 16-hydroxy-hexadecanoicacid; Type III sucrose ester - GV - I0; and ferulic acid.

In embodiments, the composition further comprises one or more of the following compounds (hereinafter "List 3"): 2-methoxy-phenol; p-cresol; furaneol; indole; 3-methyl-pentanoic acid; butanoic acid; 3-methyl-butanoic acid; D-limonene; acetophenone; 1-(4-methylphenyl)-ethanone; vanillin; 2,6-dimethoxy-phenol; benzaldehyde; benzeneacetic acid; 2-methyl-butanoic acid; pentanoic acid; hexanoic acid; acetic acid; 2-methyl-propanoic acid; and octanoic acid.

In embodiments, the composition further comprises one or more of the following compounds (hereinafter "List 4"): 3-ethyl-2-hydroxy-2-cyclopenten-1-one; 5-methyl-2-furancarboxaldehyde; propanoic acid; 3-methyl-pyridine; butanoic acid; 3-ethyl-pyridine; trimethyl-pyrazine; benzaldehyde; tiglic acid; 2-hydroxy-3-methyl-2-cyclopenten-1-one; acetophenone; 2-ethyl-phenol; 2-methoxy-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; octanoic acid; 2,6-dimethoxy-phenol; 2-methyl-propanoic acid; 3-methyl-butanoic acid; D-limonene; 2-acetyl-5-methylfuran; 1-(4-methylphenyl)-ethanone; vanillin; pentanoic acid; benzoic acid; triacetin; benzeneacetic acid; maltol; and nicotine.

In embodiments, the composition comprises at least 3 compounds from List 1, at least 3 compounds from List 2, at least 3 compounds from List 3 and at least 3 compounds from List 4. In embodiments, the composition comprises at least 5 compounds from List 1, at least 5 compounds from List 2, at least 5 compounds from List 3 and at least 5 compounds from List 4. In embodiments, the composition comprises at least 10 compounds from List 1, at least 10 compounds from List 2, at least 10 compounds from List 3 and at least 10 compounds from List 4.

In embodiments, at least 1 of the compounds included in the composition from List 1 is a purified compound. In embodiments, at least 3 of the compounds included in the composition from List 1 are purified compounds. In embodiments, at least about 25%, at least about 50% or at least about 75% of the compounds included in the composition from List 1 are purified compounds. In embodiments, all of the compounds included in the composition from List 1 are purified compounds.

In embodiments, if present at least 1 of the compounds included in the composition from List 2 is a purified compound. In embodiments, if present at least 3 of the compounds included in the composition from List 2 are purified compounds. In embodiments, if present at least about 25%, at least about 50% or at least about 75% of the compounds included in the composition from List 2 are purified compounds. In embodiments, if present all of the compounds included in the composition from List 2 are purified compounds.

In embodiments, if present at least 1 of the compounds included in the composition from List 3 is a purified compound. In embodiments, if present at least 3 of the compounds included in the composition from List 3 are purified compounds. In embodiments, if present at least about 25%, at least about 50% or at least about 75% of the compounds included in the composition from List 3 are purified compounds. In embodiments, if present all of the compounds included in the composition from List 3 are purified compounds.

In embodiments, if present at least 1 of the compounds included in the composition from List 4 is a purified compound. In embodiments, if present at least 3 of the compounds included in the composition from List 4 are purified compounds. In embodiments, if present at least about 25%, at least about 50% or at least about 75% of the compounds included in the composition from List 4 are purified compounds. In embodiments, if present all of the compounds included in the composition from List 4 are purified compounds.

In embodiments, the composition is not, or does not contain, a tobacco extract.

In embodiments, the composition is formed from a tobacco extract.

In embodiments the composition or tobacco extract comprises some, but not all, of the compounds listed in List 1.

In embodiments, the composition comprises a tobacco extract. In this case, one or more of the key chemical markers described herein may be present in or provided from the extract.

In embodiments, the composition comprises a tobacco extract and at least one further compound, wherein said at least one further compound is one of the key chemical markers described herein. The at least one further compound is not otherwise present in the tobacco extract (i.e. it must be added to the extract).

In embodiments, the composition comprises a tobacco extract and at least 1 further compound from List 1. In embodiments, the composition comprises a tobacco extract and at least 2 further compounds from List 1. In embodiments, the composition comprises a tobacco extract and at least 3 further compounds from List 1. In embodiments, the composition comprises a tobacco extract and at least 4 further compounds from List 1. In embodiments, the composition comprises a tobacco extract and at least 5 further compounds from List 1. In embodiments, the composition comprises a tobacco extract and at least 6 further compounds from List 1. In embodiments, the composition comprises a tobacco extract and at least 7 further compounds from List 1. In embodiments, the composition comprises a tobacco extract and at least 8 further compounds from List 1. In embodiments, the composition comprises a tobacco extract and at least 9 further compounds from List 1. In embodiments, the composition comprises a tobacco extract and at least 10 further compounds from List 1. In embodiments, the composition comprises a tobacco extract and at least 11 further compounds from List 1. In embodiments, the composition comprises a tobacco extract and at least 12 further compounds from List 1. In embodiments, the composition comprises a tobacco extract and at least 13 further compounds from List 1. In embodiments, the composition comprises a tobacco extract and at least 14 further compounds from List 1. In embodiments, the composition comprises a tobacco extract and at least 15 further compounds from List 1. In embodiments, the composition comprises a tobacco extract and at least 16 further compounds from List 1. In embodiments, the composition comprises a tobacco extract and at least 17 further compounds from List 1. In embodiments, the composition comprises a tobacco extract and at least 18 further compounds from List 1. In embodiments, the composition comprises a tobacco extract and at least 19 further compounds from List 1. In embodiments, the composition comprises a tobacco extract and, in addition, all of the compounds from List 1.

In embodiments, the composition comprises a tobacco extract and at least 1 (such as at least 2, 3, 4 or 5) further compound(s) from List 2. In embodiments, the composition comprises a tobacco extract and at least 1 (such as at least 2, 3, 4 or 5) further compound(s) from List 3. In embodiments, the composition comprises a tobacco extract and at least 1 (such as at least 2, 3, 4 or 5) further compound(s) from List 4.

In embodiments, the composition comprises a tobacco extract and at least 1 (such as at least 2, 3, 4 or 5) further compound(s) from each of Lists 1 and 2. In embodiments, the composition comprises a tobacco extract and at least 1 (such as at least 2, 3, 4 or 5) further compound(s) from each of Lists 1 and 3. In embodiments, the composition comprises a tobacco extract and at least 1 (such as at least 2, 3, 4 or 5) further compound(s) from each of Lists 1 and 4. In embodiments, the composition comprises a tobacco extract and at least 1 (such as at least 2, 3, 4 or 5) further compound(s) from each of Lists 2 and 3. In embodiments, the composition comprises a tobacco extract and at least 1 (such as at least 2, 3, 4 or 5) further compound(s) from each of Lists 2 and 4. In embodiments, the composition comprises a tobacco extract and at least 1 (such as at least 2, 3, 4 or 5) further compound(s) from each of Lists 3 and 4.

In embodiments, the composition comprises a tobacco extract and at least 1 (such as at least 2, 3, 4 or 5) further compound(s) from each of Lists 1, 2 and 3. In embodiments, the composition comprises a tobacco extract and at least 1 (such as at least 2, 3, 4 or 5) further compound(s) from each of Lists 1, 2 and 4. In embodiments, the composition comprises a tobacco extract and at least 1 (such as at least 2, 3, 4 or 5) further compound(s) from each of Lists 1, 3 and 4. In embodiments, the composition comprises a tobacco extract and at least 1 (such as at least 2, 3, 4 or 5) further compound(s) from each of Lists 2, 3 and 4.

In embodiments, the composition comprises a tobacco extract and at least 1 (such as at least 2, 3, 4 or 5) further compound(s) from each of Lists 1, 2, 3 and 4.

In each case, said further compound(s) may be isolated or purified compounds.

In embodiments, the composition is a tobacco extract.

In embodiments, the composition comprises a total of from about 0.00001 to about 5 wt% of the compounds listed in Lists 1, 2 and 3. In embodiments, the composition comprises a total of from about 0.0001 to about 2 wt% of the compounds listed in Lists 1, 2 and 3. In embodiments, the composition comprises a total of from about 0.001 to about 1 wt% of the compounds listed in Lists 1, 2 and 3. In embodiments, the composition comprises a total of from about 0.005 to about 0.5 wt% of the compounds listed in Lists 1, 2 and 3.

In embodiments, the composition comprises a total of from about 0.00001 to about 5 wt% of the compounds listed in Lists 1, 2, 3 and 4. In embodiments, the composition comprises a total of from about 0.0001 to about 2 wt% of the compounds listed in Lists 1, 2, 3 and 4. In embodiments, the composition comprises a total of from about 0.001 to about 1 wt% of the compounds listed in Lists 1, 2, 3 and 4. In embodiments, the composition comprises a total of from about 0.005 to about 0.5 wt% of the compounds listed in Lists 1, 2, 3 and 4.

In embodiments, the composition is a solid, semi-solid (e.g. gel) or liquid. In embodiments the composition is a solid. In embodiments the composition is a gel. In embodiments the composition is a liquid. In embodiments, the composition is not an aerosol. In embodiments, the composition is not a gaseous composition.

In embodiments, the composition is an aerosol-generating composition. The aerosol-generating composition may be a solid, semi-solid (e.g. gel) or liquid. In embodiments the composition is a solid. In embodiments the aerosol-generating composition is a gel. In embodiments the aerosol-generating composition is a liquid.

In embodiments, the composition is a composition for use in a non-combustible aerosol provision system.

In embodiments, the composition is a composition for use in an aerosol-free delivery system.

In embodiments, the composition is a composition for oral delivery of the compounds contained therein.

In embodiments, the composition (e.g. the aerosol-generating composition) further comprises an aerosol-former material, such as glycerol and/or propylene glycol.

In embodiments the total number of chemical compounds present in the composition is less than about 500. In embodiments the total number of chemical compounds present in the composition is less than about 300. In embodiments the total number of chemical compounds present in the composition is less than about 200. In embodiments the total number of chemical compounds present in the composition is less than about 150. In embodiments the total number of chemical compounds present in the composition is less than about 100. In embodiments the total number of chemical compounds present in the composition is less than about 80. In embodiments the total number of chemical compounds present in the composition is less than about 60.

In embodiments the total TSNA content of the compositions or materials described herein is less than about 800 ppb, less than about 500 ppb, less than about 200 ppb, less than about 100 ppb, less than about 50 ppb, less than about 20 ppb, or less than about 10 ppb. In embodiments there are no TSNAs in the compositions or materials described herein.

According to another aspect of the present disclosure, there is provided a consumable for use in a non-combustible aerosol provision device, the consumable comprising a composition as described herein.

According to another aspect of the present disclosure, there is provided a non-combustible aerosol provision system comprising a composition or consumable as described herein and a non-combustible aerosol provision device, the non-combustible aerosol provision device comprising an aerosol-generation device arranged to generate aerosol from the composition or consumable when the composition or consumable is used with the non-combustible aerosol provision device.

The aerosol-generation device may be a tobacco heating device, also known as a heat-not-burn device.

The aerosol-generation device may be a vaping device or e-cigarette.

According to another aspect of the present disclosure, there is provided an aerosol-free delivery system comprising a composition or consumable as described herein. Said aerosol-free delivery system may be configured to deliver the composition or consumable to a user orally, nasally, transdermally or in another way without forming an aerosol, including but not limited to, lozenges, gums, patches, articles comprising inhalable powders, and oral products such as oral tobacco which includes snus or moist snuff.

According to another aspect of the present disclosure, there is provided an aerosol formed from a non-combustible aerosol provision device, wherein the aerosol comprises the composition described herein, e.g. at least three of the following compounds: nicotine; 2-hydroxy-4-methyl-2-cyclopenten-1-one; quinic acid; sucrose; N-(1-deoxy-1-fructosyl)proline; 3,4-dimethyl-2(3H)-furanone; lipid peroxidation inhibitor 1; pseudooxynicotine and/or oxynicotine; 2-ethyl-quinoxaline; 1,2,3,4,5,6-hexahydro-7H-cyclopenta[b]pyridin-7-one; 2-ethyl-pyridine; scopoletin; 2-ethyl-5-methyl-pyridine; rutin; retinol; 2-methyl-3-butyl-pyrazine; megastigmatrienone; 3,5-di(1,1-dimethylethyl)-4-hydroxy-benzaldehyde; L-alpha-amino-1H-pyrrole-1-hexanoic acid; and solanesol.

According to another aspect of the present disclosure, there is provided a method of forming a composition as described herein, comprising mixing the compounds together. The compounds may first be isolated or purified.

According to another aspect of the present disclosure, there is provided a method of forming a composition as described herein, comprising mixing a tobacco extract with one or more of the key chemical markers described herein. The one or more chemical markers added may first be isolated or purified.

According to another aspect of the present disclosure, there is provided a method of forming a composition as described herein, comprising forming a tobacco extract, and optionally adding one or more of the key chemical markers (e.g. key sense markers) described herein. The one or more chemical markers added may first be isolated or purified.

In embodiments, there is provided a method of forming a composition as described herein, wherein the method comprises
(a) forming a slurry comprising a solvent, the compounds (i.e. the key chemical markers), and any other components of the composition or precursors thereof;
(b) forming a layer of the slurry; and
(c) drying the slurry to form the composition.

In embodiments, there is provided a method of forming a composition as described herein, wherein the method comprises
(a) forming a slurry comprising a solvent, a tobacco extract, any other additional key chemical markers, and any other components of the composition or precursors thereof;
(b) forming a layer of the slurry; and
(c) drying the slurry to form the composition.

In other embodiments, one or more of the key chemical markers or additional key chemical markers are applied to the slurry during and/or after steps (b) and/or (c). For example, chemical markers (which may first be isolated or purified) may be applied to the slurry before or after drying.

### Brief description of the drawings

Figure 1 is a schematic (exploded) diagram of an electronic aerosol provision system such as an e-cigarette.
Figure 2 is a block diagram of an aerosol generating device, such as a tobacco heated product.
Figure 3 is a perspective view of an oral product.

### Detailed Description

A cigarette (also known as a factory-made cigarette or FMC) gives rise to a cigarette-like or FMC-like experience when smoked by a consumer. During smoking the cigarette will be burnt. The inventors have determined that this cigarette-like or FMC-like experience is made up of a combination of three different "flavour dimensions", namely sense, taste and flavour.

The inventors have identified a range of compounds (also called chemical markers) responsible for these flavour dimensions. For example, some compounds or chemical markers have been found to contribute towards providing the sense of a cigarette, whilst some contribute towards providing the taste of a cigarette and some contribute towards providing the flavour of a cigarette. The compounds were identified by collecting and analysing the smoke produced from burning and smoking cigarettes, as well as using machine learning and human sensory assessment.

In total, the inventors identified 324 chemical markers which are important for providing the different flavour dimensions of a cigarette. Specifically, 259 compounds were identified as chemical markers for the sense, 206 compounds were identified as chemical markers for the taste and 148 compounds were identified as chemical markers for the flavour of a cigarette. Some chemical markers act on more than one dimension. For example, a single compound or chemical marker may contribute both to a cigarette-like sense and taste.

By the "sense" of a cigarette, it is meant the overall feel of smoking a cigarette, which includes, for example, impact, irritation, harshness, mouth drying, and mouth coating. The "taste" of a cigarette includes known taste attributes, such as hay-like, bread-like, nutty, bitter, smoky, tannin, resinous, musk, etc. The "flavour" of a cigarette includes known flavour attributes, such as creamy, fruity, floral, green, spicy, savoury, roasted, woody, etc.

During their investigations, the inventors also identified all of the compounds which are present in the smoke produced by burning different cigarettes containing types of tobacco. The inventors also formed tobacco extracts from the same tobacco, and identified all of the compounds which were present in these extracts. They found some compounds were present in the tobacco smoke, but not in the tobacco extracts. These compounds are also considered to be chemical markers responsible for the cigarette-like experience. They are termed the chemical smoke markers (i.e. chemical markers for smoke) herein. Over 100 chemical smoke markers were found for each extract tested (i.e. there were over 100 chemicals present in tobacco smoke but not in a tobacco extract formed from the same tobacco). These chemical markers will contribute towards recreating the feel, taste and/or flavour of smoke produced when burning a cigarette. Such compounds may not be produced when forming a tobacco extract, or when heating (but not burning) the tobacco extract or a composition comprising said extract.

The inventors have now found that by utilising some of these chemical markers, a cigarette-like experience can be created without the need to smoke and burn a cigarette. For example, the inventors have found that the experience produced by when using a tobacco extract (or a composition comprising a tobacco extract) can be made more cigarette-like by adding some of these chemical markers to the extract (or the composition comprising the extract). This is possible even without burning the extract or a composition comprising the extract (e.g. when the extract or composition is used in an aerosol-free delivery system and/or a non-combustible aerosol provision system).

Furthermore, it is possible to create a composition for use in an aerosol-free delivery system (e.g. an oral product which is chewed), or a composition for use in a non-combustible aerosol provision system, where the composition provides a cigarette-like experience despite no cigarette being smoked. In particular, it is possible to create a cigarette-like experience without burning tobacco, the composition of the invention, or any material containing the composition of the invention. This may allow for a more user-friendly and healthier cigarette-like experience to be created.

### Key chemical markers

The present invention provides a specific list of important chemical markers (herein called the "key chemical markers"), which the inventors have found to be the most important compounds in re-creating a cigarette-like or FMC-like experience. This includes the key sense markers, key taste markers and key flavour markers. Also provided is a list of key smoke markers which are those smoke markers which were absent from at least one of the tobacco extracts tested but present in the smoke produced from the same tobacco, and which can be used under current toxicity regulations. These key smoke markers are also useful in re-creating a cigarette-like or FMC-like experience.

In particular, it has been found that by using these compounds or chemical markers, it is possible to formulate a composition which may be used to give a consumer a cigarette-like or FMC-like experience, but without the need to burn and smoke a cigarette or FMC. This has particular benefits because many consumers who smoke traditional cigarettes may previously have been reluctant or unwilling to use non-combustible aerosol provision devices (e.g. heat-not-burn, hybrid or vapour devices), or oral compositions, because the experience of using these devices or compositions may differ from the experience of burning and smoking a cigarette.

The compositions and extracts of the present invention may allow for consumers to get the same or similar experience to smoking a cigarette when using oral compositions and/or non-combustible aerosol provision devices. For example, by using the compositions or extracts of the present invention in a non-combustible aerosol provision system, a more cigarette-like experience and/or aerosol can be created compared to using a previously-known or commercially-available composition or extract for use in such devices. For example, the aerosol produced by such a device when using one of the compositions or extracts of the invention may have more chemical markers in common with cigarette smoke than aerosol produced by such a device using a previously-known or commercially-available composition.

The inventors have found that the following compounds (referred to herein as the "key sense markers" or "List 1") are important for creating the sense of a cigarette: nicotine; 2-hydroxy-4-methyl-2-cyclopenten-1-one; quinic acid; sucrose; N-(1-deoxy-1-fructosyl)proline; 3,4-dimethyl-2(3H)-furanone; lipid peroxidation inhibitor 1; pseudooxynicotine and/or oxynicotine; 2-ethyl-quinoxaline; 1,2,3,4,5,6-hexahydro-7H-cyclopenta[b]pyridin-7-one; 2-ethyl-pyridine; scopoletin; 2-ethyl-5-methyl-pyridine; rutin; retinol; 2-methyl-3-butyl-pyrazine; megastigmatrienone; 3,5-di(1,1-dimethylethyl)-4-hydroxy-benzaldehyde; L-alpha-amino-1H-pyrrole-1-hexanoic acid; and solanesol.

The inventors have found that the following compounds (referred to herein as the "key taste markers" or "List 2") are important for creating the taste of a cigarette: nicotine; pseudooxynicotine and/or oxynicotine; lipid peroxidation inhibitor 1; scopoletin; N-(1-deoxy-1-fructosyl)proline; sucrose; Type I sucrose ester - GV - I0; Type I sucrose ester - GIV - I0; chlorogenic acid; 1,2,3,4,5,6-Hexahydro-7H-cyclopenta[b]pyridin-7-one; L-alpha-amino-1H-pyrrole-1-hexanoic acid; retinol; quinic acid; Type I sucrose ester - Gill - I0; rutin; megastigmatrienone; 2-ethyl-5-methyl-pyridine; 16-hydroxy-hexadecanoicacid; Type III sucrose ester - GV - I0; and ferulic acid.

The inventors have found that the following compounds (referred to herein as the "key flavour markers" or "List 3") are important for creating the flavour of a cigarette: 2-methoxy-phenol; p-cresol; furaneol; indole; 3-methyl-pentanoic acid; butanoic acid; 3-methyl-butanoic acid; D-limonene; acetophenone; 1-(4-methylphenyl)-ethanone; vanillin; 2,6-dimethoxy-phenol; benzaldehyde; benzeneacetic acid; 2-methyl-butanoic acid; pentanoic acid; hexanoic acid; acetic acid; 2-methyl-propanoic acid; and octanoic acid.

The inventors have found that the following compounds (referred to herein as the "key smoke markers" or "List 4") may be absent from tobacco extracts but present in smoke produced from burning the same tobacco: 3-ethyl-2-hydroxy-2-cyclopenten-1-one; 5-methyl-2-furancarboxaldehyde; propanoic acid; 3-methyl-pyridine; butanoic acid; 3-ethyl-pyridine; trimethyl-pyrazine; benzaldehyde; tiglic acid; 2-hydroxy-3-methyl-2-cyclopenten-1-one; acetophenone; 2-ethyl-phenol; 2-methoxy-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; octanoic acid; 2,6-dimethoxy-phenol; 2-methyl-propanoic acid; 3-methyl-butanoic acid; D-limonene; 2-acetyl-5-methylfuran; 1-(4-methylphenyl)-ethanone; vanillin; pentanoic acid; benzoic acid; triacetin; benzeneacetic acid; maltol; and nicotine.

Many of these chemical markers have a boiling point of less than 250°C and therefore have good potential to be used in a non-combustible aerosol provision device or system, such as a heat-not-burn or vaping device or system.

By formulating a composition comprising some or all of the above compounds, it is possible to formulate a composition which may be able to simulate a cigarette-like experience. In particular, the key sense markers will contribute towards simulating the sense of a cigarette. The key taste markers will contribute towards simulating the taste of a cigarette. The key flavour markers will contribute towards simulating the flavour of a cigarette.

As will be described in more detail below, certain tobacco extracts have been obtained and found to contain some of these compounds (specifically from Lists 1, 2, 3 and 4). However, none of the extracts tested contained all of the key chemical markers from Lists 1, 2, 3 and 4. As a result, instead of obtaining and using an extract from tobacco (e.g. for use in a non-combustible aerosol provision system), it would be beneficial to create a synthetic or artificial composition (e.g. a solid, semi-solid or liquid composition) which includes the necessary chemical markers to produce a cigarette-like experience when used by the consumer.

Alternatively, it would also be beneficial to obtain tobacco extracts containing as many of the key chemical markers as possible. A tobacco extract which does not contain all of the key chemical markers identified herein can be supplemented with additional key chemical markers. That is, one or more of the key chemical markers can be added to a tobacco extract. Said tobacco extract may, in some embodiments, already contain one or more of the key chemical markers identified herein.

Compounds from just a single flavour dimension (e.g. sense, taste or flavour) can be enough to provide a composition having a cigarette-like sense, taste or flavour, or to increase the cigarette-like sense, taste or flavour of a given composition. However, in some embodiments compounds may be included from two or three of the flavour dimensions, e.g. to provide a composition having a cigarette-like sense and taste, sense and flavour, taste and flavour, or sense, taste and flavour. A composition comprising compounds from all three of the flavour dimensions will allow for a composition providing a cigarette-like sense, taste and flavour, or to increase the cigarette-like sense, taste and flavour of a given composition.

It is also possible to add further compounds to the compositions which may be absent from a tobacco extract, but present in smoke formed by burning the same tobacco used to form the extract (i.e. the "smoke markers"). This further improves the cigarette-like experience produced by the composition.

In one embodiment, the invention provides a composition (e.g. a solid, semi-solid or liquid composition) comprising at least three of the key chemical sense markers, namely the following compounds: nicotine; 2-hydroxy-4-methyl-2-cyclopenten-1-one; quinic acid; sucrose; N-(1-deoxy-1-fructosyl)proline; 3,4-dimethyl-2(3H)-furanone; lipid peroxidation inhibitor 1; pseudooxynicotine and/or oxynicotine; 2-ethyl-quinoxaline; 1,2,3,4,5,6-hexahydro-7H-cyclopenta[b]pyridin-7-one; 2-ethyl-pyridine; scopoletin; 2-ethyl-5-methyl-pyridine; rutin; retinol; 2-methyl-3-butyl-pyrazine; megastigmatrienone; 3,5-di(1,1-dimethylethyl)-4-hydroxybenzaldehyde; L-alpha-amino-1H-pyrrole-1-hexanoic acid; and solanesol.

The inventors have found that by combining at least 3 (e.g. at least 4, at least 5, at least 6, at least 8, at least 10 or at least 15) of the key sense markers (i.e. the compounds from List 1), a composition can be created which will achieve a more cigarette-like sensation than a composition without any of these markers (e.g. when used in a non-combustible aerosol provision system).

The chemical structure and CAS number of the key markers is set out below.

**Table 1 - chemical structures and CAS numbers of the key chemical markers for sense**

| **Key sense marker** | **Structure** | **CAS Number** |
|---|---|---|
| Nicotine | 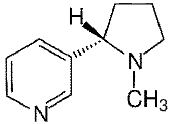 | 54-11-5 |
| 2-hydroxy-4-methyl-2-cyclopenten-1-one | 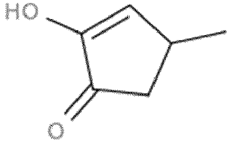 | 15899-72-6 |
| Quinic acid | 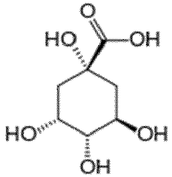 | 77-95-2 |
| Sucrose | 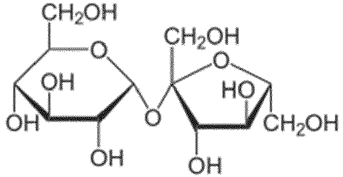 | 57-50-1 |
| N-(1-deoxy-1-fructosyl)proline | 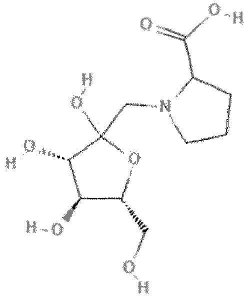 | 29118-61-4 |
| 3,4-dimethyl-2(3H)-furanone | 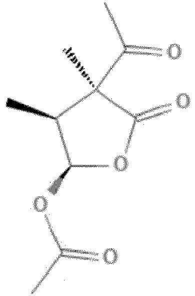 | 510-18-9 |
| Lipid peroxidation inhibitor 1 | 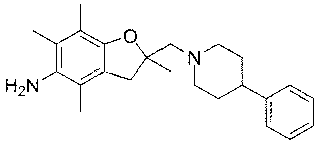 | 142873-41-4 |
| Pseudooxynicotine and/or oxynicotine | 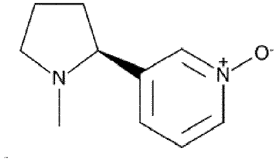 | 2820-55-5 |
| 2-ethyl-quinoxaline | 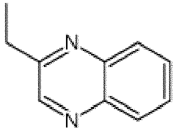 | 29750-44-5 |
| 1,2,3,4,5,6-hexahydro-7H-cyclopenta[b]pyridin-7-one | 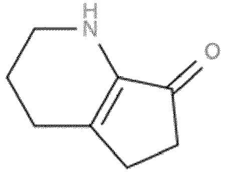 | 104704-30-5 |
| 2-ethyl-pyridine | 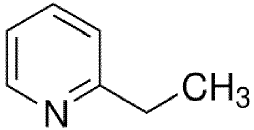 | 100-71-0 |
| Scopoletin | 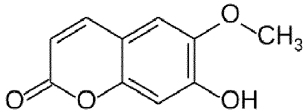 | 92-61-5 |
| 2-ethyl-5-methyl-pyridine | 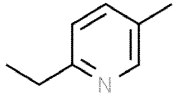 | 18113-81-0 |
| Rutin | 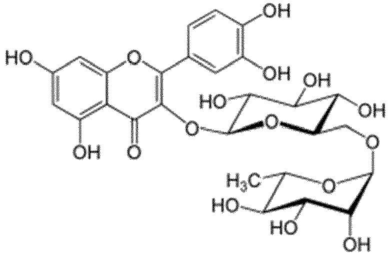 | 153-18-4 |
| Retinol | 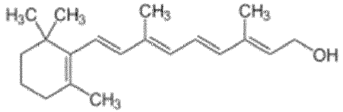 | 68-26-8 |
| 2-methyl-3-butyl-pyrazine | 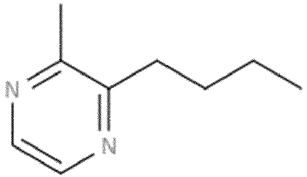 | 15987-00-5 |
| Megastigmatrienone | 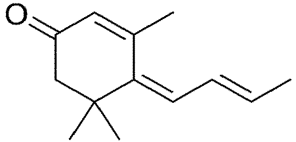 | 38818-55-2 |
| 3,5-di(1,1-dimethylethyl)-4-hydroxy-benzaldehyde | 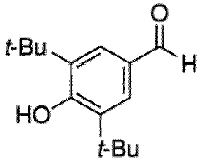 | 1620-98-0 |
| L-alpha-amino-1H-pyrrole-1-hexanoic acid | 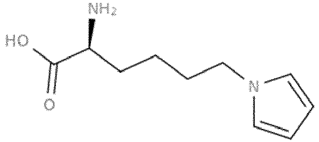 | 156539-32-1 |
| Solanesol | 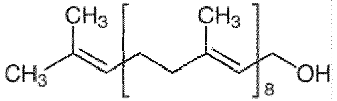 | 13190-97-1 |

**Table 2 - chemical structures and CAS numbers of the key chemical markers for taste**

| **Key taste marker** | **Structure** | **CAS Number** |
|---|---|---|
| Nicotine | 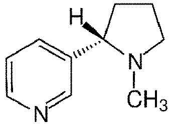 | 54-11-5 |
| Pseudooxynicotine and/or oxynicotine | 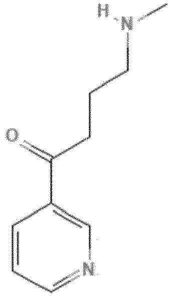 | 2820-55-5 |
| Lipid peroxidation inhibitor 1 | 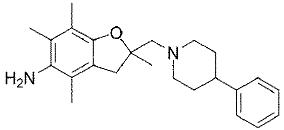 | 142873-41-4 |
| Scopoletin | 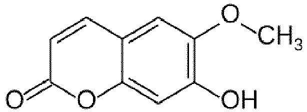 | 92-61-5 |
| N-(1-deoxy-1-fructosyl)proline | 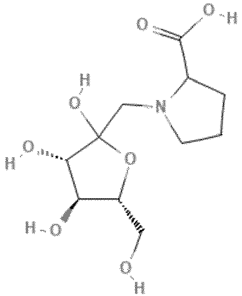 | 29118-61-4 |
| Sucrose | 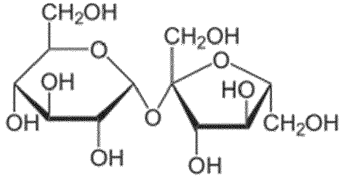 | 57-50-1 |
| Type I sucrose ester - GV - I0 | 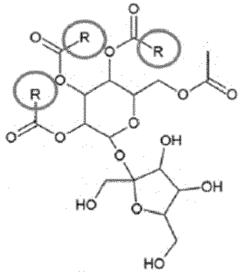 each R = -CH₂-CH(CH₃)-CH₂-CH₃ | |
| Type I sucrose ester - GIV - I0 | 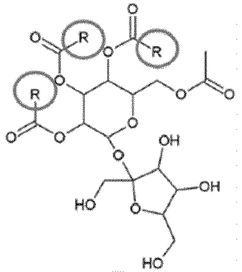 R = -CH₂-CH(CH₃)-CH₂-CH₃, | |
| | -CH₂-CH(CH₃)-CH₂-CH₃ | |
| | and -(CH₂)₃CH₃ | |
| Chlorogenic acid | 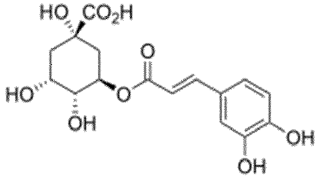 | 202650-88-2 |
| 1,2,3,4,5,6-Hexahydro-7H-cyclopenta[b]pyridin-7-one | 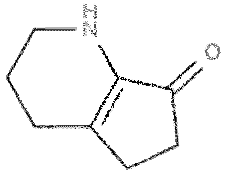 | 104704-30-5 |
| L-alpha-amino-1H-pyrrole-1-hexanoic acid | 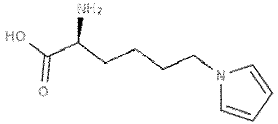 | 156539-32-1 |
| Retinol | 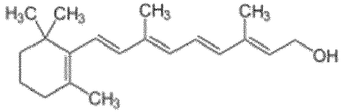 | 68-26-8 |
| Quinic acid | 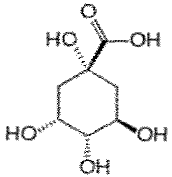 | 77-95-2 |
| Type I sucrose ester - GIII - I0 | 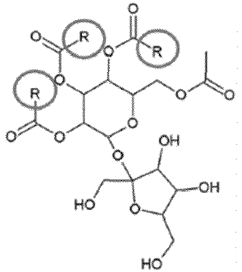 R = -CH₂-CH(CH₃)-CH₂-CH₃, | |
| | -(CH₂)₃CH₃ | |
| | and -(CH₂)₃CH₃ | |
| | or | |
| | R = -CH₂-CH(CH₃)-CH₂-CH₃, | |
| | -CH₂-CH(CH₃)-CH₂-CH₃, | |
| | and -(CH₂)₂CH₃ | |
| Rutin | 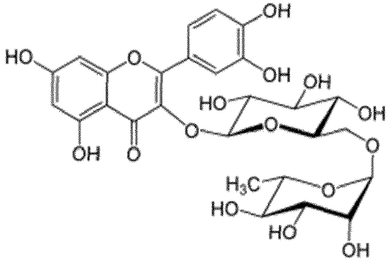 | 153-18-4 |
| Megastigmatrienone | 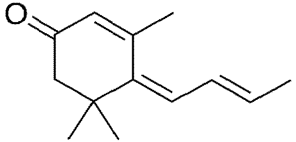 | 38818-55-2 |
| 2-ethyl-5-methyl-pyridine | 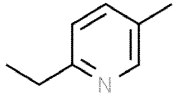 | 18113-81-0 |
| 16-hydroxy-hexadecanoic acid | 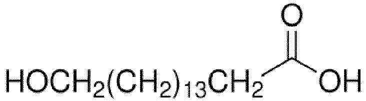 | 506-13-8 |
| Type III sucrose ester - GV - I0 | 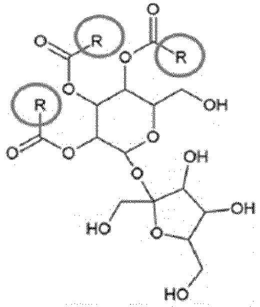 each R = -CH₂-CH(CH₃)-CH₂-CH₃, | |
| Ferulic acid | 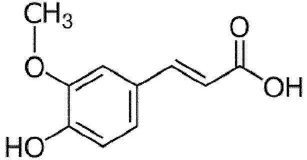 | 537-98-4 |

**Table 3-chemical structures and CAS numbers of the key chemical markers for flavour**

| **Key flavour marker** | **Structure** | **CAS Number** |
|---|---|---|
| 2-methoxy-phenol | 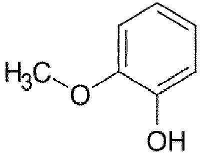 | 90-05-1 |
| p-Cresol | 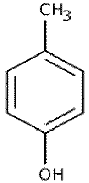 | 106-44-5 |
| Furaneol | 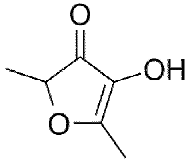 | 3658-77-3 |
| Indole | 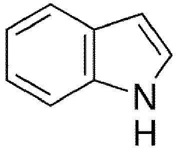 | 120-72-9 |
| 3-methyl-pentanoic acid | 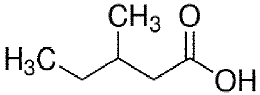 | 105-43-1 |
| Butanoic acid | 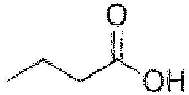 | 107-92-6 |
| 3-methyl-butanoic acid | 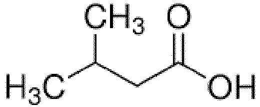 | 503-74-2 |
| D-limonene | 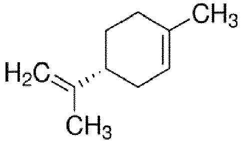 | 5989-27-5 |
| Acetophenone | 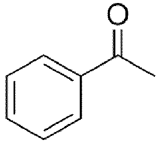 | 98-86-2 |
| 1-(4-methylphenyl)-ethanone | 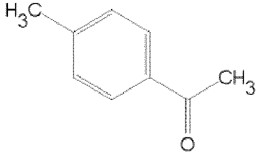 | 122-00-9 |
| Vanillin | 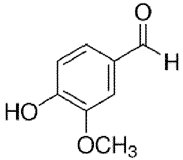 | 121-33-5 |
| 2,6-dimethoxy-phenol | 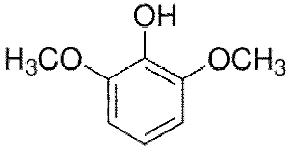 | 91-10-1 |
| Benzaldehyde | 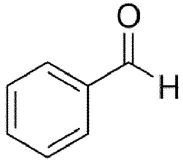 | 100-52-7 |
| Benzeneacetic acid | 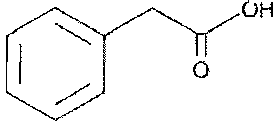 | 103-82-2 |
| 2-methyl-butanoic acid | 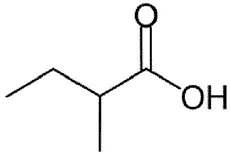 | 116-53-0 |
| Pentanoic acid | 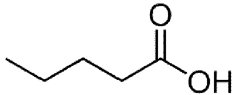 | 109-52-4 |
| Hexanoic acid | 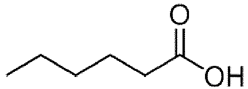 | 142-62-1 |
| Acetic acid | 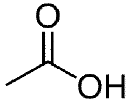 | 64-19-7 |
| 2-methyl-propanoic acid | 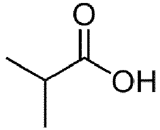 | 79-31-2 |
| Octanoic acid | 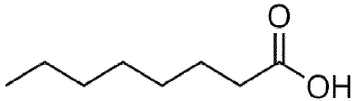 | 124-07-2 |

**Table 4 - chemical structures and CAS numbers of the key smoke markers**

| **Key chemical marker** | **Structure** | **CAS Number** |
|---|---|---|
| 3-Ethyl-2-hydroxy-2-cyclopenten-1-one | 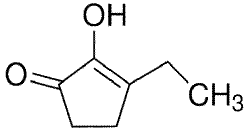 | 21835-01-8 |
| 5-Methyl-2-furancarboxaldehyde | 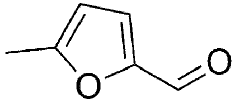 | 620-02-0 |
| Propanoic acid | 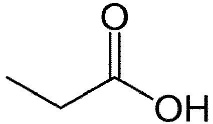 | 79-09-4 |
| 3-Methyl-pyridine | 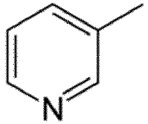 | 108-99-6 |
| Butanoic acid | 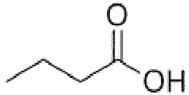 | 107-92-6 |
| 3-Ethyl-pyridine | 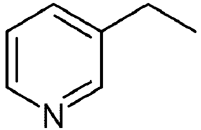 | 536-78-7 |
| Trimethyl-pyrazine | 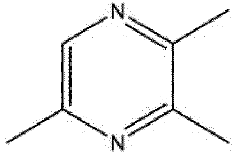 | 14667-55-1 |
| Benzaldehyde | 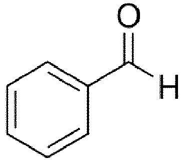 | 100-52-7 |
| Tiglic acid | 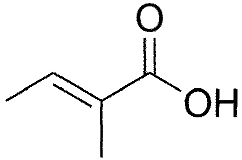 | 80-59-1 |
| 2-Hydroxy-3-methyl-2-cyclopenten-1-one | 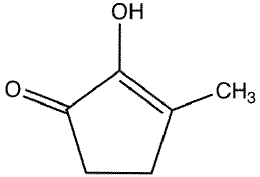 | 80-71-7 |
| Acetophenone | 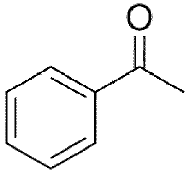 | 98-86-2 |
| 2-Ethyl-phenol | 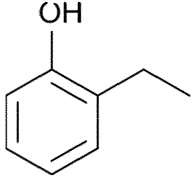 | 90-00-6 |
| 2-Methoxy-phenol | 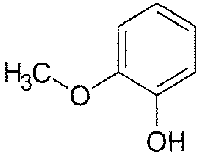 | 90-05-1 |
| Furaneol | 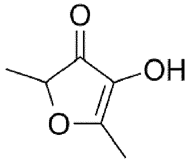 | 3658-77-3 |
| 4-Ethyl-phenol | 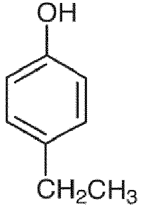 | 123-07-9 |
| 3-Ethyl-phenol | 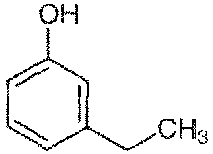 | 620-17-7 |
| 1-(1H-pyrrol-2-yl)-ethanone | 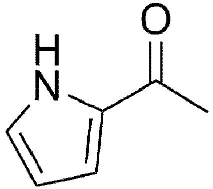 | 1072-83-9 |
| 2-Methoxy-4-vinylphenol | 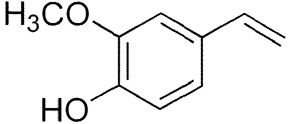 | 7786-61-0 |
| 4-Ethyl-2-methoxy-phenol | 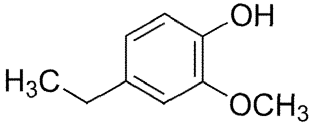 | 2785-89-9 |
| Octanoic acid | 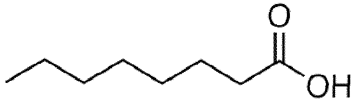 | 124-07-2 |
| 2,6-Dimethoxy-phenol | 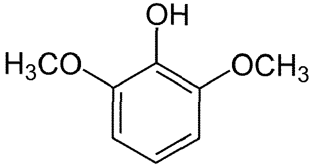 | 91-10-1 |
| 2-Methyl-propanoic acid | 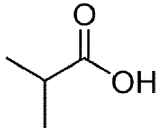 | 79-31-2 |
| 3-Methyl-butanoic acid | 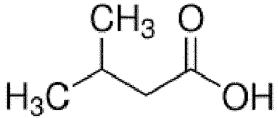 | 503-74-2 |
| D-Limonene | 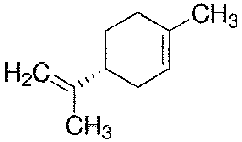 | 5989-27-5 |
| 2-Acetyl-5-methylfuran | 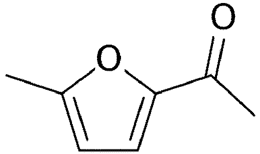 | 1193-79-9 |
| 1-(4-Methylphenyl)-ethanone | 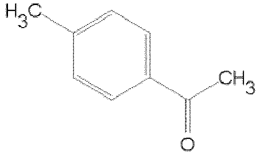 | 122-00-9 |
| Vanillin | 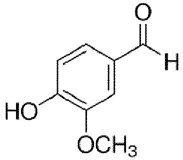 | 121-33-5 |
| Pentanoic acid | 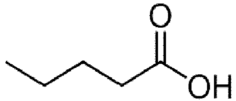 | 109-52-4 |
| Benzoic acid | 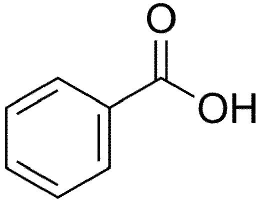 | 65-85-0 |
| Triacetin | 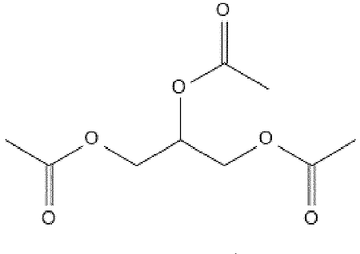 | 102-76-1 |
| Benzeneacetic acid | 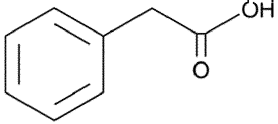 | 103-82-2 |
| Maltol | 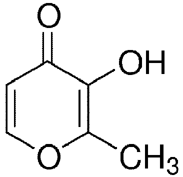 | 118-71-8 |
| Nicotine | 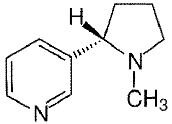 | 54-11-5 |

Nicotine is shown in the above tables in unprotonated form, but may also be present in protonated form, or as a mixture of protonated and unprotonated nicotine.

Some of the chemical markers described herein are sucrose esters. The structure of the sucrose esters can be explained taking into consideration two basic differences among them; "type" (T) is strictly related to acetylation of the glucose and fructose unit, whilst "group" (G) is more related to the number of carbon molecules from organic acid bonded in glucose unit.

At the present time four types of sucrose esters are known, namely, type I, type II, type III and type IV (see structures below).

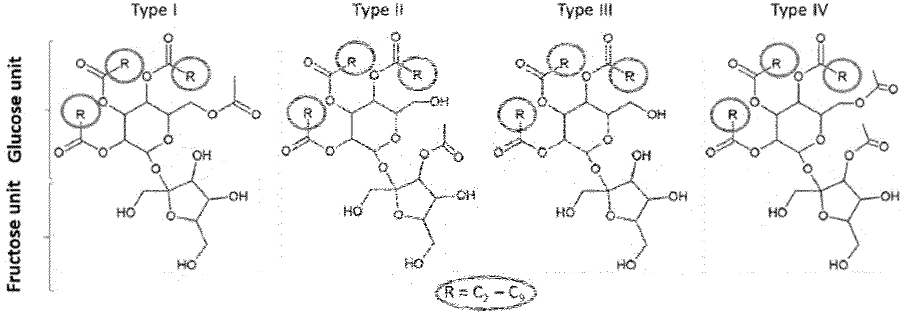

Type I and type II are mono-acetylated, differing by the acetylation at glucose and fructose unit respectively. Type IV is di-acetylated in both the glucose and fructose units. Type III doesn't include any acetylation at the glucose and fructose units.

The different sucrose esters groups are related to the sets of organic acid(s) that esterify the hydroxyl groups in the glucose unit. Five major sucrose esters groups include G1 to G5, for each sucrose ester type (as set out below).

| **Sucrose ester group** | **Set of organic acids esterifying the glucose unit (R)** |
|---|---|
| G I | C₆C₅C₃; C₆C₆C₂; C₆C₄C₄; C₅C₅C₄ |
| G II | C₆C₅C₄; C₆C₆C₃; C₅C₅C₅ |
| G III | C₆C₅C₅; C₆C₆C₄ |
| G IV | C₆C₆C₅ |
| G V | C₆C₆C₆ |

| | |
|---|---|
| Note: C₂ = acetic acid (C₂H₄O₂); C₃ = propionic acid (C₃H₆O₂); C₄ = butyric acid (CaHsOz); C₅ = valeric acid (C₅H₁₀O₂); C₆ = 3-methyl valeric acid (C₆H₁₂O₂) | |

Sucrose esters are named by type (e.g. T1, TI or Type 1 means Sucrose Ester Type I), group (e.g. GV or G5 means C₆C₆C₆ acids esterify the hydroxy groups on the glucose unit) and the number of double bonds on the acid groups (e.g. I0 means zero double bonds, or saturated acid groups).

### Key sense markers

In embodiments, the composition comprises at least 3, at least 4, at least 5, at least 6, at least 7, at least 8, at least 9, at least 10, at least 11, at least 12, at least 13, at least 14, at least 15, at least 16, at least 17, at least 18, or at least 19 of the key sense markers (see List 1).

In embodiments, the composition comprises all of the key sense markers.

In embodiments, the composition comprises at least nicotine.

In embodiments, the composition comprises at least nicotine and 2-hydroxy-4-methyl-2-cyclopenten-1-one.

In embodiments, the composition comprises at least nicotine; 2-hydroxy-4-methyl-2-cyclopenten-1-one; and quinic acid.

In embodiments, the composition comprises at least nicotine; 2-hydroxy-4-methyl-2-cyclopenten-1-one; quinic acid; and sucrose.

In embodiments, the composition comprises at least nicotine; 2-hydroxy-4-methyl-2-cyclopenten-1-one; quinic acid; sucrose; and N-(1-deoxy-1-fructosyl)proline.

In embodiments, the composition comprises at least nicotine; 2-hydroxy-4-methyl-2-cyclopenten-1-one; quinic acid; sucrose; N-(1-deoxy-1-fructosyl)proline; and 3,4-dimethyl-2(3H)-furanone.

In embodiments, the composition comprises at least nicotine; 2-hydroxy-4-methyl-2-cyclopenten-1-one; quinic acid; sucrose; N-(1-deoxy-1-fructosyl)proline; 3,4-dimethyl-2(3H)-furanone; and lipid peroxidation inhibitor 1.

In embodiments, the composition comprises at least nicotine; 2-hydroxy-4-methyl-2-cyclopenten-1-one; quinic acid; sucrose; N-(1-deoxy-1-fructosyl)proline; 3,4-dimethyl-2(3H)-furanone; lipid peroxidation inhibitor 1; and pseudooxynicotine and/or oxynicotine.

In embodiments, the composition comprises at least nicotine; 2-hydroxy-4-methyl-2-cyclopenten-1-one; quinic acid; sucrose; N-(1-deoxy-1-fructosyl)proline; 3,4-dimethyl-2(3H)-furanone; lipid peroxidation inhibitor 1; pseudooxynicotine and/or oxynicotine; and 2-ethyl-quinoxaline.

In embodiments, the composition comprises at least nicotine; 2-hydroxy-4-methyl-2-cyclopenten-1-one; quinic acid; sucrose; N-(1-deoxy-1-fructosyl)proline; 3,4-dimethyl-2(3H)-furanone; lipid peroxidation inhibitor 1; pseudooxynicotine and/or oxynicotine; 2-ethyl-quinoxaline; and 1,2,3,4,5,6-hexahydro-7H-cyclopenta[b]pyridin-7-one.

In embodiments, the composition comprises at least nicotine; 2-hydroxy-4-methyl-2-cyclopenten-1-one; quinic acid; sucrose; N-(1-deoxy-1-fructosyl)proline; 3,4-dimethyl-2(3H)-furanone; lipid peroxidation inhibitor 1; pseudooxynicotine and/or oxynicotine; 2-ethyl-quinoxaline; 1,2,3,4,5,6-hexahydro-7H-cyclopenta[b]pyridin-7-one; and 2-ethyl-pyridine.

In embodiments, the composition comprises at least nicotine; 2-hydroxy-4-methyl-2-cyclopenten-1-one; quinic acid; sucrose; N-(1-deoxy-1-fructosyl)proline; 3,4-dimethyl-2(3H)-furanone; lipid peroxidation inhibitor 1; pseudooxynicotine and/or oxynicotine; 2-ethyl-quinoxaline; 1,2,3,4,5,6-hexahydro-7H-cyclopenta[b]pyridin-7-one; 2-ethyl-pyridine; and scopoletin.

In embodiments, the composition comprises at least nicotine; 2-hydroxy-4-methyl-2-cyclopenten-1-one; quinic acid; sucrose; N-(1-deoxy-1-fructosyl)proline; 3,4-dimethyl-2(3H)-furanone; lipid peroxidation inhibitor 1; pseudooxynicotine and/or oxynicotine; 2-ethyl-quinoxaline; 1,2,3,4,5,6-hexahydro-7H-cyclopenta[b]pyridin-7-one; 2-ethyl-pyridine; scopoletin; and 2-ethyl-5-methyl-pyridine.

In embodiments, the composition comprises at least nicotine; 2-hydroxy-4-methyl-2-cyclopenten-1-one; quinic acid; sucrose; N-(1-deoxy-1-fructosyl)proline; 3,4-dimethyl-2(3H)-furanone; lipid peroxidation inhibitor 1; pseudooxynicotine and/or oxynicotine; 2-ethyl-quinoxaline; 1,2,3,4,5,6-hexahydro-7H-cyclopenta[b]pyridin-7-one; 2-ethyl-pyridine; scopoletin; 2-ethyl-5-methyl-pyridine; and rutin.

In embodiments, the composition comprises at least nicotine; 2-hydroxy-4-methyl-2-cyclopenten-1-one; quinic acid; sucrose; N-(1-deoxy-1-fructosyl)proline; 3,4-dimethyl-2(3H)-furanone; lipid peroxidation inhibitor 1; pseudooxynicotine and/or oxynicotine; 2-ethyl-quinoxaline; 1,2,3,4,5,6-hexahydro-7H-cyclopenta[b]pyridin-7-one; 2-ethyl-pyridine; scopoletin; 2-ethyl-5-methyl-pyridine; rutin; and retinol.

In embodiments, the composition comprises at least nicotine; 2-hydroxy-4-methyl-2-cyclopenten-1-one; quinic acid; sucrose; N-(1-deoxy-1-fructosyl)proline; 3,4-dimethyl-2(3H)-furanone; lipid peroxidation inhibitor 1; pseudooxynicotine and/or oxynicotine; 2-ethyl-quinoxaline; 1,2,3,4,5,6-hexahydro-7H-cyclopenta[b]pyridin-7-one; 2-ethyl-pyridine; scopoletin; 2-ethyl-5-methyl-pyridine; rutin; retinol; and 2-methyl-3-butyl-pyrazine.

In embodiments, the composition comprises at least nicotine; 2-hydroxy-4-methyl-2-cyclopenten-1-one; quinic acid; sucrose; N-(1-deoxy-1-fructosyl)proline; 3,4-dimethyl-2(3H)-furanone; lipid peroxidation inhibitor 1; pseudooxynicotine and/or oxynicotine; 2-ethyl-quinoxaline; 1,2,3,4,5,6-hexahydro-7H-cyclopenta[b]pyridin-7-one; 2-ethyl-pyridine; scopoletin; 2-ethyl-5-methyl-pyridine; rutin; retinol; 2-methyl-3-butyl-pyrazine; and megastigmatrienone.

In embodiments, the composition comprises at least nicotine; 2-hydroxy-4-methyl-2-cyclopenten-1-one; quinic acid; sucrose; N-(1-deoxy-1-fructosyl)proline; 3,4-dimethyl-2(3H)-furanone; lipid peroxidation inhibitor 1; pseudooxynicotine and/or oxynicotine; 2-ethyl-quinoxaline; 1,2,3,4,5,6-hexahydro-7H-cyclopenta[b]pyridin-7-one; 2-ethyl-pyridine; scopoletin; 2-ethyl-5-methyl-pyridine; rutin; retinol; 2-methyl-3-butyl-pyrazine; megastigmatrienone; and 3,5-di(1,1-dimethylethyl)-4-hydroxy-benzaldehyde.

In embodiments, the composition comprises at least nicotine; 2-hydroxy-4-methyl-2-cyclopenten-1-one; quinic acid; sucrose; N-(1-deoxy-1-fructosyl)proline; 3,4-dimethyl-2(3H)-furanone; lipid peroxidation inhibitor 1; pseudooxynicotine and/or oxynicotine; 2-ethyl-quinoxaline; 1,2,3,4,5,6-hexahydro-7H-cyclopenta[b]pyridin-7-one; 2-ethyl-pyridine; scopoletin; 2-ethyl-5-methyl-pyridine; rutin; retinol; 2-methyl-3-butyl-pyrazine; megastigmatrienone; 3,5-di(1,1-dimethylethyl)-4-hydroxy-benzaldehyde; and L-alpha-amino-1H-pyrrole-1-hexanoic acid.

In embodiments, the composition comprises at least nicotine; 2-hydroxy-4-methyl-2-cyclopenten-1-one; quinic acid; sucrose; N-(1-deoxy-1-fructosyl)proline; 3,4-dimethyl-2(3H)-furanone; lipid peroxidation inhibitor 1; pseudooxynicotine and/or oxynicotine; 2-ethyl-quinoxaline; 1,2,3,4,5,6-hexahydro-7H-cyclopenta[b]pyridin-7-one; 2-ethyl-pyridine; scopoletin; 2-ethyl-5-methyl-pyridine; rutin; retinol; 2-methyl-3-butyl-pyrazine; megastigmatrienone; 3,5-di(1,1-dimethylethyl)-4-hydroxy-benzaldehyde; L-alpha-amino-1H-pyrrole-1-hexanoic acid; and solanesol.

### Key taste, flavour and smoke markers

In embodiments, the composition also comprises 1 or more of the key taste markers (see List 2). In embodiments, the composition also comprises 2 or more of the key taste markers (see List 2). In embodiments, the composition also comprises at least 3, at least 4, at least 5, at least 6, at least 7, at least 8, at least 9, at least 10, at least 11, at least 12, at least 13, at least 14, at least 15, at least 16, at least 17, at least 18, at least 19 or at least 20 of the key taste markers (see List 2).

In embodiments, the composition also comprises 1 or more of the key flavour markers (see List 3). In embodiments, the composition also comprises 2 or more of the key flavour markers (see List 3). In embodiments, the composition also comprises at least 3, at least 4, at least 5, at least 6, at least 7, at least 8, at least 9, at least 10, at least 11, at least 12, at least 13, at least 14, at least 15, at least 16, at least 17, at least 18, at least 19 or at least 20 of the key flavour markers (see List 3).

In embodiments, the composition also comprises 1 or more of the key smoke markers (see List 4). In embodiments, the composition also comprises 2 or more of the key smoke markers (see List 4). In embodiments, the composition also comprises at least 3, at least 4, at least 5, at least 6, at least 7, at least 8, at least 9, at least 10, at least 11, at least 12, at least 13, at least 14, at least 15, at least 16, at least 17, at least 18, at least 19 or at least 20 of the key smoke markers (see List 4).

In embodiments, the composition comprises, in addition to the key sense markers, at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19 or at least 20 of each of the key taste and flavour markers (see Lists 2 and 3 respectively).

In embodiments, the composition comprises, in addition to the key sense markers, at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19 or at least 20 of each of the key taste markers and key smoke markers (see Lists 2 and 4 respectively).

In embodiments, the composition comprises, in addition to the key sense markers, at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19 or at least 20 of each of the key flavour markers and key smoke markers (see Lists 3 and 4 respectively).

In embodiments, the composition comprises, in addition to the key sense markers, at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19 or at least 20 of each of the key taste markers, key flavour markers and key smoke markers (see Lists 2, 3 and 4 respectively).

In embodiments, the composition comprises at least 3 (e.g. at least 4, at least 5, at least 6, at least 8, at least 10 or at least 15) compounds from List 1, at least 3 (e.g. at least 4, at least 5, at least 6, at least 8, at least 10 or at least 15) compounds from List 2, at least 3 (e.g. at least 4, at least 5, at least 6, at least 8, at least 10 or at least 15) compounds from List 3 and at least 3 (e.g. at least 4, at least 5, at least 6, at least 8, at least 10 or at least 15) compounds from List 4.

In some embodiments, where compounds from more than one of Lists 1, 2, 3 and 4 are present, the compounds may be the same or different. For example, a single compound (e.g. nicotine) can be both a compound from List 1 and from List 2.

In some embodiments, where compounds from more than one of Lists 1, 2, 3 and 4 are present, the compounds must be different. In this case, for example, a single compound (e.g. nicotine) can only count as a compound from List 1, but not also as a compound from List 2 or List 4. In this case any compound(s) included in the composition from List 2 and/or List 4 must be different to those already present in the composition from List 1.

In embodiments, the composition comprises at least nicotine and 2-methoxy-phenol. In embodiments, the composition comprises at least nicotine; 2-hydroxy-4-methyl-2-cyclopenten-1-one; pseudooxynicotine and/or oxynicotine; 2-methoxy-phenol; and p-cresol. In embodiments, the composition comprises at least nicotine; 2-hydroxy-4-methyl-2-cyclopenten-1-one; quinic acid; pseudooxynicotine and/or oxynicotine; lipid peroxidation inhibitor 1; 2-methoxy-phenol; p-cresol; and furaneol.

As used herein, reference to "pseudooxynicotine and/or oxynicotine" means that one or both of these compounds may be present. In some embodiments, only one of these is present (i.e. "pseudooxynicotine or oxynicotine").

In embodiments, the composition comprises all of the compounds listed in Lists 1, 2 and 3.

In embodiments, the composition comprises all of the compounds listed in Lists 1, 2, 3 and 4.

In embodiments, the composition comprises a total of up to about 10 wt%, 5 wt%, 4 wt%, 3, wt%, 2 wt%, 1 wt% or 0.5 wt%, such as from about 0.0001 to about 2 wt%, from about 0.001 to about 1 wt% or from about 0.005 to about 0.5 wt% of the compounds as defined in List 1.

In embodiments, the composition comprises a total of up to about 10 wt%, 5 wt%, 4 wt%, 3, wt%, 2 wt%, 1 wt% or 0.5 wt%, such as from about 0.0001 to about 2 wt%, from about 0.001 to about 1 wt% or from about 0.005 to about 0.5 wt% of the compounds as defined in List 2.

In embodiments, the composition comprises a total of up to about 10 wt%, 5 wt%, 4 wt%, 3, wt%, 2 wt%, 1 wt% or 0.5 wt%, such as from about 0.0001 to about 2 wt%, from about 0.001 to about 1 wt% or from about 0.005 to about 0.5 wt% of the compounds as defined in List 3.

In embodiments, the composition comprises a total of up to about 10 wt%, 5 wt%, 4 wt%, 3, wt%, 2 wt%, 1 wt% or 0.5 wt%, such as from about 0.0001 to about 2 wt%, from about 0.001 to about 1 wt% or from about 0.005 to about 0.5 wt% of the compounds as defined in List 4.

In embodiments, the composition comprises a total of up to about 10 wt%, 5 wt%, 4 wt%, 3, wt%, 2 wt%, 1 wt% or 0.5 wt%, such as from about 0.0001 to about 2 wt%, from about 0.001 to about 1 wt% or from about 0.005 to about 0.5 wt% of the compounds listed in Lists 1, 2 and 3.

In embodiments, the composition comprises a total of up to about 10 wt%, 5 wt%, 4 wt%, 3, wt%, 2 wt%, 1 wt% or 0.5 wt%, such as from about 0.0001 to about 2 wt%, from about 0.001 to about 1 wt% or from about 0.005 to about 0.5 wt% of the compounds listed in Lists 1, 2, 3 and 4.

### Marker combinations

In embodiments, the composition comprises at least 3,4-dimethyl-2(3H)-furanone; pseudooxynicotine and/or oxynicotine; 2-methyl-3-butyl-pyrazine; and megastigmatrienone. In this embodiment the composition may comprise or be a tobacco extract.

In embodiments, the composition comprises at least 3,4-dimethyl-2(3H)-furanone; pseudooxynicotine and/or oxynicotine; 2-methyl-3-butyl-pyrazine; megastigmatrienone; 2-methyl-butanoic acid; and acetic acid. In this embodiment the composition may comprise or be a tobacco extract.

In embodiments, the composition comprises at least 3,4-dimethyl-2(3H)-furanone; pseudooxynicotine and/or oxynicotine; 2-methyl-3-butyl-pyrazine; megastigmatrienone; 3-ethyl-2-hydroxy-2-cyclopenten-1-one; 3-methyl-pyridine; butanoic acid; 3-ethyl-pyridine; 2-hydroxy-3-methyl-2-cyclopenten-1-one; acetophenone; 2-ethyl-phenol; furaneol; 4-ethyl-phenol; 3-ethyl-phenol; 2-methoxy-4-vinylphenol; 4-ethyl-2-methoxy-phenol; and 2,6-dimethoxy-phenol. In this embodiment the composition may comprise or be a tobacco extract.

In embodiments, the composition comprises at least 3,4-dimethyl-2(3H)-furanone; pseudooxynicotine and/or oxynicotine; 2-methyl-3-butyl-pyrazine; megastigmatrienone; 2-methyl-butanoic acid; acetic acid; 3-ethyl-2-hydroxy-2-cyclopenten-1-one; 3-methyl-pyridine; butanoic acid; 3-ethyl-pyridine; 2-hydroxy-3-methyl-2-cyclopenten-1-one; acetophenone; 2-ethyl-phenol; furaneol; 4-ethyl-phenol; 3-ethyl-phenol; 2-methoxy-4-vinylphenol; 4-ethyl-2-methoxy-phenol; and 2,6-dimethoxy-phenol. In this embodiment the composition may comprise or be a tobacco extract.

In embodiments, the composition comprises at least nicotine; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; 2-methyl-3-butyl-pyrazine; megastigmatrienone; and solanesol. In this embodiment the composition may comprise or be a tobacco extract, which may be obtained using supercritical fluid extraction (SFE).

In embodiments, the composition comprises at least nicotine; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; 2-methyl-3-butyl-pyrazine; megastigmatrienone; solanesol; and 16-hydroxy-hexadecanoicacid. In this embodiment the composition may comprise or be a tobacco extract, which may be obtained using supercritical fluid extraction (SFE).

In embodiments, the composition comprises at least nicotine; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; 2-methyl-3-butyl-pyrazine; megastigmatrienone; solanesol; 3-methyl-pentanoic acid; benzeneacetic acid; 2-methyl-butanoic acid; pentanoic acid; hexanoic acid; and acetic acid. In this embodiment the composition may comprise or be a tobacco extract, which may be obtained using supercritical fluid extraction (SFE).

In embodiments, the composition comprises at least nicotine; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; 2-methyl-3-butyl-pyrazine; megastigmatrienone; solanesol; 3-ethyl-2-hydroxy-2-cyclopenten-1-one; 5-methyl-2-furancarboxaldehyde; propanoic acid; 3-methyl-pyridine; butanoic acid; 3-ethyl-pyridine; benzaldehyde; 2-hydroxy-3-methyl-2-cyclopenten-1-one; acetophenone; 2-ethyl-phenol; 2-methoxy-phenol; furaneol; 4-ethyl-phenol; 3-ethyl-phenol; 2-methoxy-4-vinylphenol; 4-ethyl-2-methoxy-phenol; octanoic acid; and 2,6-dimethoxy-phenol. In this embodiment the composition may comprise or be a tobacco extract, which may be obtained using supercritical fluid extraction (SFE).

In embodiments, the composition comprises at least nicotine; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 16-hydroxy-hexadecanoicacid; 2-ethyl-5-methyl-pyridine; 2-methyl-3-butyl-pyrazine; megastigmatrienone; solanesol; 3-methyl-pentanoic acid; benzeneacetic acid; 2-methyl-butanoic acid; pentanoic acid; hexanoic acid; and acetic acid. In this embodiment the composition may comprise or be a tobacco extract, which may be obtained using supercritical fluid extraction (SFE).

In embodiments, the composition comprises at least nicotine; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; 2-methyl-3-butyl-pyrazine; megastigmatrienone; solanesol; 16-hydroxy-hexadecanoicacid; 3-ethyl-2-hydroxy-2-cyclopenten-1-one; 5-methyl-2-furancarboxaldehyde; propanoic acid; 3-methyl-pyridine; butanoic acid; 3-ethyl-pyridine; benzaldehyde; 2-hydroxy-3-methyl-2-cyclopenten-1-one; acetophenone; 2-ethyl-phenol; 2-methoxy-phenol; furaneol; 4-ethyl-phenol; 3-ethyl-phenol; 2-methoxy-4-vinylphenol; 4-ethyl-2-methoxy-phenol; octanoic acid; and 2,6-dimethoxy-phenol. In this embodiment the composition may comprise or be a tobacco extract, which may be obtained using supercritical fluid extraction (SFE).

In embodiments, the composition comprises at least nicotine; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; 2-methyl-3-butyl-pyrazine; megastigmatrienone; solanesol; 3-methyl-pentanoic acid; benzeneacetic acid; 2-methyl-butanoic acid; pentanoic acid; hexanoic acid; acetic acid; 3-ethyl-2-hydroxy-2-cyclopenten-1-one; 5-methyl-2-furancarboxaldehyde; propanoic acid; 3-methyl-pyridine; butanoic acid; 3-ethyl-pyridine; benzaldehyde; 2-hydroxy-3-methyl-2-cyclopenten-1-one; acetophenone; 2-ethyl-phenol; 2-methoxy-phenol; furaneol; 4-ethyl-phenol; 3-ethyl-phenol; 2-methoxy-4-vinylphenol; 4-ethyl-2-methoxy-phenol; octanoic acid; and 2,6-dimethoxy-phenol. In this embodiment the composition may comprise or be a tobacco extract, which may be obtained using supercritical fluid extraction (SFE).

In embodiments, the composition comprises at least nicotine; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 16-hydroxy-hexadecanoicacid; 2-ethyl-5-methyl-pyridine; 2-methyl-3-butyl-pyrazine; megastigmatrienone; solanesol; 3-methyl-pentanoic acid; benzeneacetic acid; 2-methyl-butanoic acid; pentanoic acid; hexanoic acid; acetic acid; 3-ethyl-2-hydroxy-2-cyclopenten-1-one; 5-methyl-2-furancarboxaldehyde; propanoic acid; 3-methyl-pyridine; butanoic acid; 3-ethyl-pyridine; benzaldehyde; 2-hydroxy-3-methyl-2-cyclopenten-1-one; acetophenone; 2-ethyl-phenol; 2-methoxy-phenol; furaneol; 4-ethyl-phenol; 3-ethyl-phenol; 2-methoxy-4-vinylphenol; 4-ethyl-2-methoxy-phenol; octanoic acid; and 2,6-dimethoxy-phenol. In this embodiment the composition may comprise or be a tobacco extract, which may be obtained using supercritical fluid extraction (SFE).

In embodiments, the composition comprises at least 3,4-dimethyl-2(3H)-furanone; pseudooxynicotine and/or oxynicotine; retinol; 2-methyl-3-butyl-pyrazine; megastigmatrienone; and L-alpha-amino-1H-pyrrole-1-hexanoic acid. In this embodiment the composition may comprise or be a tobacco extract, which may be obtained using simultaneous distillation extraction (SDE).

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; and Type III sucrose ester - GV - I0. In this embodiment the composition may comprise or be a tobacco extract, which may be obtained using simultaneous distillation extraction (SDE).

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; 2-methoxy-phenol; 3-methyl-butanoic acid; 1-(4-methylphenyl)-ethanone; benzaldehyde; 2-methyl-butanoic acid; acetic acid; and octanoic acid. In this embodiment the composition may comprise or be a tobacco extract, which may be obtained using simultaneous distillation extraction (SDE).

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; 3-ethyl-2-hydroxy-2-cyclopenten-1-one; 3-methyl-pyridine; butanoic acid; 3-ethyl-pyridine; tiglic acid; 2-hydroxy-3-methyl-2-cyclopenten-1-one; acetophenone; 2-ethyl-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; 2,6-dimethoxy-phenol; benzoic acid; triacetin; benzeneacetic acid; maltol; and nicotine. In this embodiment the composition may comprise or be a tobacco extract, which may be obtained using simultaneous distillation extraction (SDE).

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; Type III sucrose ester - GV - I0; 2-methoxy-phenol; 3-methyl-butanoic acid; 1-(4-methylphenyl)-ethanone; benzaldehyde; 2-methyl-butanoic acid; acetic acid; and octanoic acid. In this embodiment the composition may comprise or be a tobacco extract, which may be obtained using simultaneous distillation extraction (SDE).

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; Type III sucrose ester - GV - I0; 3-ethyl-2-hydroxy-2-cyclopenten-1-one; 3-methyl-pyridine; butanoic acid; 3-ethyl-pyridine; tiglic acid; 2-hydroxy-3-methyl-2-cyclopenten-1-one; acetophenone; 2-ethyl-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; 2,6-dimethoxy-phenol; benzoic acid; triacetin; benzeneacetic acid; maltol; and nicotine. In this embodiment the composition may comprise or be a tobacco extract, which may be obtained using simultaneous distillation extraction (SDE).

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; 2-methoxy-phenol; 3-methyl-butanoic acid; 1-(4-methylphenyl)-ethanone; benzaldehyde; 2-methyl-butanoic acid; acetic acid; octanoic acid; 3-ethyl-2-hydroxy-2-cyclopenten-1-one; 3-methyl-pyridine; butanoic acid; 3-ethyl-pyridine; tiglic acid; 2-hydroxy-3-methyl-2-cyclopenten-1-one; acetophenone; 2-ethyl-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; 2,6-dimethoxy-phenol; benzoic acid; triacetin; benzeneacetic acid; maltol; and nicotine. In this embodiment the composition may comprise or be a tobacco extract, which may be obtained using simultaneous distillation extraction (SDE).

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; Type III sucrose ester - GV - I0; 2-methoxy-phenol; 3-methyl-butanoic acid; 1-(4-methylphenyl)-ethanone; benzaldehyde; 2-methyl-butanoic acid; acetic acid; octanoic acid; 3-ethyl-2-hydroxy-2-cyclopenten-1-one; 3-methyl-pyridine; butanoic acid; 3-ethyl-pyridine; tiglic acid; 2-hydroxy-3-methyl-2-cyclopenten-1-one; acetophenone; 2-ethyl-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; 2,6-dimethoxy-phenol; benzoic acid; triacetin; benzeneacetic acid; maltol; and nicotine. In this embodiment the composition may comprise or be a tobacco extract, which may be obtained using simultaneous distillation extraction (SDE).

In embodiments, the composition comprises at least 3,4-dimethyl-2(3H)-furanone; pseudooxynicotine and/or oxynicotine; retinol; 2-methyl-3-butyl-pyrazine; and megastigmatrienone. In this embodiment the composition may comprise or be a tobacco extract, which may be an extract of a blend of flue-cured Virginia, air-cured burley and sun-cured oriental tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; Type I sucrose ester - GV - I0; and Type III sucrose ester - GV - I0. In this embodiment the composition may comprise or be a tobacco extract, which may be an extract of a blend of flue-cured Virginia, air-cured burley and sun-cured oriental tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; 3-methyl-pentanoic acid; 3-methyl-butanoic acid; 1-(4-methylphenyl)-ethanone; 2-methyl-butanoic acid; pentanoic acid; hexanoic acid; acetic acid; and 2-methyl-propanoic acid. In this embodiment the composition may comprise or be a tobacco extract, which may be an extract of a blend of flue-cured Virginia, air-cured burley and sun-cured oriental tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; 3-ethyl-2-hydroxy-2-cyclopenten-1-one; 3-methyl-pyridine; butanoic acid; 3-ethyl-pyridine; trimethyl-pyrazine; tiglic acid; 2-hydroxy-3-methyl-2-cyclopenten-1-one; acetophenone; 2-ethyl-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; and 2,6-dimethoxy-phenol. In this embodiment the composition may comprise or be a tobacco extract, which may be an extract of a blend of flue-cured Virginia, air-cured burley and sun-cured oriental tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; Type I sucrose ester - GV - I0; Type III sucrose ester - GV - I0; 3-methyl-pentanoic acid; 3-methyl-butanoic acid; 1-(4-methylphenyl)-ethanone; 2-methyl-butanoic acid; pentanoic acid; hexanoic acid; acetic acid; and 2-methyl-propanoic acid. In this embodiment the composition may comprise or be a tobacco extract, which may be an extract of a blend of flue-cured Virginia, air-cured burley and sun-cured oriental tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; Type I sucrose ester - GV - I0; Type III sucrose ester - GV - I0; 3-ethyl-2-hydroxy-2-cyclopenten-1-one; 3-methyl-pyridine; butanoic acid; 3-ethyl-pyridine; trimethyl-pyrazine; tiglic acid; 2-hydroxy-3-methyl-2-cyclopenten-1-one; acetophenone; 2-ethyl-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; and 2,6-dimethoxy-phenol. In this embodiment the composition may comprise or be a tobacco extract, which may be an extract of a blend of flue-cured Virginia, air-cured burley and sun-cured oriental tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; 3-methyl-pentanoic acid; 3-methyl-butanoic acid; 1-(4-methylphenyl)-ethanone; 2-methyl-butanoic acid; pentanoic acid; hexanoic acid; acetic acid; 2-methyl-propanoic acid; 3-ethyl-2-hydroxy-2-cyclopenten-1-one; 3-methyl-pyridine; butanoic acid; 3-ethyl-pyridine; trimethyl-pyrazine; tiglic acid; 2-hydroxy-3-methyl-2-cyclopenten-1-one; acetophenone; 2-ethyl-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; and 2,6-dimethoxy-phenol. In this embodiment the composition may comprise or be a tobacco extract, which may be an extract of a blend of flue-cured Virginia, air-cured burley and sun-cured oriental tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; Type I sucrose ester - GV - I0; Type III sucrose ester - GV - I0; 3-methyl-pentanoic acid; 3-methyl-butanoic acid; 1-(4-methylphenyl)-ethanone; 2-methyl-butanoic acid; pentanoic acid; hexanoic acid; acetic acid; and 2-methyl-propanoic acid; 3-ethyl-2-hydroxy-2-cyclopenten-1-one; 3-methyl-pyridine; butanoic acid; 3-ethyl-pyridine; trimethyl-pyrazine; tiglic acid; 2-hydroxy-3-methyl-2-cyclopenten-1-one; acetophenone; 2-ethyl-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; and 2,6-dimethoxy-phenol. In this embodiment the composition may comprise or be a tobacco extract, which may be an extract of a blend of flue-cured Virginia, air-cured burley and sun-cured oriental tobacco.

In embodiments, the composition comprises at least 3,4-dimethyl-2(3H)-furanone; pseudooxynicotine and/or oxynicotine; 2-methyl-3-butyl-pyrazine; megastigmatrienone; and L-alpha-amino-1H-pyrrole-1-hexanoic acid. In this embodiment the composition may comprise or be a tobacco extract, which may be an extract of flue-cured Virginia tobacco.

In embodiments, the composition comprises at least 3,4-dimethyl-2(3H)-furanone; pseudooxynicotine and/or oxynicotine; 2-methyl-3-butyl-pyrazine; megastigmatrienone; L-alpha-amino-1H-pyrrole-1-hexanoic; 2-methyl-butanoic acid; and acetic acid. In this embodiment the composition may comprise or be a tobacco extract, which may be an extract of flue-cured Virginia tobacco.

In embodiments, the composition comprises at least 3,4-dimethyl-2(3H)-furanone; pseudooxynicotine and/or oxynicotine; 2-methyl-3-butyl-pyrazine; megastigmatrienone; L-alpha-amino-1H-pyrrole-1-hexanoic acid; 3-ethyl-2-hydroxy-2-cyclopenten-1-one; 3-methyl-pyridine; butanoic acid; 3-ethyl-pyridine; 2-hydroxy-3-methyl-2-cyclopenten-1-one; acetophenone; 2-ethyl-phenol; furaneol; 4-ethyl-phenol; 3-ethyl-phenol; 2-methoxy-4-vinylphenol; 4-ethyl-2-methoxy-phenol; 2,6-dimethoxy-phenol; 2-methyl-propanoic acid; and vanillin. In this embodiment the composition may comprise or be a tobacco extract, which may be an extract of flue-cured Virginia tobacco.

In embodiments, the composition comprises at least 3,4-dimethyl-2(3H)-furanone; pseudooxynicotine and/or oxynicotine; 2-methyl-3-butyl-pyrazine; megastigmatrienone; L-alpha-amino-1H-pyrrole-1-hexanoic; 2-methyl-butanoic acid; acetic acid; 3-ethyl-2-hydroxy-2-cyclopenten-1-one; 3-methyl-pyridine; butanoic acid; 3-ethyl-pyridine; 2-hydroxy-3-methyl-2-cyclopenten-1-one; acetophenone; 2-ethyl-phenol; furaneol; 4-ethyl-phenol; 3-ethyl-phenol; 2-methoxy-4-vinylphenol; 4-ethyl-2-methoxy-phenol; 2,6-dimethoxy-phenol; 2-methyl-propanoic acid; and vanillin. In this embodiment the composition may comprise or be a tobacco extract, which may be an extract of flue-cured Virginia tobacco.

In embodiments, the composition comprises at least nicotine; quinic acid; sucrose; N-(1-deoxy-1-fructosyl)proline; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; rutin; 2-methyl-3-butyl-pyrazine; megastigmatrienone; L-alpha-amino-1H-pyrrole-1-hexanoic acid; and solanesol. In this embodiment the composition may comprise or be a tobacco extract, which may be obtained using supercritical fluid extraction (SFE) and/or which may be an extract of flue-cured Virginia tobacco.

In embodiments, the composition comprises at least nicotine; quinic acid; sucrose; N-(1-deoxy-1-fructosyl)proline; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; rutin; 2-methyl-3-butyl-pyrazine; megastigmatrienone; L-alpha-amino-1H-pyrrole-1-hexanoic acid; solanesol; chlorogenic acid; 16-hydroxy-hexadecanoicacid; and ferulic acid. In this embodiment the composition may comprise or be a tobacco extract, which may be obtained using supercritical fluid extraction (SFE) and/or which may be an extract of flue-cured Virginia tobacco.

In embodiments, the composition comprises at least nicotine; quinic acid; sucrose; N-(1-deoxy-1-fructosyl)proline; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; rutin; 2-methyl-3-butyl-pyrazine; megastigmatrienone; L-alpha-amino-1H-pyrrole-1-hexanoic acid; solanesol; 3-methyl-pentanoic acid; benzeneacetic acid; 2-methyl-butanoic acid; pentanoic acid; hexanoic acid; and acetic acid. In this embodiment the composition may comprise or be a tobacco extract, which may be obtained using supercritical fluid extraction (SFE) and/or which may be an extract of flue-cured Virginia tobacco.

In embodiments, the composition comprises at least nicotine; quinic acid; sucrose; N-(1-deoxy-1-fructosyl)proline; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; rutin; 2-methyl-3-butyl-pyrazine; megastigmatrienone; L-alpha-amino-1H-pyrrole-1-hexanoic acid; solanesol; 3-ethyl-2-hydroxy-2-cyclopenten-1-one; 5-methyl-2-furancarboxaldehyde; propanoic acid; 3-methyl-pyridine; butanoic acid; 3-ethyl-pyridine; benzaldehyde; 2-hydroxy-3-methyl-2-cyclopenten-1-one; acetophenone; 2-ethyl-phenol; 2-methoxy-phenol; furaneol; 4-ethyl-phenol; 3-ethyl-phenol; 2-methoxy-4-vinylphenol; 4-ethyl-2-methoxy-phenol; octanoic acid; 2,6-dimethoxy-phenol; 2-methyl-propanoic acid; 3-methyl-butanoic acid; D-limonene; 2-acetyl-5-methylfuran; 1-(4-methylphenyl)-ethanone; and vanillin. In this embodiment the composition may comprise or be a tobacco extract, which may be obtained using supercritical fluid extraction (SFE) and/or which may be an extract of flue-cured Virginia tobacco.

In embodiments, the composition comprises at least nicotine; quinic acid; sucrose; N-(1-deoxy-1-fructosyl)proline; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; rutin; 2-methyl-3-butyl-pyrazine; megastigmatrienone; L-alpha-amino-1H-pyrrole-1-hexanoic acid; solanesol chlorogenic acid; 16-hydroxy-hexadecanoicacid; ferulic acid; 3-methyl-pentanoic acid; benzeneacetic acid; 2-methyl-butanoic acid; pentanoic acid; hexanoic acid; and acetic acid. In this embodiment the composition may comprise or be a tobacco extract, which may be obtained using supercritical fluid extraction (SFE) and/or which may be an extract of flue-cured Virginia tobacco.

In embodiments, the composition comprises at least nicotine; quinic acid; sucrose; N-(1-deoxy-1-fructosyl)proline; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; rutin; 2-methyl-3-butyl-pyrazine; megastigmatrienone; L-alpha-amino-1H-pyrrole-1-hexanoic acid; solanesol; chlorogenic acid; 16-hydroxy-hexadecanoicacid; ferulic acid; 3-ethyl-2-hydroxy-2-cyclopenten-1-one; 5-methyl-2-furancarboxaldehyde; propanoic acid; 3-methyl-pyridine; butanoic acid; 3-ethyl-pyridine; benzaldehyde; 2-hydroxy-3-methyl-2-cyclopenten-1-one; acetophenone; 2-ethyl-phenol; 2-methoxy-phenol; furaneol; 4-ethyl-phenol; 3-ethyl-phenol; 2-methoxy-4-vinylphenol; 4-ethyl-2-methoxy-phenol; octanoic acid; 2,6-dimethoxy-phenol; 2-methyl-propanoic acid; 3-methyl-butanoic acid; D-limonene; 2-acetyl-5-methylfuran; 1-(4-methylphenyl)-ethanone; and vanillin. In this embodiment the composition may comprise or be a tobacco extract, which may be obtained using supercritical fluid extraction (SFE) and/or which may be an extract of flue-cured Virginia tobacco.

In embodiments, the composition comprises at least nicotine; quinic acid; sucrose; N-(1-deoxy-1-fructosyl)proline; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; rutin; 2-methyl-3-butyl-pyrazine; megastigmatrienone; L-alpha-amino-1H-pyrrole-1-hexanoic acid; solanesol; 3-methyl-pentanoic acid; benzeneacetic acid; 2-methyl-butanoic acid; pentanoic acid; hexanoic acid; acetic acid; 3-ethyl-2-hydroxy-2-cyclopenten-1-one; 5-methyl-2-furancarboxaldehyde; propanoic acid; 3-methyl-pyridine; butanoic acid; 3-ethyl-pyridine; benzaldehyde; 2-hydroxy-3-methyl-2-cyclopenten-1-one; acetophenone; 2-ethyl-phenol; 2-methoxy-phenol; furaneol; 4-ethyl-phenol; 3-ethyl-phenol; 2-methoxy-4-vinylphenol; 4-ethyl-2-methoxy-phenol; octanoic acid; 2,6-dimethoxy-phenol; 2-methyl-propanoic acid; 3-methyl-butanoic acid; D-limonene; 2-acetyl-5-methylfuran; 1-(4-methylphenyl)-ethanone; and vanillin. In this embodiment the composition may comprise or be a tobacco extract, which may be obtained using supercritical fluid extraction (SFE) and/or which may be an extract of flue-cured Virginia tobacco.

In embodiments, the composition comprises at least nicotine; quinic acid; sucrose; N-(1-deoxy-1-fructosyl)proline; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; rutin; 2-methyl-3-butyl-pyrazine; megastigmatrienone; L-alpha-amino-1H-pyrrole-1-hexanoic acid; solanesol chlorogenic acid; 16-hydroxy-hexadecanoicacid; ferulic acid; 3-methyl-pentanoic acid; benzeneacetic acid; 2-methyl-butanoic acid; pentanoic acid; hexanoic acid; acetic acid; 3-ethyl-2-hydroxy-2-cyclopenten-1-one; 5-methyl-2-furancarboxaldehyde; propanoic acid; 3-methyl-pyridine; butanoic acid; 3-ethyl-pyridine; benzaldehyde; 2-hydroxy-3-methyl-2-cyclopenten-1-one; acetophenone; 2-ethyl-phenol; 2-methoxy-phenol; furaneol; 4-ethyl-phenol; 3-ethyl-phenol; 2-methoxy-4-vinylphenol; 4-ethyl-2-methoxy-phenol; octanoic acid; 2,6-dimethoxy-phenol; 2-methyl-propanoic acid; 3-methyl-butanoic acid; D-limonene; 2-acetyl-5-methylfuran; 1-(4-methylphenyl)-ethanone; and vanillin. In this embodiment the composition may comprise or be a tobacco extract, which may be obtained using supercritical fluid extraction (SFE) and/or which may be an extract of flue-cured Virginia tobacco.

In embodiments, the composition comprises at least nicotine; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; retinol; 2-methyl-3-butyl-pyrazine; megastigmatrienone; and solanesol. In this embodiment the composition may comprise or be a tobacco extract, which may be obtained using supercritical fluid extraction (SFE) and/or which may be an extract of a blend of flue-cured Virginia, air-cured burley and sun-cured oriental tobacco.

In embodiments, the composition comprises at least nicotine; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; retinol; 2-methyl-3-butyl-pyrazine; megastigmatrienone; solanesol; Type I sucrose ester - GV - I0; Type I sucrose ester - GIV - I0; Type I sucrose ester - Gill - I0; 16-hydroxy-hexadecanoicacid; and Type III sucrose ester - GV - I0. In this embodiment the composition may comprise or be a tobacco extract, which may be obtained using supercritical fluid extraction (SFE) and/or which may be an extract of a blend of flue-cured Virginia, air-cured burley and sun-cured oriental tobacco.

In embodiments, the composition comprises at least nicotine; quinic acid; sucrose; N-(1-deoxy-1-fructosyl)proline; 3,4-dimethyl-2(3H)-furanone; Pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; rutin; 2-methyl-3-butyl-pyrazine; megastigmatrienone; L-alpha-amino-1H-pyrrole-1-hexanoic acid; solanesol; 3-methyl-pentanoic acid; 3-methyl-butanoic acid; 1-(4-methylphenyl)-ethanone; benzeneacetic acid; 2-methyl-butanoic acid; pentanoic acid; hexanoic acid; acetic acid; and 2-methyl-propanoic acid. In this embodiment the composition may comprise or be a tobacco extract, which may be obtained using supercritical fluid extraction (SFE) and/or which may be an extract of a blend of flue-cured Virginia, air-cured burley and sun-cured oriental tobacco.

In embodiments, the composition comprises at least nicotine; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; retinol; 2-methyl-3-butyl-pyrazine; megastigmatrienone; solanesol; 3-ethyl-2-hydroxy-2-cyclopenten-1-one; 5-methyl-2-furancarboxaldehyde; propanoic acid; 3-methyl-pyridine; butanoic acid; 3-ethyl-pyridine; trimethyl-pyrazine; benzaldehyde; tiglic acid; 2-hydroxy-3-methyl-2-cyclopenten-1-one; acetophenone; 2-ethyl-phenol; 2-methoxy-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; octanoic acid; and 2,6-dimethoxy-phenol. In this embodiment the composition may comprise or be a tobacco extract, which may be obtained using supercritical fluid extraction (SFE) and/or which may be an extract of a blend of flue-cured Virginia, air-cured burley and sun-cured oriental tobacco.

In embodiments, the composition comprises at least nicotine; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; retinol; 2-methyl-3-butyl-pyrazine; megastigmatrienone; solanesol; Type I sucrose ester - GV - I0; Type I sucrose ester - GIV - I0; Type I sucrose ester - Gill - I0; 16-hydroxy-hexadecanoicacid; Type III sucrose ester - GV - I0; 3-methyl-pentanoic acid; 3-methyl-butanoic acid; 1-(4-methylphenyl)-ethanone; benzeneacetic acid; 2-methyl-butanoic acid; pentanoic acid; hexanoic acid; acetic acid; and 2-methyl-propanoic acid. In this embodiment the composition may comprise or be a tobacco extract, which may be obtained using supercritical fluid extraction (SFE) and/or which may be an extract of a blend of flue-cured Virginia, air-cured burley and sun-cured oriental tobacco.

In embodiments, the composition comprises at least nicotine; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; retinol; 2-methyl-3-butyl-pyrazine; megastigmatrienone; solanesol; Type I sucrose ester - GV - I0; Type I sucrose ester - GIV - I0; Type I sucrose ester - Gill - I0; 16-hydroxy-hexadecanoicacid; Type III sucrose ester - GV - I0; 3-ethyl-2-hydroxy-2-cyclopenten-1-one; 5-methyl-2-furancarboxaldehyde; propanoic acid; 3-methyl-pyridine; butanoic acid; 3-ethyl-pyridine; trimethyl-pyrazine; benzaldehyde; tiglic acid; 2-hydroxy-3-methyl-2-cyclopenten-1-one; acetophenone; 2-ethyl-phenol; 2-methoxy-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; octanoic acid; and 2,6-dimethoxy-phenol. In this embodiment the composition may comprise or be a tobacco extract, which may be obtained using supercritical fluid extraction (SFE) and/or which may be an extract of a blend of flue-cured Virginia, air-cured burley and sun-cured oriental tobacco.

In embodiments, the composition comprises at least nicotine; quinic acid; sucrose; N-(1-deoxy-1-fructosyl)proline; 3,4-dimethyl-2(3H)-furanone; Pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; rutin; 2-methyl-3-butyl-pyrazine; megastigmatrienone; L-alpha-amino-1H-pyrrole-1-hexanoic acid; solanesol; 3-methyl-pentanoic acid; 3-methyl-butanoic acid; 1-(4-methylphenyl)-ethanone; benzeneacetic acid; 2-methyl-butanoic acid; pentanoic acid; hexanoic acid; acetic acid; 2-methyl-propanoic acid; 3-ethyl-2-hydroxy-2-cyclopenten-1-one; 5-methyl-2-furancarboxaldehyde; propanoic acid; 3-methyl-pyridine; butanoic acid; 3-ethyl-pyridine; trimethyl-pyrazine; benzaldehyde; tiglic acid; 2-hydroxy-3-methyl-2-cyclopenten-1-one; acetophenone; 2-ethyl-phenol; 2-methoxy-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; octanoic acid; and 2,6-dimethoxy-phenol. In this embodiment the composition may comprise or be a tobacco extract, which may be obtained using supercritical fluid extraction (SFE) and/or which may be an extract of a blend of flue-cured Virginia, air-cured burley and sun-cured oriental tobacco.

In embodiments, the composition comprises at least nicotine; 3,4-dimethyl-2(3H)-furanone; pseudooxynicotine and/or oxynicotine; 1,2,3,4,5,6-hexahydro-7H-cyclopenta[b]pyridin-7-one; 2-ethyl-pyridine; scopoletin; 2-ethyl-5-methyl-pyridine; retinol; 2-methyl-3-butyl-pyrazine; megastigmatrienone; solanesol; Type I sucrose ester - GV - I0; Type I sucrose ester - GIV - I0; Type I sucrose ester - Gill - I0; 16-hydroxy-hexadecanoicacid; Type III sucrose ester - GV - I0; 3-methyl-pentanoic acid; 3-methyl-butanoic acid; 1-(4-methylphenyl)-ethanone; benzeneacetic acid; 2-methyl-butanoic acid; pentanoic acid; hexanoic acid; acetic acid; 2-methyl-propanoic acid; 3-ethyl-2-hydroxy-2-cyclopenten-1-one; 5-methyl-2-furancarboxaldehyde; propanoic acid; 3-methyl-pyridine; butanoic acid; 3-ethyl-pyridine; trimethyl-pyrazine; benzaldehyde; tiglic acid; 2-hydroxy-3-methyl-2-cyclopenten-1-one; acetophenone; 2-ethyl-phenol; 2-methoxy-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; octanoic acid; and 2,6-dimethoxy-phenol. In this embodiment the composition may comprise or be a tobacco extract, which may be obtained using supercritical fluid extraction (SFE) and/or which may be an extract of a blend of flue-cured Virginia, air-cured burley and sun-cured oriental tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; and L-alpha-amino-1H-pyrrole-1-hexanoic acid. In this embodiment the composition may comprise or be a tobacco extract, which may be obtained using simultaneous distillation extraction (SDE) and/or which may be an extract of flue-cured Virginia tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; and Type III sucrose ester - GV - I0. In this embodiment the composition may comprise or be a tobacco extract, which may be obtained using simultaneous distillation extraction (SDE) and/or which may be an extract of flue-cured Virginia tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; 2-methoxy-phenol; 3-methyl-butanoic acid; D-limonene; 1-(4-methylphenyl)-ethanone; benzaldehyde; 2-methyl-butanoic acid; acetic acid; and octanoic acid. In this embodiment the composition may comprise or be a tobacco extract, which may be obtained using simultaneous distillation extraction (SDE) and/or which may be an extract of flue-cured Virginia tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; 3-ethyl-2-hydroxy-2-cyclopenten-1-one; 3-methyl-pyridine; butanoic acid; 3-ethyl-pyridine; tiglic acid; 2-hydroxy-3-methyl-2-cyclopenten-1-one; acetophenone; 2-ethyl-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; 2,6-dimethoxy-phenol; 2-methyl-propanoic acid; vanillin; pentanoic acid; benzoic acid; triacetin; benzeneacetic acid; maltol; and nicotine. In this embodiment the composition may comprise or be a tobacco extract, which may be obtained using simultaneous distillation extraction (SDE) and/or which may be an extract of flue-cured Virginia tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; Type III sucrose ester - GV - I0; 2-methoxy-phenol; 3-methyl-butanoic acid; D-limonene; 1-(4-methylphenyl)-ethanone; benzaldehyde; 2-methyl-butanoic acid; acetic acid; and octanoic acid. In this embodiment the composition may comprise or be a tobacco extract, which may be obtained using simultaneous distillation extraction (SDE) and/or which may be an extract of flue-cured Virginia tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; Type III sucrose ester - GV - I0; 3-ethyl-2-hydroxy-2-cyclopenten-1-one; 3-methyl-pyridine; butanoic acid; 3-ethyl-pyridine; tiglic acid; 2-hydroxy-3-methyl-2-cyclopenten-1-one; acetophenone; 2-ethyl-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; 2,6-dimethoxy-phenol; 2-methyl-propanoic acid; vanillin; pentanoic acid; benzoic acid; triacetin; benzeneacetic acid; maltol; and nicotine. In this embodiment the composition may comprise or be a tobacco extract, which may be obtained using simultaneous distillation extraction (SDE) and/or which may be an extract of flue-cured Virginia tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; 2-methoxy-phenol; 3-methyl-butanoic acid; D-limonene; 1-(4-methylphenyl)-ethanone; benzaldehyde; 2-methyl-butanoic acid; acetic acid; octanoic acid; 3-ethyl-2-hydroxy-2-cyclopenten-1-one; 3-methyl-pyridine; butanoic acid; 3-ethyl-pyridine; tiglic acid; 2-hydroxy-3-methyl-2-cyclopenten-1-one; acetophenone; 2-ethyl-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; 2,6-dimethoxy-phenol; 2-methyl-propanoic acid; vanillin; pentanoic acid; benzoic acid; triacetin; benzeneacetic acid; maltol; and nicotine. In this embodiment the composition may comprise or be a tobacco extract, which may be obtained using simultaneous distillation extraction (SDE) and/or which may be an extract of flue-cured Virginia tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; Type III sucrose ester - GV - I0; 2-methoxy-phenol; 3-methyl-butanoic acid; D-limonene; 1-(4-methylphenyl)-ethanone; benzaldehyde; 2-methyl-butanoic acid; acetic acid; octanoic acid; 3-ethyl-2-hydroxy-2-cyclopenten-1-one; 3-methyl-pyridine; butanoic acid; 3-ethyl-pyridine; tiglic acid; 2-hydroxy-3-methyl-2-cyclopenten-1-one; acetophenone; 2-ethyl-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; 2,6-dimethoxy-phenol; 2-methyl-propanoic acid; vanillin; pentanoic acid; benzoic acid; triacetin; benzeneacetic acid; maltol; and nicotine. In this embodiment the composition may comprise or be a tobacco extract, which may be obtained using simultaneous distillation extraction (SDE) and/or which may be an extract of flue-cured Virginia tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; and L-alpha-amino-1H-pyrrole-1-hexanoic acid. In this embodiment the composition may comprise or be a tobacco extract, which may be obtained using simultaneous distillation extraction (SDE) and/or which may be an extract of a blend of flue-cured Virginia, air-cured burley and sun-cured oriental tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; Type I sucrose ester - GV - I0; and Type III sucrose ester - GV - I0. In this embodiment the composition may comprise or be a tobacco extract, which may be obtained using simultaneous distillation extraction (SDE) and/or which may be an extract of a blend of flue-cured Virginia, air-cured burley and sun-cured oriental tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; 2-methoxy-phenol; 3-methyl-pentanoic acid; 3-methyl-butanoic acid; 1-(4-methylphenyl)-ethanone; benzaldehyde; 2-methyl-butanoic acid; pentanoic acid; hexanoic acid; acetic acid; 2-methyl-propanoic acid; and octanoic acid. In this embodiment the composition may comprise or be a tobacco extract, which may be obtained using simultaneous distillation extraction (SDE) and/or which may be an extract of a blend of flue-cured Virginia, air-cured burley and sun-cured oriental tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; 3-ethyl-2-hydroxy-2-cyclopenten-1-one; 3-methyl-pyridine; butanoic acid; 3-ethyl-pyridine; trimethyl-pyrazine; tiglic acid; 2-hydroxy-3-methyl-2-cyclopenten-1-one; acetophenone; 2-ethyl-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; 2,6-dimethoxy-phenol; benzoic acid; triacetin; benzeneacetic acid; maltol; and nicotine. In this embodiment the composition may comprise or be a tobacco extract, which may be obtained using simultaneous distillation extraction (SDE) and/or which may be an extract of a blend of flue-cured Virginia, air-cured burley and sun-cured oriental tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; Type I sucrose ester - GV - I0; Type III sucrose ester - GV - I0; 2-methoxy-phenol; 3-methyl-pentanoic acid; 3-methyl-butanoic acid; 1-(4-methylphenyl)-ethanone; benzaldehyde; 2-methyl-butanoic acid; pentanoic acid; hexanoic acid; acetic acid; 2-methyl-propanoic acid; and octanoic acid. In this embodiment the composition may comprise or be a tobacco extract, which may be obtained using simultaneous distillation extraction (SDE) and/or which may be an extract of a blend of flue-cured Virginia, air-cured burley and sun-cured oriental tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; Type I sucrose ester - GV - I0; Type III sucrose ester - GV - I0; 3-ethyl-2-hydroxy-2-cyclopenten-1-one; 3-methyl-pyridine; butanoic acid; 3-ethyl-pyridine; trimethyl-pyrazine; tiglic acid; 2-hydroxy-3-methyl-2-cyclopenten-1-one; acetophenone; 2-ethyl-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; 2,6-dimethoxy-phenol; benzoic acid; triacetin; benzeneacetic acid; maltol; and nicotine. In this embodiment the composition may comprise or be a tobacco extract, which may be obtained using simultaneous distillation extraction (SDE) and/or which may be an extract of a blend of flue-cured Virginia, air-cured burley and sun-cured oriental tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; 2-methoxy-phenol; 3-methyl-pentanoic acid; 3-methyl-butanoic acid; 1-(4-methylphenyl)-ethanone; benzaldehyde; 2-methyl-butanoic acid; pentanoic acid; hexanoic acid; acetic acid; 2-methyl-propanoic acid; octanoic acid; 3-ethyl-2-hydroxy-2-cyclopenten-1-one; 3-methyl-pyridine; butanoic acid; 3-ethyl-pyridine; trimethyl-pyrazine; tiglic acid; 2-hydroxy-3-methyl-2-cyclopenten-1-one; acetophenone; 2-ethyl-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; 2,6-dimethoxy-phenol; benzoic acid; triacetin; benzeneacetic acid; maltol; and nicotine. In this embodiment the composition may comprise or be a tobacco extract, which may be obtained using simultaneous distillation extraction (SDE) and/or which may be an extract of a blend of flue-cured Virginia, air-cured burley and sun-cured oriental tobacco.

In embodiments, the composition comprises at least 3,4-dimethyl-2(3H)-furanone; pseudooxynicotine and/or oxynicotine; retinol; 2-methyl-3-butyl-pyrazine; megastigmatrienone; L-alpha-amino-1H-pyrrole-1-hexanoic acid; Type I sucrose ester - GV - I0; Type III sucrose ester - GV - I0; 2-methoxy-phenol; 3-methyl-pentanoic acid; 3-methyl-butanoic acid; 1-(4-methylphenyl)-ethanone; benzaldehyde; 2-methyl-butanoic acid; pentanoic acid; hexanoic acid; acetic acid; 2-methyl-propanoic acid; octanoic acid; 3-ethyl-2-hydroxy-2-cyclopenten-1-one; 3-methyl-pyridine; butanoic acid; 3-ethyl-pyridine; trimethyl-pyrazine; tiglic acid; 2-hydroxy-3-methyl-2-cyclopenten-1-one; acetophenone; 2-ethyl-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; 2,6-dimethoxy-phenol; benzoic acid; triacetin; benzeneacetic acid; maltol; and nicotine. In this embodiment the composition may comprise or be a tobacco extract, which may be obtained using simultaneous distillation extraction (SDE) and/or which may be an extract of a blend of flue-cured Virginia, air-cured burley and sun-cured oriental tobacco.

### Key smoke markers

In some embodiments (including all those described above), the compositions may further comprise 1 or more (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32 or 33) of the following compounds: 3-ethyl-2-hydroxy-2-cyclopenten-1-one; 5-methyl-2-furancarboxaldehyde; propanoic acid; 3-methyl-pyridine; butanoic acid; 3-ethyl-pyridine; trimethyl-pyrazine; benzaldehyde; tiglic acid; 2-hydroxy-3-methyl-2-cyclopenten-1-one; acetophenone; 2-ethyl-phenol; 2-methoxy-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; octanoic acid; 2,6-dimethoxy-phenol; 2-methyl-propanoic acid; 3-methyl-butanoic acid; D-limonene; 2-acetyl-5-methylfuran; 1-(4-methylphenyl)-ethanone; vanillin; pentanoic acid; benzoic acid; triacetin; benzeneacetic acid; maltol; and nicotine.

In some embodiments (including all those described above), the compositions may further comprise 1 or more (e.g. 1, 2, 3, 4, 5, 6, 7, 8, 9, 10, 11, 12, 13, 14, 15, 16 or 17) of the following compounds: 3-ethyl-2-hydroxy-2-cyclopenten-1-one; 5-methyl-2-furancarboxaldehyde; propanoic acid; 3-methyl-pyridine; 3-ethyl-pyridine; trimethyl-pyrazine; tiglic acid; 2-hydroxy-3-methyl-2-cyclopenten-1-one; 2-ethyl-phenol; 4-ethyl-phenol; 3-ethyl-phenol; 1-(1H-pyrrol-2-yl)-ethanone; 2-methoxy-4-vinylphenol; 2-acetyl-5-methylfuran; benzoic acid; triacetin; and maltol.

In some embodiments (including all those described above), the compositions may further comprise 1 or more (e.g. 1, 2, 3, 4, 5, 6, 7, 8, 9, 10, 11, 12, 13, 14, 15, 16, 17, 18, 19, 20, 21 or 22) of the following compounds: 3-ethyl-2-hydroxy-2-cyclopenten-1-one; 3-methyl-pyridine; butanoic acid; 3-ethyl-pyridine; benzaldehyde; 2-hydroxy-3-methyl-2-cyclopenten-1-one; acetophenone; 2-ethyl-phenol; 2-methoxy-phenol; furaneol; 4-ethyl-phenol; 3-ethyl-phenol; 2-methoxy-4-vinylphenol; 4-ethyl-2-methoxy-phenol; octanoic acid; 2,6-dimethoxy-phenol; 2-methyl-propanoic acid; 3-methyl-butanoic acid; 1-(4-methylphenyl)-ethanone; vanillin; pentanoic acid; and benzeneacetic acid.

In some embodiments (including all those described above), the compositions may further comprise 1 or more (e.g. 1, 2, 3, 4, 5, 6, 7, 8, 9, 10, 11, 12 or 13) of the following compounds: 3-ethyl-2-hydroxy-2-cyclopenten-1-one; 3-methyl-pyridine; butanoic acid; 3-ethyl-pyridine; 2-hydroxy-3-methyl-2-cyclopenten-1-one; acetophenone; 2-ethyl-phenol; furaneol; 4-ethyl-phenol; 3-ethyl-phenol; 2-methoxy-4-vinylphenol; 4-ethyl-2-methoxy-phenol; and 2,6-dimethoxy-phenol.

In some embodiments (including all those described above), the compositions may further comprise 1 or more (e.g. 1, 2, 3, 4, 5, 6, 7 or 8) of the following compounds: 3-ethyl-2-hydroxy-2-cyclopenten-1-one; 3-methyl-pyridine; 3-ethyl-pyridine; 2-hydroxy-3-methyl-2-cyclopenten-1-one; 2-ethyl-phenol; 4-ethyl-phenol; 3-ethyl-phenol; and 2-methoxy-4-vinylphenol.

### Synthetic composition

The present invention provides for the formulation of a partially or totally synthetic or artificial composition. In this case, at least one of the key sense markers may be a purified, isolated or synthetic compound. Such a compound may be obtained from a natural source (e.g. from tobacco), but before being added to the composition it will first have been purified or isolated. The compound(s) may also be available commercially.

By "purified compound" it is meant a compound which, prior to being incorporated into the composition, has a purity of at least about 80 wt%, such as at least about 85 wt%, at least about 90 wt%, at least about 95 wt%, at least about 99 wt%, or at least about 99.5 wt%. Such a compound may have been bought (e.g. because it is commercially available), synthesised, or isolated from a natural or naturally-obtained mixture of compounds. By way of example, some of the chemical markers described herein (such as nicotine) can be isolated from a tobacco extract.

In embodiments the claimed composition is not an extract of tobacco. In embodiments the composition does not comprise or contain a tobacco extract.

In embodiments at least 1 (e.g. at least 2, 3, 4, 5, 6, 7, 8, 9, 10, 11, 12, 13, 14, 15, 16, 17, 18, 19 or 20) of the key sense markers is a purified, isolated or synthetic compound.

In embodiments all of the key sense markers are purified, isolated or synthetic compounds.

In embodiments, if present at least 1 (e.g. at least 2, 3, 4, 5, 6, 7, 8, 9, 10, 11, 12, 13, 14, 15, 16, 17, 18, 19 or 20) of the key taste and/or flavour markers is a purified, isolated or synthetic compound.

In embodiments, if present at least 1 (e.g. at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32 or 33) of the key smoke markers is a purified, isolated or synthetic compound.

In embodiments, at least about 10%, at least about 20%, at least about 25%, at least about 30%, at least about 50%, at least about 60%, or at least about 75% of the key chemical markers included in the composition are purified compounds.

In embodiments, less than about 90%, less than about 80%, less than about 60%, less than about 50%, less than about 20% or less than about 10% of the key chemical markers included in the composition are purified compounds.

In embodiments, from about 10% to about 90% of the key chemical markers included in the composition is a purified, isolated or synthetic compound. In embodiments, from about 10% to about 50% of the key chemical markers included in the composition is a purified, isolated or synthetic compound. In embodiments, from about 10% to about 30% of the key chemical markers included in the composition is a purified, isolated or synthetic compound. In embodiments, from about 10% to about 20% of the key chemical markers included in the composition is a purified, isolated or synthetic compound.

Note that numbered of markers obtained from percentages should be rounded up to the nearest whole number when a ranger refers to "at least" a certain percentage, and down to the nearest whole number when a ranger refers to "less than" a certain percentage. For example, at least 10% of 4 chemical markers would mean 1 or more, and less than 80% of 8 chemical markers would mean 6 or less.

In embodiments, at least 1 (e.g. at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, or 93) of the key chemical markers included in the composition is a purified, isolated or synthetic compound.

In embodiments, exactly 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91 92 or 93 of the key chemical markers included in the composition is a purified, isolated or synthetic compound.

In embodiments all of the key taste markers which are present are purified, isolated or synthetic compounds.

In embodiments all of the key sense markers which are present are purified, isolated or synthetic compounds.

In embodiments all of the key flavour markers which are present are purified, isolated or synthetic compounds.

In embodiments, all of the key smoke markers which are present are purified, isolated or synthetic compounds.

In embodiments all of the key sense and taste markers which are present are purified, isolated or synthetic compounds.

In embodiments all of the key sense and flavour markers which are present are purified, isolated or synthetic compounds.

In embodiments all of the key sense and smoke markers which are present are purified, isolated or synthetic compounds.

In embodiments all of the key taste and flavour markers which are present are purified, isolated or synthetic compounds.

In embodiments all of the key taste and smoke markers which are present are purified, isolated or synthetic compounds.

In embodiments all of the key flavour and smoke markers which are present are purified, isolated or synthetic compounds.

In embodiments all of the key sense, taste and flavour markers which are present are purified, isolated or synthetic compounds.

In embodiments all of the key sense, taste and smoke markers which are present are purified, isolated or synthetic compounds.

In embodiments all of the key sense, flavour and smoke markers which are present are purified, isolated or synthetic compounds.

In embodiments all of the key taste, flavour and smoke markers which are present are purified, isolated or synthetic compounds.

In embodiments all of the key sense, taste, flavour and smoke markers which are present are purified, isolated or synthetic compounds.

The total number of compounds present in the composition (or any material comprising or formed from the composition) can also be an indication of whether the composition is synthetic or naturally-derived. This is because tobacco extracts will often contain a large number of different chemical compounds. Any composition comprising a tobacco extract will often also contain a large number of different chemical compounds.

In embodiments the total number of chemical compounds present in the compositions or materials described herein is less than about 500, such as less than about 300, less than about 200, less than about 150, less than about 100, less than about 80 or less than about 60. By total number of chemical compounds it is meant the number of different chemical compounds present at a detectable level. In embodiments the total number of chemical compounds means the number of different chemical compounds present at a level of 1 ppm or more. By way of example only, a composition comprising (at least 1 ppm of) glycerol, propylene glycol, and all of the key sense markers described herein would contain 22 chemical compounds. In contrast, a composition comprising glycerol, propylene glycol and a commercially available tobacco extract might contain hundreds of different chemical compounds (e.g. more than 500, more than 300 or more than 200), each of which may be present in the amount of at least 1 ppm.

Similarly, the amount of tobacco-specific nitrosamines (TSNAs) present in the composition (or any material comprising or formed from the composition) can also be an indication of whether the composition is synthetic or naturally-derived. This is because such compounds might be present in tobacco extracts, but would not be specifically added to a (synthetic) composition. As used herein, the term TSNA includes N-nitrosonornicotine (NNN), N'-nitrosoanatabine (NAT), N-nitrosoanabasine (NAB) and nicotine-derived nitrosamine ketone (NNK).

In embodiments the total content of any one of NNN, NAT, NAB and NNK in the compositions or materials described herein is less than about 800 ppb, less than about 500 ppb, less than about 200 ppb, less than about 100 ppb, less than about 50 ppb, less than about 20 ppb, or less than about 10 ppb. In embodiments the total content of any one of NNN, NAT, NAB and NNK in the compositions or materials described herein is less than about 1 ppb.

In embodiments the total TSNA content (i.e. the content of NNN, NAT, NAB and NNK) of the compositions or materials described herein is less than about 800 ppb, less than about 500 ppb, less than about 200 ppb, less than about 100 ppb, less than about 50 ppb, less than about 20 ppb, or less than about 10 ppb. In embodiments the total TSNA content of the compositions or materials described herein is less than about 1 ppb. In embodiments there are no TSNAs (i.e. no NNN, NAT, NAB and NNK) in the compositions or materials described herein.

Other chemical compounds indicative of non-synthetic tobacco extracts might include formaldehyde and/or acetaldehyde. In embodiments the total content of formaldehyde and acetaldehyde in the compositions or materials described herein is less than about 100 ppm, less than about 50 ppm, less than about 10 ppm, less than about 5 ppm, less than about 2 ppm, or less than about 1 ppm. In embodiments there is no formaldehyde and/or acetaldehyde in the compositions or materials described herein.

### Tobacco extract

In embodiments, the composition comprises a tobacco extract. In this case, 1 or more of the key chemical markers described herein may be present in or provided from the extract. That is, any tobacco extract used in the present invention may comprise 1 or more (e.g. 2 or more, 3 or more, etc.) of the key chemical markers, such as the key sense markers, described herein. The disclosures made herein in relation to the number and type of chemical markers present in the overall composition also apply equally to a tobacco extract comprising said markers.

In other embodiments a tobacco extract does not comprise any of the key chemical markers described herein, in which case these markers must be added to the extract to form a composition of the invention.

In embodiments a tobacco extract does not comprise any of the key sense markers described herein, in which case these markers must be added to the extract to form a composition of the invention. The extract may, however, comprise one or more of the key taste, flavour and/or smoke markers.

In embodiments a tobacco extract comprises some, but not all, of the key sense markers described herein, in which case some or all of the missing sense markers may be added to the extract or composition. The extract may also comprise one or more of the key flavour, taste and/or smoke markers.

In embodiments, the composition comprises a tobacco extract and at least one further compound, wherein said further compound(s) is one of the key chemical markers described herein. The further compound(s) or key marker(s) is not otherwise present in the tobacco extract (i.e. it must be added to the extract).

In embodiments, the composition comprises a tobacco extract and at least 1 (such as at least 2, 3, 4 or 5) further compound(s) from List 1. In embodiments, the composition comprises a tobacco extract and at least 1 (such as at least 2, 3, 4 or 5) further compound(s) from List 2. In embodiments, the composition comprises a tobacco extract and at least 1 (such as at least 2, 3, 4 or 5) further compound(s) from List 3. In embodiments, the composition comprises a tobacco extract and at least 1 (such as at least 2, 3, 4 or 5) further compound(s) from List 4.

In embodiments, the composition comprises a tobacco extract and at least 1 (such as at least 2, 3, 4 or 5) further compound(s) from each of Lists 1 and 2. In embodiments, the composition comprises a tobacco extract and at least 1 (such as at least 2, 3, 4 or 5) further compound(s) from each of Lists 1 and 3. In embodiments, the composition comprises a tobacco extract and at least 1 (such as at least 2, 3, 4 or 5) further compound(s) from each of Lists 1 and 4. In embodiments, the composition comprises a tobacco extract and at least 1 (such as at least 2, 3, 4 or 5) further compound(s) from each of Lists 2 and 3. In embodiments, the composition comprises a tobacco extract and at least 1 (such as at least 2, 3, 4 or 5) further compound(s) from each of Lists 2 and 4. In embodiments, the composition comprises a tobacco extract and at least 1 (such as at least 2, 3, 4 or 5) further compound(s) from each of Lists 3 and 4.

In embodiments, the composition comprises a tobacco extract and at least 1 (such as at least 2, 3, 4 or 5) further compound(s) from each of Lists 1, 2 and 3. In embodiments, the composition comprises a tobacco extract and at least 1 (such as at least 2, 3, 4 or 5) further compound(s) from each of Lists 1, 2 and 4. In embodiments, the composition comprises a tobacco extract and at least 1 (such as at least 2, 3, 4 or 5) further compound(s) from each of Lists 1, 3 and 4. In embodiments, the composition comprises a tobacco extract and at least 1 (such as at least 2, 3, 4 or 5) further compound(s) from each of Lists 2, 3 and 4.

In embodiments, the composition comprises a tobacco extract and at least 1 (such as at least 2, 3, 4 or 5) further compound(s) from each of Lists 1, 2, 3 and 4.

In each of the above embodiments relating to an extract it is not necessary for the overall number of key sense markers to be at least 3, although this will usually be the case. These key sense markers may be added to a tobacco extract, or may be present in the given tobacco extract.

In each case, said further compound(s) may be isolated or purified compounds.

In embodiments, the composition is a tobacco extract.

In embodiments any tobacco extract used in the present invention is an extract of Virginia tobacco, burley tobacco, oriental tobacco, or a combination thereof. In embodiments any tobacco extract used in the present invention is an extract of Virginia tobacco.

The tobacco may be cured or uncured, but in embodiments the tobacco is cured. Cured tobacco may be flue-cured, air-cured and/or sun-cured.

In embodiments any tobacco extract used in the present invention is an extract of cured Virginia, cured burley and cured oriental tobacco, or a combination thereof.

In embodiments any tobacco extract used in the present invention is an extract of flue-cured Virginia, air-cured burley and sun-cured oriental tobacco, or a combination thereof.

In embodiments any tobacco extract used in the present invention is an extract of cured Virginia tobacco.

In embodiments any tobacco extract used in the present invention is an extract of flue-cured Virginia tobacco.

In embodiments any tobacco extract used in the present invention is an extract of a blend of flue-cured Virginia, air-cured burley and sun-cured oriental tobacco.

In embodiments any tobacco extract used in the present invention is obtained by supercritical fluid extraction (SFE).

In embodiments any tobacco extract used in the present invention is obtained by simultaneous distillation extraction (SDE).

In embodiments any tobacco extract used in the present invention is an extract of flue-cured Virginia tobacco and is obtained by supercritical fluid extraction (SFE).

In embodiments any tobacco extract used in the present invention is an extract of a blend of flue-cured Virginia, air-cured burley and sun-cured oriental tobacco and is obtained by supercritical fluid extraction (SFE).

### Aerosol-generating composition

In embodiments, the composition is an aerosol-generating composition.

In embodiments, the aerosol-generating composition is incorporated into or formed into an aerosol-generating material. As used herein, the term aerosol-generating material in some embodiments refers to a solid material or a semi-solid material (e.g. gel).

The aerosol-generating materials and compositions described herein are materials and compositions that are capable of generating aerosol, for example when heated, irradiated or energized in any other way.

The aerosol-generating material may be an "amorphous solid". In some embodiments, the aerosol-generating material comprises an aerosol-generating film that is an amorphous solid. In some embodiments, the amorphous solid is a "monolithic solid". The aerosol-generating material may be non-fibrous or fibrous. For example, the aerosol-generating material may be substantially non-fibrous. In some embodiments, the aerosol-generating material may be a dried gel.

The aerosol-generating material may be a solid material that may retain some fluid, such as liquid, within it. In some embodiments the retained fluid may be water (such as water absorbed from the surroundings of the materials) or the retained fluid may be solvent (such as when the first material (and optionally also the second material) is formed from a slurry). In some embodiments, the solvent may be water.

In some embodiments, the aerosol-generating material is a hydrogel. In some embodiments the aerosol-generating material comprises less than about 20 wt% of water calculated on a wet weight basis, such as less than about 15 wt%, 12 wt% or 10 wt%. In some embodiments the aerosol-generating composition contains from about 1 to about 15 wt% water, such as from about 3 to about 12 wt% water (WWB).

### Liquid composition

The composition described herein may be a liquid composition.

In addition to the components of the composition defined elsewhere herein (e.g. the key chemical markers described herein), the liquid composition may comprise a number of other components well-known in the art of electronic cigarettes, such as water, a liquid carrier such as glycerol and/or propylene glycol, and nicotine (if not already part of the composition). The amounts of these components can generally be varied.

Extra or additional nicotine (e.g. to increase the level to amounts known to be used in the art) may also be included in the composition even if it is already present in the composition as a key marker.

The liquid composition solution may also comprise one or more active and/or flavouring components, as will be described in more detail below in relation to the composition in general.

The liquid composition may comprise any suitable solvent or liquid carrier such that the liquid composition can be vaporized for use. In one embodiment the liquid carrier is selected from glycerol, propylene glycol and mixtures thereof.

In one embodiment the liquid carrier comprises glycerol. In one embodiment the liquid carrier consists essentially of glycerol. In one embodiment the solvent consists of glycerol.

In one embodiment the liquid carrier comprises propylene glycol. In one embodiment the liquid carrier consists essentially of propylene glycol. In one embodiment the liquid carrier consists of propylene glycol.

In one embodiment the liquid carrier comprises a mixture of propylene glycol and glycerol. In one embodiment the liquid carrier consists essentially of a mixture of propylene glycol and glycerol. In one embodiment the liquid carrier consists of a mixture of propylene glycol and glycerol.

The liquid carrier may be present in any suitable amount. In one embodiment the liquid carrier is present in an amount of from about 1 wt%, 5 wt%, 10 wt%, 20 wt%, 30 wt%, 40 wt%, 50 wt%, 60 wt%, 70 wt%, 80 wt% or 90 wt% to about 99 wt %, 98 wt%, 97 wt%, 95 wt%, 93 wt%, or 90 wt%, based on the overall composition.

In one embodiment the liquid carrier is present in an amount of from about 1 to about 99 wt % based on the overall composition, such as from about 30 wt% to about 98 wt%, from about 50 to about 98 wt%, from about 60 to about 97 wt% or from about 70 to about 95 wt%.

In some embodiments the liquid composition further comprises water. The water may be present in any suitable amount. In one embodiment water is present in an amount of from about 1 wt%, 5 wt%, 10 wt%, 15 wt%, 20 wt% or 22 wt% to about 50 wt%, 40 wt%, 30 wt% or 27 wt%, based on the overall composition.

In some embodiments the liquid composition comprises from about 1 wt% to about 50 wt% water, such as from about 15 wt% to about 40 wt%, from about 20 wt% to about 30 wt%, from about 22 wt% to about 27 wt% or about 25 wt%.

In some embodiments the combined amount of liquid carrier and water in the liquid composition is from about 1 wt%, 5 wt%, 10 wt%, 20 wt%, 30 wt%, 40 wt%, 50 wt%, 60 wt%, 70 wt%, 80 wt% or 90 wt% to about 99 wt %, 98 wt%, 97 wt%, 95 wt%, 93 wt%, or 90 wt%, based on the overall composition.

In one embodiment the combined amount of liquid carrier and water in the liquid composition is from about 50 to about 99 wt % based on the overall composition, such as from about 40 wt% to about 98 wt%, from about 60 to about 98 wt%, from about 70 to about 97 wt% or from about 80 to about 95 wt%.

In some embodiments, the liquid composition comprises from about 10 to about 80 wt% glycerol, such as from about 20 to about 70 wt%, from about 30 to about 60 wt% or from about 40 to about 60 wt%.

In some embodiments, the liquid composition comprises from about 10 to about 50 wt% propylene glycol, such as from about 15 to about 40 wt%, or from about 20 to about 35 wt%

In some embodiments, the liquid composition comprises up to about 5 wt.% nicotine, such as up to about 4 wt% or up to about 3 wt%.

In some embodiments, the liquid composition comprises from about 20 to about 70 wt% glycerol, from about 10 to about 50 wt% propylene glycol, and 0 to about 5 wt% nicotine.

In some embodiments, the liquid composition comprises from about 30 to about 60 wt% glycerol, from about 15 to about 40 wt% propylene glycol, and 0 to about 4 wt% nicotine.

In some embodiments, the liquid composition comprises from about 40 to about 60 wt% glycerol, from about 20 to about 35 wt% propylene glycol, and 0 to about 3 wt% nicotine.

### Aerosol-former material

In embodiments, the aerosol-generating composition or the aerosol-generating material may further comprise an aerosol-former material. The aerosol-former material may comprise one or more constituents capable of forming an aerosol.

In some embodiments, the aerosol-former material comprises one or more of glycerol, propylene glycol, diethylene glycol, triethylene glycol, tetraethylene glycol, 1,3-butylene glycol, erythritol, meso-Erythritol, ethyl vanillate, ethyl laurate, a diethyl suberate, triethyl citrate, triacetin, a diacetin mixture, benzyl benzoate, benzyl phenyl acetate, tributyrin, lauryl acetate, lauric acid, myristic acid, and propylene carbonate.

In some embodiments, the aerosol-former material comprises one or more polyhydric alcohols, such as propylene glycol, triethylene glycol, 1,3-butanediol and glycerin; esters of polyhydric alcohols, such as glycerol mono-, di- or triacetate; and/or aliphatic esters of mono, di- or polycarboxylic acids, such as dimethyl dodecanedioate and dimethyl tetradecanedioate.

In some embodiments, the aerosol-former material comprises glycerol in combination with propylene glycol. In some embodiments, the aerosol-former material comprises glycerol.

In some embodiments, the aerosol-generating composition or the aerosol-generating material comprises from about 1 wt%, 5 wt%, 10 wt%, 20 wt%, 30 wt% or 40 wt% to about 60 wt%, 55 wt%, 50 wt%, 40 wt%, 30 wt%, 20 wt% or 15 wt% of aerosol-former material.

In some embodiments, the aerosol-generating composition or the aerosol-generating material comprises from about 1 to about 60 wt%, from about 5 to about 50 wt% or from about 10 to about 30 wt% aerosol-former material.

In some embodiments, the aerosol-generating composition or the aerosol-generating material comprises from about 20 to about 40 wt% or from about 25 to about 35 wt% aerosol-former material, such as about 30 wt%.

In some embodiments, the aerosol-generating composition or the aerosol-generating material comprises from about 40 to about 60 wt% or from about 45 to about 55 wt% aerosol-former material, such as about 50 wt%.

### Binder

In embodiments, the aerosol-generating composition or the aerosol-generating material may further comprise a binder. In some embodiments, the binder comprises one or more compounds selected from polysaccharide binders, such as alginate, pectin, cellulose or a derivative thereof, pullulan, carrageenan, agar and agarose; non-galactomannan gums, such as xanthan gum, and acacia gum; silica or silicone compounds, such as PDMS and sodium silicate; clays, such as kaolin; and polyvinyl alcohol.

In some embodiments the binder comprises one or more polysaccharide binders. In some embodiments, the binder is selected from alginate, pectin, and cellulose or a derivative thereof.

In some embodiments, alginate is the only binder present in the aerosol-generating material.

In other embodiments, the gelling agent comprises a combination of binders, such as alginate and at least one further binder (e.g. pectin, cellulose or a cellulose derivative).

Examples of cellulosic binders (also referred to herein as cellulose derivatives) include, but are not limited to, hydroxymethyl cellulose, hydroxyethyl cellulose, hydroxypropyl cellulose, carboxymethylcellulose (CMC), hydroxypropyl methylcellulose (HPMC), methyl cellulose, ethyl cellulose, cellulose acetate (CA), cellulose acetate butyrate (CAB), and cellulose acetate propionate (CAP). In some embodiments the cellulose or derivative thereof is selected from hydroxymethyl cellulose, hydroxyethyl cellulose, hydroxypropyl cellulose, carboxymethylcellulose (CMC), hydroxypropyl methylcellulose (HPMC), methyl cellulose, ethyl cellulose, cellulose acetate (CA), cellulose acetate butyrate (CAB), and cellulose acetate propionate (CAP). In particular embodiments, the cellulose derivative is CMC.

In some embodiments, the aerosol-generating composition or the aerosol-generating material comprises from about 1 wt%, 5 wt%, 10, 15 or 20 wt% to about 80 wt%, 70 wt%, 50, 40, 30 or 20 wt% binder. For example, in some embodiments the aerosol-generating composition or the aerosol-generating material comprises from about 1 to about 80 wt%, from about 5 to about 70 wt%, or from about 10 to about 50 wt% binder.

### Filler

The aerosol-generating composition or aerosol-generating material may comprise a filler. Use of a filler may help to reduce tackiness of the aerosol-generating material, for example if high levels of aerosol-former material are present.

In some embodiments, the aerosol-generating composition or aerosol-generating material comprises filler in a total amount of about 1 to 80 wt% on a dry weight basis. For example, in some embodiments, the aerosol-generating composition or aerosol-generating material comprises from about 30 to about 70 wt% filler, or from about 40 to about 60 wt% filler.

In some embodiments, the aerosol-generating composition or aerosol-generating material comprises less than about 50 wt% of a filler, such as from about 1 wt% to 50 wt%, or 5 wt% to 40 wt%, or 5 wt% to 30 wt%, or 10 wt% to 20 wt%. In other embodiments, the aerosol-generating composition or aerosol-generating material comprises less than 20 wt%, suitably less than 10 wt% or less than 5 wt% of a filler. In some cases, the aerosol-generating composition or aerosol-generating material comprises less than 1 wt% of a filler, and in some cases, comprises no filler.

In some embodiments, the filler comprises (or is) one or more inorganic filler materials, such as calcium carbonate, perlite, vermiculite, diatomaceous earth, colloidal silica, magnesium oxide, magnesium sulphate, magnesium carbonate, and suitable inorganic sorbents, such as molecular sieves.

In some embodiments, the filler comprises (or is) one or more organic filler materials such as wood pulp; tobacco pulp; hemp fibre; cellulose and cellulose derivatives, such as microcrystalline cellulose and/or nanocrystalline cellulose.

As would be well understood by the skilled person, microcrystalline cellulose may be formed by depolymerising cellulose by a chemical process (e.g. using an acid or enzyme). One example method for forming microcrystalline cellulose involves acid hydrolysis of cellulose, using an acid such as HCl. The cellulose produced after this treatment is crystalline (i.e. no amorphous regions remain). Suitable methods and conditions for forming microcrystalline cellulose are well-known in the art.

In some embodiments, the aerosol-generating composition or aerosol-generating material does not comprise any inorganic filler.

In some embodiments, the aerosol-generating composition or aerosol-generating material comprises less than about 20 wt%, less than about 10 wt%, less than about 5 wt% or less than about 1 wt% calcium carbonate, such as chalk.

In some embodiments, the aerosol-generating composition or aerosol-generating material does not comprise calcium carbonate, such as chalk.

In particular embodiments, the aerosol-generating composition or aerosol-generating material comprises filler and the filler is fibrous. For example, the filler may be a fibrous organic filler material such as wood pulp, tobacco pulp, hemp fibre, cellulose or cellulose derivatives. In some embodiments, the fibrous organic filler material may be wood pulp, hemp fibre, cellulose or cellulose derivatives. In particular embodiments, the fibrous filler is wood pulp. Without wishing to be bound by theory, it is believed that including fibrous filler may increase the tensile strength of the material. This may be particularly advantageous in examples wherein the aerosol-generating material is provided as a sheet, such as when an aerosol-generating material sheet circumscribes a rod of aerosolisable material.

In particular embodiments, the filler comprises microcrystalline cellulose and/or wood pulp.

### Actives and flavourants

As mentioned above, the compositions and materials described herein may comprise certain actives and/or flavourants, other than those compounds listed herein as key chemical markers. Any actives and/or flavourants may be used to, for example, provide a consumer with a desirable experience and/or flavour in combination with the cigarette-like attributes conferred by the key chemical markers discussed herein. In this case it may be desirable for the composition to comprise only some of the key chemical markers. By way of example only, key sense and/or taste chemical markers could be used (without the key flavour markers) to provide a composition having a cigarette-like sense and/or taste. Such a composition could be combined or used with a known active or flavourants, such as menthol, for example to create a cigarette-like experience but with the flavour of menthol. Similarly, a drug (such as CBD) could be added to a composition of the invention to provide a cigarette-like experience whilst also experiencing the effects provided by the drug.

In some embodiments the composition or the aerosol-generating material described herein additionally comprises an active substance.

The active substance as used herein may be a physiologically active material, which is a material intended to achieve or enhance a physiological response. The active substance may for example be selected from nutraceuticals, nootropics, psychoactives. The active substance may be naturally occurring or synthetically obtained. The active substance may comprise for example nicotine, caffeine, taurine, theine, vitamins such as B6 or B12 or C, melatonin, cannabinoids, or constituents, derivatives, or combinations thereof. The active substance may comprise one or more constituents, derivatives or extracts of tobacco, cannabis or another botanical.

In one embodiment the active substance is a legally permissible recreational drug

In some embodiments, the active substance comprises nicotine. In some embodiments, the active substance comprises caffeine, melatonin or vitamin B12.

In some cases, the composition or the aerosol-generating material comprises from about 1wt%, 5wt%, 10wt%, 15wt%, 20wt% or 25wt% to about 70wt%, 60wt%, 50wt%, 45wt%, 40wt%, 35wt%, or 30wt% (calculated on a dry weight basis) of an active substance (such as tobacco and/or nicotine).

In some cases, the composition or the aerosol-generating material may comprise a botanical extract. The composition or the aerosol-generating material may comprise about 1 wt%, 3 wt%, 5 wt%, 10 wt%, 15 wt%, 20 wt %, 30 wt%, 35 wt% or 40 wt% to about to 30 wt%, 35 wt%, 40 wt%, 50 wt%, 60 wt%, 65 wt % or 70 wt% of botanical extract (all calculated on a dry weight basis), such as from about 1 to about 70 wt%, from about 5 to about 60 wt%, or from about 10 to about 50 wt%.

As used herein, the term "botanical extract" includes an extract of any material derived from plants including, but not limited to, extracts, leaves, bark, fibres, stems, roots, seeds, flowers, fruits, pollen, husk, shells or the like. Alternatively, the botanical extract may comprise an active compound naturally existing in a botanical, obtained synthetically. Example botanicals are tobacco, eucalyptus, star anise, hemp, cocoa, cannabis, fennel, lemongrass, peppermint, spearmint, rooibos, chamomile, flax, ginger, ginkgo biloba, hazel, hibiscus, laurel, licorice (liquorice), matcha, mate, orange skin, papaya, rose, sage, tea such as green tea or black tea, thyme, clove, cinnamon, coffee, aniseed (anise), basil, bay leaves, cardamom, coriander, cumin, nutmeg, oregano, paprika, rosemary, saffron, lavender, lemon peel, mint, juniper, elderflower, vanilla, wintergreen, beefsteak plant, curcuma, turmeric, sandalwood, cilantro, bergamot, orange blossom, myrtle, cassis, valerian, pimento, mace, damien, marjoram, olive, lemon balm, lemon basil, chive, carvi, verbena, tarragon, geranium, mulberry, ginseng, theanine, theacrine, maca, ashwagandha, damiana, guarana, chlorophyll, baobab or any combination thereof. The mint may be chosen from the following mint varieties: Mentha Arventis, Mentha c.v.,Mentha niliaca, Mentha piperita, Mentha piperita citrata c.v.,Mentha piperita c.v, Mentha spicata crispa, Mentha cardifolia, Memtha longifolia, Mentha suaveolens variegata, Mentha pulegium, Mentha spicata c.v. and Mentha suaveolens.

In some embodiments, the active substance comprises or is derived from one or more botanicals or constituents, derivatives or extracts thereof and the botanical is tobacco.

In some embodiments, the active substance comprises or derived from one or more botanicals or constituents, derivatives or extracts thereof and the botanical is selected from eucalyptus, star anise, cocoa and hemp.

In some embodiments, the active substance comprises or derived from one or more botanicals or constituents, derivatives or extracts thereof and the botanical is selected from rooibos and fennel.

In some embodiments, the active substance may comprise one or more constituents, derivatives or extracts of cannabis, such as one or more cannabinoids or terpenes.

Cannabinoids are a class of natural or synthetic chemical compounds which act on cannabinoid receptors (i.e., CB1 and CB2) in cells that repress neurotransmitter release in the brain. Cannabinoids may be naturally occurring (phytocannabinoids) from plants such as cannabis, from animals (endocannabinoids), or artificially manufactured (synthetic cannabinoids). Cannabis species express at least 85 different phytocannabinoids, and are divided into subclasses, including cannabigerols, cannabichromenes, cannabidiols, tetrahydrocannabinols, cannabinols and cannabinodiols, and other cannabinoids. Cannabinoids found in cannabis include, without limitation: cannabigerol (CBG), cannabichromene (CBC), cannabidiol (CBD), tetrahydrocannabinol (THC), cannabinol (CBN), cannabinodiol (CBDL), cannabicyclol (CBL), cannabivarin (CBV), tetrahydrocannabivarin (THCV), cannabidivarin (CBDV), cannabichromevarin (CBCV), cannabigerovarin (CBGV), cannabigerol monomethyl ether (CBGM), cannabinerolic acid, cannabidiolic acid (CBDA), Cannabinol propyl variant (CBNV), cannabitriol (CBO), tetrahydrocannabmolic acid (THCA), and tetrahydrocannabivarinic acid (THCV A).

In some embodiments, the active substance may comprise a cannabinoid, such as cannabidiol (CBD).

As used herein, the terms "flavour" and "flavourant" refer to materials which, where local regulations permit, may be used to create a desired taste, aroma or other somatosensorial sensation in a product for adult consumers. They may include naturally occurring flavour materials, botanicals, extracts of botanicals, synthetically obtained materials, or combinations thereof (e.g., tobacco, cannabis, licorice (liquorice), hydrangea, eugenol, Japanese white bark magnolia leaf, chamomile, fenugreek, clove, maple, matcha, menthol, Japanese mint, aniseed (anise), cinnamon, turmeric, Indian spices, Asian spices, herb, wintergreen, cherry, berry, red berry, cranberry, peach, apple, orange, mango, clementine, lemon, lime, tropical fruit, papaya, rhubarb, grape, durian, dragon fruit, cucumber, blueberry, mulberry, citrus fruits, Drambuie, bourbon, scotch, whiskey, gin, tequila, rum, spearmint, peppermint, lavender, aloe vera, cardamom, celery, cascarilla, nutmeg, sandalwood, bergamot, geranium, khat, naswar, betel, shisha, pine, honey essence, rose oil, vanilla, lemon oil, orange oil, orange blossom, cherry blossom, cassia, caraway, cognac, jasmine, ylang-ylang, sage, fennel, wasabi, piment, ginger, coriander, coffee, hemp, a mint oil from any species of the genus Mentha, eucalyptus, star anise, cocoa, lemongrass, rooibos, flax, ginkgo biloba, hazel, hibiscus, laurel, mate, orange skin, rose, tea such as green tea or black tea, thyme, juniper, elderflower, basil, bay leaves, cumin, oregano, paprika, rosemary, saffron, lemon peel, mint, beefsteak plant, curcuma, cilantro, myrtle, cassis, valerian, pimento, mace, damien, marjoram, olive, lemon balm, lemon basil, chive, carvi, verbena, tarragon, limonene, thymol, camphene), flavour enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, liquid such as an oil, solid such as a powder, or gas.

In some embodiments, the flavour comprises menthol, spearmint and/or peppermint. In some embodiments, the flavour comprises flavour components of cucumber, blueberry, citrus fruits and/or redberry. In some embodiments, the flavour comprises eugenol. In some embodiments, the flavour comprises flavour components extracted from tobacco. In some embodiments, the flavour comprises flavour components extracted from cannabis.

In some embodiments, the flavour may comprise a sensate, which is intended to achieve a somatosensorial sensation which are usually chemically induced and perceived by the stimulation of the fifth cranial nerve (trigeminal nerve), in addition to or in place of aroma or taste nerves, and these may include agents providing heating, cooling, tingling, numbing effect. A suitable heat effect agent may be, but is not limited to, vanillyl ethyl ether and a suitable cooling agent may be, but not limited to eucolyptol, WS-3.

### Other functional materials

In some embodiments, the aerosol-generating composition or material may further comprise one or more other functional materials. The one or more other functional materials may comprise one or more of pH regulators, colouring agents, preservatives, stabilizers, and/or antioxidants.

The aerosol-generating composition or material may comprise an acid. The acid may be an organic acid. In some of these embodiments, the acid may be at least one of a monoprotic acid, a diprotic acid and a triprotic acid. In some such embodiments, the acid may contain at least one carboxyl functional group. In some such embodiments, the acid may be at least one of an alpha-hydroxy acid, carboxylic acid, dicarboxylic acid, tricarboxylic acid and keto acid. In some such embodiments, the acid may be an alpha-keto acid.

In some such embodiments, the acid may be at least one of succinic acid, lactic acid, benzoic acid, citric acid, tartaric acid, fumaric acid, levulinic acid, acetic acid, malic acid, formic acid, sorbic acid, benzoic acid, propanoic and pyruvic acid.

Suitably the acid is lactic acid. In other embodiments, the acid is benzoic acid. In other embodiments the acid may be an inorganic acid. In some of these embodiments the acid may be a mineral acid. In some such embodiments, the acid may be at least one of sulphuric acid, hydrochloric acid, boric acid and phosphoric acid. In some embodiments, the acid is levulinic acid.

The inclusion of an acid is particularly preferred in embodiments in which the aerosol-generating composition or material comprises nicotine. In such embodiments, the presence of an acid may stabilise dissolved species in the slurry from which the aerosol-generating composition is formed. The presence of the acid may reduce or substantially prevent evaporation of nicotine during drying of the slurry, thereby reducing loss of nicotine during manufacturing.

The aerosol-generating material may comprise a colourant. The addition of a colourant may alter the visual appearance of the aerosol-generating material. The presence of colourant in the aerosol-generating material may enhance the visual appearance of the aerosol-generating material and the aerosol-generating composition. By adding a colourant to the aerosol-generating material, the aerosol-generating material may be colour-matched to other components of the aerosol-generating composition or to other components of a consumable comprising the aerosol-generating material.

A variety of colourants may be used depending on the desired colour of the aerosol-generating material. The colour of aerosol-generating material may be, for example, white, green, red, purple, blue, brown or black. Other colours are also envisaged. Natural or synthetic colourants, such as natural or synthetic dyes, food-grade colourants and pharmaceutical-grade colourants may be used. In certain embodiments, the colourant is caramel, which may confer the aerosol-generating material with a brown appearance.

The colourant may be incorporated during the formation of the aerosol-generating material (e.g. when forming a slurry comprising the materials that form the aerosol-generating material) or it may be applied to the aerosol-generating material after its formation (e.g. by spraying it onto the aerosol-generating material).

In some embodiments, the aerosol-generating material is formed as a sheet. In some cases, the aerosol-generating material sheet may be incorporated into the non-combustible aerosol provision system or consumable in sheet form. The aerosol-generating material sheet may be incorporated as a planar sheet, as a gathered or bunched sheet, as a crimped sheet, or as a rolled sheet (i.e. in the form of a tube). In some such cases, the aerosol-generating material of these embodiments may be included in the system/consumable as a sheet, such as a sheet circumscribing a rod of aerosolisable material (e.g. tobacco). For example, the aerosol-generating material sheet may be formed on a wrapping paper which circumscribes an aerosolisable material such as tobacco. In other cases, the sheet may be shredded and then incorporated into the assembly, suitably mixed into an aerosolisable material such as cut rag tobacco.

In some cases, the aerosol-generating material may be in the form of a sheet or layer having a thickness of about 0.015 mm to about 1.0 mm. Suitably, the thickness may be in the range of about 0.05 mm, 0.1 mm or 0.15 mm to about 0.5 mm or 0.3 mm, for example 0.1-3 mm or 0.15-3 mm. A material having a thickness of 0.2 mm may be particularly suitable. The aerosol-generating material may comprise more than one layer, and the thickness described herein refers to the aggregate thickness of those layers.

If the aerosol-generating material is too thick, then heating efficiency may be compromised. This adversely affects the power consumption in use. Conversely, if the aerosol-generating material is too thin, it may be difficult to manufacture and handle; a very thin material is harder to cast and may be fragile, compromising aerosol formation in use.

The thickness stipulated herein is a mean thickness for the material. In some cases, the aerosol-generating material thickness may vary by no more than 25%, 20%, 15%, 10%, 5% or 1%.

In some embodiments, the aerosol-generating material in sheet form may have sufficient tensile strength such that it can be wound onto, or unwound from, a bobbin without breakages. In some examples, the aerosol-generating material in sheet form has a tensile strength of greater than or equal to about 250 N/m.

The aerosol-generating material may have any suitable area density, such as from 30 g/m² to 120 g/m². In some cases, the aerosol-generating material may have a mass per unit area of from about 80 to 120 g/m², or from about 70 to 110 g/m², or particularly from about 90 to 110 g/m², or suitably about 100 g/m² (so that it will not readily separate when mixed with tobacco, such as cut rag tobacco). Such area densities may be particularly suitable where the aerosol-generating material is included in the consumable/system in sheet form, or as a shredded sheet (described further herein below).

### Carrier

The aerosol-generating substrate or consumable may comprise a carrier on which the aerosol-generating material is provided. The aerosol-generating material may be bonded or otherwise joined to the carrier. The carrier functions as a support on which the aerosol-generating material layer forms, easing manufacture. The carrier may provide tensile strength to the aerosol-generating material layer, easing handling.

In some cases, the carrier may be formed from materials selected from metal foil, paper, carbon paper, greaseproof paper, ceramic, carbon allotropes such as graphite and graphene, plastic, cardboard, wood or combinations thereof. In some cases, the carrier may comprise or consist of a tobacco material, such as a sheet of reconstituted tobacco. In some cases, the carrier may be formed from materials selected from metal foil, paper, cardboard, wood or combinations thereof. In some cases, the carrier itself be a laminate structure comprising layers of materials selected from the preceding lists. In some cases, the carrier may also function as a flavour carrier. For example, the carrier may be impregnated with a flavour or with tobacco extract.

In some cases, the carrier may be magnetic. This functionality may be used to fasten the carrier to the non-combustible aerosol provision device in use, or may be used to generate particular aerosol-generating material shapes. In some cases, the aerosol-generating composition may comprise one or more magnets which can be used to fasten the material to an induction heater in use.

In some cases, the carrier may be substantially or wholly impermeable to gas and/or aerosol. This prevents aerosol or gas passage through the carrier layer, thereby controlling the flow and ensuring it is delivered to the user. This can also be used to prevent condensation or other deposition of the gas/aerosol in use on, for example, the surface of a heater provided in an aerosol generating assembly. Thus, consumption efficiency and hygiene can be improved in some cases.

In some cases, the surface of the carrier that abuts the aerosol-generating material may be porous. For example, in one case, the carrier comprises paper. A porous carrier such as paper has been found to be particularly suitable; the porous (e.g. paper) layer abuts the aerosol-generating material layer and forms a strong bond. The aerosol-generating material may be formed by drying a slurry and, without being limited by theory, it is thought that the slurry partially impregnates the porous carrier (e.g. paper) so that the carrier is partially bound into the aerosol-generating material. This provides a strong binding between the aerosol-generating material and the carrier.

In some embodiments, the aerosol-generating material may be laminated to a carrier, such as a paper sheet.

In some embodiments, when the aerosol-generating material is formed from a slurry as described herein, the layer of slurry may be formed on a carrier, such as a paper sheet.

Additionally, surface roughness may contribute to the strength of bond between the aerosol-generating material and the carrier. Conversely, the surface of the carrier facing away from the aerosol-generating material may be arranged in contact with the heater, and a smoother surface may provide more efficient heat transfer. Thus, in some cases, the carrier is disposed so as to have a rougher side abutting the aerosol-generating material and a smoother side facing away from the aerosol-generating material.

In one particular case, the carrier may be a paper-backed foil; the paper layer abuts the aerosol-generating material and the properties discussed in the previous paragraphs are afforded by this abutment. The foil backing is substantially impermeable, providing control of the aerosol flow path. A metal foil backing may also serve to conduct heat to the aerosol-generating material.

In another case, the foil layer of the paper-backed foil abuts the aerosol-generating material. The foil is substantially impermeable, thereby preventing water provided in the aerosol-generating material from being absorbed into the paper which could weaken its structural integrity.

In some cases, the carrier is formed from or comprises metal foil, such as aluminium foil. A metallic carrier may allow for better conduction of thermal energy to the aerosol-generating material. Additionally, or alternatively, a metal foil may function as a susceptor in an induction heating system. In particular embodiments, the carrier comprises a metal foil layer and a support layer, such as cardboard. In these embodiments, the metal foil layer may have a thickness of less than 20 µm, such as from about 1 µm to about 10 µm, suitably about 5 µm.

In some cases, the carrier may have a thickness of between about 0.010 mm and about 2.0 mm, suitably from about 0.015 mm, 0.02 mm, 0.05 mm or 0.1 mm to about 1.5 mm, 1.0 mm, or 0.5 mm.

### Consumable

In another aspect of the disclosure, there is provided a consumable for use in a non-combustible aerosol provision device, the consumable comprising an aerosol-generating composition or material as described herein.

In some embodiments, the disclosure relates to consumables comprising an aerosol-generating composition as described herein and configured to be used with non-combustible aerosol provision devices. These consumables are sometimes referred to as articles throughout the disclosure.

The consumable may be used with any suitable non-combustible aerosol provision device.

A consumable is an article comprising or consisting of an aerosol-generating composition or material, part or all of which is intended to be consumed during use by a user. A consumable may comprise one or more other components, such as an aerosol-generating composition or material storage area, an aerosol-generating composition transfer component, an aerosol generation area, a housing, a wrapper, a mouthpiece, a filter and/or an aerosol-modifying agent. A consumable may also comprise an aerosol generator, such as a heater, that emits heat to cause the aerosol-generating composition to generate aerosol in use. The heater may, for example, comprise combustible material, a material heatable by electrical conduction, or a susceptor.

A susceptor is a material that is heatable by penetration with a varying magnetic field, such as an alternating magnetic field. The susceptor may be an electrically-conductive material, so that penetration thereof with a varying magnetic field causes induction heating of the heating material. The heating material may be magnetic material, so that penetration thereof with a varying magnetic field causes magnetic hysteresis heating of the heating material. The susceptor may be both electrically-conductive and magnetic, so that the susceptor is heatable by both heating mechanisms. The device that is configured to generate the varying magnetic field is referred to as a magnetic field generator, herein.

An aerosol-modifying agent is a substance, typically located downstream of the aerosol generation area, that is configured to modify the aerosol generated, for example by changing the taste, flavour, acidity or another characteristic of the aerosol. The aerosol-modifying agent may be provided in an aerosol-modifying agent release component, that is operable to selectively release the aerosol-modifying agent.

The aerosol-modifying agent may, for example, be an additive or a sorbent. The aerosol-modifying agent may, for example, comprise one or more of a flavourant, a colourant, water, and a carbon adsorbent. The aerosol-modifying agent may, for example, be a solid, a liquid, or a gel. The aerosol-modifying agent may be in powder, thread or granule form. The aerosol-modifying agent may be free from filtration material.

An aerosol generator is an apparatus configured to cause aerosol to be generated from the aerosol-generating composition. In some embodiments, the aerosol generator is a heater configured to subject the aerosol-generating composition to heat energy, so as to release one or more volatiles from the aerosol-generating composition to form an aerosol. In some embodiments, the aerosol generator is configured to cause an aerosol to be generated from the aerosol-generating composition without heating. For example, the aerosol generator may be configured to subject the aerosol-generating composition to one or more of vibration, increased pressure, or electrostatic energy.

### Non-combustible aerosol provision system

In another aspect of the disclosure, there is provided a non-combustible aerosol provision system comprising the consumable described herein and a non-combustible aerosol provision device.

According to the present disclosure, a "non-combustible" aerosol provision system is one where a constituent aerosol-generating composition of the aerosol provision system (or component thereof) is not combusted or burned in order to facilitate delivery of at least one substance to a user.

In some embodiments, the delivery system is a non-combustible aerosol provision system, such as a powered non-combustible aerosol provision system.

In some embodiments, the non-combustible aerosol provision system is an electronic cigarette, also known as a vaping device or electronic nicotine delivery system (END), although it is noted that the presence of nicotine in the aerosol-generating composition or material is not a requirement.

In some embodiments, the non-combustible aerosol provision system is an aerosol-generating composition heating system, also known as a heat-not-burn system. An example of such a system is a tobacco heating system.

In some embodiments, the non-combustible aerosol provision device is a heat-not-burn device.

In some embodiments, the non-combustible aerosol provision device is a vaping device or electronic cigarette (e-cigarette). Electronic cigarettes (e-cigarettes) generally contain a reservoir of a liquid (which may be or comprise the composition of the present invention), from which an aerosol is generated, e.g. through heat vaporization. An aerosol source for an aerosol provision system may thus comprise a heater having a heating element arranged to receive source liquid from the reservoir, for example through wicking/capillary action. While a user inhales on the device, electrical power is supplied to the heating element to vaporize source liquid in the vicinity of the heating element to generate an aerosol for inhalation by the user. Such devices are usually provided with one or more air inlet holes located away from a mouthpiece end of the system. When a user sucks on a mouthpiece connected to the mouthpiece end of the system, air is drawn in through the inlet holes and past the aerosol source. There is a flow path connecting between the aerosol source and an opening in the mouthpiece so that air drawn past the aerosol source continues along the flow path to the mouthpiece opening, carrying some of the aerosol from the aerosol source with it. The aerosol-carrying air exits the aerosol provision system through the mouthpiece opening for inhalation by the user.

Electronic cigarettes include a mechanism for activating the heater to vaporize the source liquid during use. One approach is to provide a manual activation mechanism, such as a button, which the user presses to activate the heater. In such devices, the heater may be activated (i.e. supplied with electrical power) while the user is pressing the button, and deactivated when the user releases the button. Another approach is to provide an automatic activation mechanism, such as a pressure sensor arranged to detect when a user is drawing air through the device by inhaling on the mouthpiece. In such devices, the heater may be activated when it is detected the user is inhaling through the device and deactivated when it is detected the user has stopped inhaling through the device.

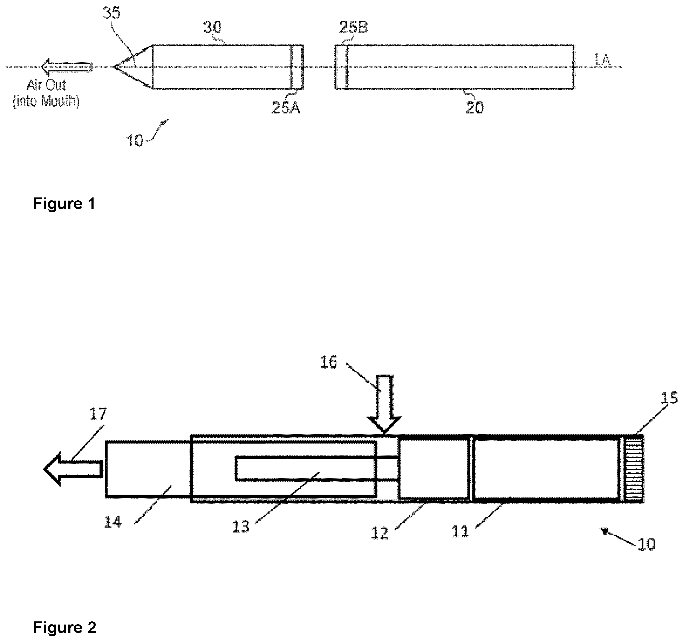

The e-cigarette 10 shown in Figure 1 has a generally cylindrical shape, extending along a longitudinal axis indicated by dashed line LA, and comprising two main components, namely a body 20 and a cartomizer 30. The cartomizer 30 includes an internal chamber containing a reservoir of a source liquid comprising a liquid formulation from which an aerosol is to be generated, a heating element, and a liquid transport element (in this example a wicking element) for transporting source liquid to the vicinity of the heating element. In some example implementations the heating element may itself provide the liquid transport function. The heating element and the element providing the liquid transport function may sometimes be collectively referred to as an aerosol generator/aerosol source/aerosol forming member/vaporizer/atomizer/distiller.

The cartomizer 30 further includes a mouthpiece 35 having an opening through which a user may inhale the aerosol from the aerosol generator. The source liquid may be of a conventional kind used in e-cigarettes, for example comprising 0 to 5% nicotine dissolved in a solvent comprising glycerol, water, and/or propylene glycol. The source liquid may also comprise flavourings. The source liquid may further comprise the composition described herein. In some embodiments, the source liquid is the composition described herein.

The reservoir for the source liquid may comprise a porous matrix or any other structure within a housing for retaining the source liquid until such time that it is required to be delivered to the aerosol generator/vaporizer. In some examples the reservoir may comprise a housing defining a chamber containing free liquid (i.e. there may not be a porous matrix).

The body 20 includes a re-chargeable cell or battery to provide power for the e-cigarette 10 and a circuit board including control circuitry for generally controlling the e-cigarette 10. The circuit board may also include a motion sensor (e.g. an accelerometer) for detecting motion of the electronic cigarette 10 and outputting corresponding motion detection signals to the control circuitry. It will be appreciated the motion sensor need not necessarily be mounted on the circuit board but could equally be mounted elsewhere in the electronic cigarette 10 with appropriate wiring for communicating motion detection signals to the control circuitry.

In active use, i.e. when the heating element receives power from the battery, as controlled by the control circuitry, the heating element vaporizes source liquid in the vicinity of the heating element to generate an aerosol. The aerosol is inhaled by a user through the opening in the mouthpiece 35. During user inhalation the aerosol is carried from the aerosol source to the mouthpiece opening along an air channel that connects between them.

In this particular example, the body 20 and cartomizer 30 are detachable from one another by separating in a direction parallel to the longitudinal axis LA, as shown in FIG. 1, but are joined together when the device 10 is in use by a connection, indicated schematically in FIG. 1 as 25A and 25B, to provide mechanical and electrical connectivity between the body 20 and the cartomizer 30. The electrical connector on the body 20 that is used to connect to the cartomizer 30 also serves as a socket for connecting a charging device (not shown) when the body 20 is detached from the cartomizer 30. The other end of the charging device can be plugged into an external power supply, for example a USB socket, to charge or to re-charge the cell/battery in the body 20 of the e-cigarette 10. In other implementations, a cable may be provided for direct connection between the electrical connector on the body 20 and the external power supply and/or the device may be provided with a separate charging port, for example a port conforming to one of the USB formats.

The e-cigarette 10 is provided with one or more holes (not shown in FIG. 1) for air inlet. These holes connect to an air running passage (airflow path) through the e-cigarette 10 to the mouthpiece 35. The air passage includes a region around the aerosol source and a section comprising an air channel connecting from the aerosol source to the opening in the mouthpiece 35.

When a user inhales through the mouthpiece 35, air is drawn into this air passage through the one or more air inlet holes, which are suitably located on the outside of the e-cigarette 10. This airflow (or the resulting change in pressure) may be detected by an airflow sensor, e.g. a pressure sensor, for detecting airflow in electronic cigarette 10 and outputting corresponding airflow detection signals to the control circuitry. The airflow sensor may operate in accordance with conventional techniques in terms of how it is arranged within the electronic cigarette 10 to generate airflow detection signals indicating when there is a flow of air through the electronic cigarette 10 (e.g. when a user inhales or blows on the mouthpiece 35).

In some embodiments, the non-combustible aerosol provision system is a hybrid system to generate aerosol using a combination of aerosol-generating materials, one or a plurality of which may be heated. Each of the aerosol-generating materials may be, for example, in the form of a solid, liquid or gel and may or may not contain nicotine. In some embodiments, the hybrid system comprises a liquid or gel aerosol-generating material and a solid aerosol-generating material. The liquid or gel aerosol-generating material may comprise a material or composition as described herein. The solid aerosol-generating material may comprise, for example, tobacco or a non-tobacco product.

In some embodiments, the non-combustible aerosol provision device is an electronic tobacco hybrid device.

Typically, the non-combustible aerosol provision system may comprise a non-combustible aerosol provision device and a consumable as described herein for use with the non-combustible aerosol provision device.

In some embodiments, the disclosure relates to consumables comprising aerosol-generating material and configured to be used with non-combustible aerosol provision devices. These consumables are sometimes referred to as articles throughout the disclosure.

In some embodiments, the non-combustible aerosol provision system, such as a non-combustible aerosol provision device thereof, may comprise a power source and a controller. The power source may, for example, be an electric power source or an exothermic power source. In some embodiments, the exothermic power source comprises a carbon substrate which may be energised so as to distribute power in the form of heat to an aerosol-generating composition or to a heat transfer material in proximity to the exothermic power source.

In some embodiments, the non-combustible aerosol provision system, such as a non-combustible aerosol provision device thereof, may comprise an area for receiving the consumable, an aerosol generator, an aerosol generation area, a housing, a mouthpiece, a filter and/or an aerosol-modifying agent.

The non-combustible aerosol provision system or device may comprise a heater configured to heat but not burn the aerosol-generating composition/aerosol-generating material. The heater may be, in some cases, a thin film, electrically resistive heater. In other cases, the heater may comprise an induction heater or the like. In yet further cases, the heater may be a combustible heat source or a chemical heat source which undergoes an exothermic reaction to produce heat in use.

In some cases, the heater may heat but not burn the aerosolisable material(s) to between 120°C and 350°C in use. In some cases, the heater may heat but not burn the aerosolisable material(s) to between 140°C and 250°C in use.

In some cases in use, substantially all of the aerosol-generating material is less than about 4 mm, 3 mm, 2 mm or 1 mm from the heater. In some cases, the solid is disposed between about 0.017 mm and 2.0 mm from the heater, suitably between about 0.1 mm and 1.0 mm. These minimum distances may, in some cases, reflect the thickness of a carrier that supports the aerosol-generating material. In some cases, a surface of the aerosol-generating material may directly abut the heater.

In some cases, the heater may be embedded in the aerosol-generating composition/the aerosol-generating material. In some such cases, the heater may be an electrically resistive heater (with exposed contacts for connection to an electrical circuit). In other such cases, the heater may be a susceptor embedded in the aerosol-generating composition, which is heated by induction.

The non-combustible aerosol provision system may additionally comprise a cooling element and/or a filter. The cooling element, if present, may act or function to cool gaseous or aerosol components. In some cases, it may act to cool gaseous components such that they condense to form an aerosol. It may also act to space the very hot parts of the apparatus from the user. The filter, if present, may comprise any suitable filter known in the art such as a cellulose acetate plug.

In some cases, the non-combustible aerosol provision system may be a heat-not-burn system. That is, it may contain a solid material (and no liquid aerosolisable material). A heat-not-burn device is disclosed in WO 2015/062983 A2, which is incorporated by reference in its entirety.

In some cases, the non-combustible aerosol provision system may comprise an electronic tobacco hybrid device. That is, it may contain a solid aerosolisable material and a liquid aerosolisable material. The separate aerosolisable materials may be heated by separate heaters, the same heater or, in one case, a downstream aerosolisable material may be heated by a hot aerosol which is generated from the upstream aerosolisable material. An electronic tobacco hybrid device is disclosed in WO 2016/135331 A1, which is incorporated by reference in its entirety.

The consumable may alternatively be referred to herein as a cartridge. The consumable may be adapted for use in a THP, an electronic tobacco hybrid device, a vaping device or another aerosol generating device. In some cases, the consumable may additionally comprise a filter and/or cooling element, as described previously. In some cases, the consumable may be circumscribed by a wrapping material such as paper.

Figure 2 is a block diagram of an example aerosol generating device. The aerosol generating device shown in Figure 2 comprises a battery, a control circuit 12, a heater 13 and a consumable 14 (which may comprise the compositions or materials described herein). The device also includes a connector 15 (such as a USB connector). The connector 15 may enable connection to be made to a power source for charging the battery 11, for example under the control of the control circuit 12.

In the use of the device 10, the heater 13 is inserted into the consumable 14, such that the consumable may be heated (but not burnt) to generate an aerosol (and a cigarette-like experience) for the user. When a user inhales at the end of the consumable, as indicated by arrow 17, the air is drawn into the device 10, through an air inlet as indicated by arrow 16, then passes through the consumable, delivering the aerosol (and a cigarette-like experience) to the user.

The aerosol generating device 10 is provided by way of example only. Many alternative aerosol generating devices may be used in example implementations of the principles described here. For example, the device 10 maybe replaced within a vaping device in which an aerosol generating composition (e.g. a liquid) is heated to generate the aerosol.

In embodiments, the composition is a composition for oral delivery of the compounds.

### Method of producing aerosol-generating material

Another aspect of the invention provides a method of forming a composition as described herein, comprising combining or mixing the compounds together. The compounds may be isolated or purified before being combined or mixed.

The mixture of compounds may then be combined with other components of the composition. For example, a liquid composition of the invention may be formed by combining glycerol, propylene glycol and the key markers described herein, optionally together with other components of the composition such as water and any other actives and/or flavourants. A composition may also be formed by combining glycerol, propylene glycol and a tobacco extract comprising one or more of the key chemical markers described herein, optionally together with other key chemical markers and/or other components of the composition such as water and any other actives and/or flavourants.

The method may also comprise forming a tobacco extract, and optionally combining (e.g. mixing) said extract with one or more of the key chemical markers (e.g. key sense markers) described herein. The one or more chemical markers combined with the extract may first be isolated or purified.

As described above, the tobacco extract may be obtained by techniques such as supercritical fluid extraction (SFE) or simultaneous distillation extraction (SFE). The tobacco used to form the extract may be Virginia tobacco, burley tobacco, oriental tobacco, or a combination thereof, and may be cured.

SDE provides a technique in which the steps of isolation and extraction of certain compounds from a sample can be achieved simultaneously. In some embodiments, a simultaneous distillation extraction (SDE) process used for forming tobacco extracts for use in the present invention comprises
i) providing tobacco material;
ii) subjecting the tobacco material to steam distillation; and
iii) extracting one or more volatile compounds of interest from the tobacco material with a solvent;
wherein distillation step (ii) and extraction step (iii) are carried out simultaneously.

The method may comprise mixing a quantity of tobacco (e.g. ground tobacco) with water. This solution may have a pH of from about 5 to about 7, such as from about 5.5 to about 6.5, from about 5.5 to about 6, or about 6. Glass beads may be added to prevent bumping.

The solution is then heated, for example to a temperature of from about 100°C to about 130°C, such as from about 105°C to about 125°C, from about 110°C to about 125°C, from about 115°C to about 125°C, from about 120°C to about 122°C, or about 121 °C.

The distillation step (ii) and extraction step (iii) are carried out simultaneously in one single step. As such, in some embodiments, the SDE method described herein is carried out in just one single step. In some embodiments, an additional distillation and/or extraction step is excluded. In some embodiments, the entire SDE method (i.e. distillation step (ii) and extraction step (iii)) is carried out simultaneously in one single piece of apparatus with no additional processing prior to removal of the extracted product from the apparatus.

In some embodiments, the solvent is a non-polar solvent. In some embodiments, the solvent is a polar solvent. As used herein, the term "polar solvent" refers to any solvent having a dielectric constant of greater than or equal to 15. As used herein, the term "non-polar solvent" refers to any solvent having a dielectric constant of less than 15.

In some embodiments, the solvent is immiscible with water.

In some embodiments, the solvent comprises an organic-based solvent. In some embodiments, the solvent is an organic solvent. In some embodiments, the solvent is a non-polar organic solvent. The term "organic" is understood by those skilled in the art. Typically, an organic solvent is considered to be a solvent comprising carbon.

In some embodiments, the solvent is a non-polar solvent that is immiscible with water. In some embodiments, the solvent is selected from the group consisting of n-butanol, cyclohexane, dichloromethane, ethyl acetate, heptane, hexane, methyl-t-butyl ether, 2- butanone, pentane, diisopropyl ether, diethyl ether, and mixtures thereof. In some embodiments, the solvent is selected from the group consisting of pentane, diethyl ether and mixtures thereof. In some preferred embodiments, the solvent is a mixture of pentane and diethyl ether. The use of a mixture of pentane and ether is nontoxic, and may be easily replaced by ethanol after extraction of the volatile compounds of interest has been achieved.

In some embodiments, the solvent is a mixture of pentane and diethyl ether, wherein the solvent comprises the pentane and diethyl ether in a weight ratio of pentane to diethyl ether of from about 10:1 to about 1:10, such as from about 5:1 to about 1:5, such as from about 3: 1 to about 1:3, such as from 3:1 to 1:1, such as from about 3:1 to about 2:1, such as about 2:1.

In some embodiments, the solvent is a mixture of pentane and diethyl ether, wherein the solvent comprises the pentane and diethyl ether in a weight ratio of pentane to diethyl ether of about 2:1.

The period during which both the distillation step (ii) and the extraction step (iii) are carried out may be from about 1 to about 20 hours, such as from about 1 to about 7 hours, from about 2 to about 7 hours, from about 4 to about 7 hours, from about 4.5 to about 6.5 hours, from about 5 to about 6 hours, or about 6 hours.

In some embodiments, further processing of the extracted product is carried out, the further processing comprising a purifying step which comprises replacing the solvent used for extraction with another solvent suitable for use in a consumable, product or device as described herein. In some embodiments, such a purifying step comprises replacing the solvent used for extraction (e.g. pentane, diethyl ether or a mixture thereof) with ethanol.

In some embodiments, a supercritical fluid extraction (SFE) process used for forming tobacco extracts for use in the present invention comprises contacting tobacco with an extraction solvent under supercritical conditions. In some embodiments the extraction solvent comprises, or is, supercritical CO₂. In some embodiments no co-solvent is present.

In some embodiments the SFE process is performed at a pressure of from about 50 to about 500 bar, such as from about 100 to about 400 bar or from about 100 to about 300 bar.

In some embodiments the temperature during extraction may range from about 25°C to about 90°C, such as from about 30°C to about 70°C, or from about 40°C to about 60°C.

In some embodiments the SFE process is performed for from about 0.5 to about 5 hours, such as from about 1 to about 4 hours, from about 2 to about 3 hours, or about 2.5 hours.

In some embodiments, supercritical extraction is performed using a flow rate of extraction solvent (e.g. carbon dioxide) of from about 0.5, 0.8, or 1 kg/hr to about 2, 3 or 5 kg/hr. In some embodiments, supercritical extraction is performed using a flow rate of extraction solvent (e.g. carbon dioxide) of from about 0.1 to about 5 kg/hr, such as from about 0.8 to about 3 kg/hr, from about 1 to about 3 kg/hr, or about 1 kg/hr.

In some embodiments, the pressure is varied during the SFE process, for example to collect fractions having varying volatility, for example a volatile (or more volatile) fraction and a non-volatile (or less volatile) fraction.

In some embodiments, a lower pressure is used for a first period of time, followed by a higher pressure for a second period of time. The lower pressure may be, for example, from about 50 to about 200 bar, such as from about 80 to about 120 bar, or about 100 bar. The higher pressure may be, for example, from about 200 to about 500 bar, such as from about 250 to about 400 bar, such as from about 250 to about 350 bar, or about 300 bar. The first period of time may be from about 0.5 to about 2 hours, such as about 1 hour. The first period of time may be from about 0.5 to about 2 hours, such as from about 1 to about 2 hours, or about 1.5 hours. In some embodiments, the two fractions or extracts are combined (or simply collected in the same container). In some embodiments, the two fractions or extracts are collected separately and are not combined. In some embodiments, only the extract from the second period of time (at a higher pressure) is retained and/or used in the present invention.

The method may also comprise combining (e.g. mixing) a tobacco extract with one or more of the key chemical markers described herein. The one or more chemical markers added may first be isolated or purified.

The method may comprise:
(a) forming a slurry comprising a solvent, the compounds (i.e. the key chemical markers), and any other components of the composition or precursors thereof;
(b) forming a layer of the slurry; and
(c) drying the slurry to form the composition.

The method may comprise:
(a) forming a slurry comprising a solvent, a tobacco extract, one or more key chemical markers, and any other components of the composition or precursors thereof;
(b) forming a layer of the slurry; and
(c) drying the slurry to form the composition.

The method may comprise:
(a) forming a slurry comprising a solvent, a tobacco extract comprising at least one of the key chemical markers (such as the key sense markers) described herein, any additional key chemical markers, and any other components of the composition or precursors thereof;
(b) forming a layer of the slurry; and
(c) drying the slurry to form the composition.

The tobacco extract may comprise at least three of the key sense markers, as described herein.

In other embodiments, one or more of the key chemical (e.g. sense) markers or additional key chemical (e.g. sense) markers are applied to the slurry during and/or after steps (b) and/or (c). For example, chemical markers (which may first be isolated or purified) may be applied to the slurry before or after drying.

The disclosures herein relating to constituents of the aerosol-generating composition and material apply equally to the slurry. The slurry may comprise these constituents in any of the proportions given herein in relation to the composition of the aerosol-generating composition and material.

When the aerosol-generating composition comprises additional binder that comprises alginate and/or pectin, the slurry may further comprise a setting agent and/or a setting agent may be applied to the slurry. In this case, the method may further comprise a step of setting the slurry. In some examples, forming the layer of the slurry and/or setting the slurry and/or drying the slurry, at least partially, occur simultaneously (for example, during electrospraying). In some examples, the steps of forming the layer of the slurry, setting the slurry with any setting agent and drying the slurry occur sequentially, in that order.

In some examples, the solvent comprises water.

Step (b) of forming a layer of the slurry typically comprises spraying, casting or extruding the slurry. In examples, the slurry layer is formed by casting the slurry.

In some examples, the slurry is applied to a support. The layer may be formed on a support.

In some cases, a setting agent (such as a calcium source) may be added to the slurry before or during step (b). This is appropriate in instances where gelation occurs relatively slowly, and thus the slurry may be, e.g. cast, after the setting agent is added.

In other cases, a setting agent may be applied to the slurry layer after step (b). The setting agent may be sprayed onto the slurry, for example, or may be preloaded onto the surface on which the slurry is layered.

A setting agent comprising a calcium source (such as calcium chloride or calcium citrate), may be added to a slurry containing alginate and/or pectin to form a calcium-crosslinked alginate/pectin gel.

The total amount of the setting agent, such as a calcium source, may be from about 0.5 to about 5 wt% (calculated on a dry weight basis).

The drying (c) may be done at ambient or raised temperatures.

In examples, the drying (c) removes from about 50 wt%, 60 wt%, 70 wt%, 80 wt% or 90 wt% to about 80 wt%, 90 wt% or 95 wt% (wet weight basis, WWB) of water in the slurry.

In examples, the drying (c) reduces the cast material thickness by at least 80%, suitably 85% or 87%. For instance, if the slurry is cast at a thickness of 2 mm, the resulting dried aerosol-generating material may have a thickness of 0.2 mm.

In embodiments, the dried aerosol-generating material forms a sheet or layer with a thickness of about 0.015 mm to about 1.0 mm. Suitably, the thickness may be in the range of about 0.05 mm, 0.1 mm or 0.15 mm to about 0.5 mm or 0.3 mm, for example 0.05-0.3 or 0.15-0.3 mm. A material having a thickness of 0.2 mm may be particularly suitable.

Another aspect of the invention provides a method (referred to below as the second method) comprising providing the aerosol-generating material and combining the aerosol-generating material and tobacco material.

The slurry itself is an aspect of the invention. In some examples, the slurry solvent consists essentially of or consists of water. In some examples, the slurry comprises from about 50 wt%, 60 wt%, 70 wt%, 80 wt% or 90 wt% of solvent (WWB).

### Aerosol

In one aspect, the invention provides an aerosol formed from a non-combustible aerosol provision device, wherein the aerosol comprises the key chemical markers described herein. All aspects described herein in relation to the composition also apply to the aerosol. Thus, the aerosol comprises at least three of the key sense markers, i.e. at least three of the following compounds: nicotine; 2-hydroxy-4-methyl-2-cyclopenten-1-one; quinic acid; sucrose; N-(1-deoxy-1-fructosyl)proline; 3,4-dimethyl-2(3H)-furanone; lipid peroxidation inhibitor 1; pseudooxynicotine and/or oxynicotine; 2-ethyl-quinoxaline; 1,2,3,4,5,6-hexahydro-7H-cyclopenta[b]pyridin-7-one; 2-ethyl-pyridine; scopoletin; 2-ethyl-5-methyl-pyridine; rutin; retinol; 2-methyl-3-butyl-pyrazine; megastigmatrienone; 3,5-di(1,1-dimethylethyl)-4-hydroxybenzaldehyde; L-alpha-amino-1H-pyrrole-1-hexanoic acid; and solanesol.

The aerosol may further comprise one or more of the taste and/or flavour markers, as described herein in relation to the composition.

In some embodiments, the aerosol further comprises 1 or more (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32 or 33) of the key smoke markers.

In some embodiments, the aerosol further comprises 1 or more (e.g. 1, 2, 3, 4, 5, 6, 7, 8, 9, 10, 11, 12, 13, 14, 15, 16 or 17) of the following compounds: 3-ethyl-2-hydroxy-2-cyclopenten-1-one; 5-methyl-2-furancarboxaldehyde; propanoic acid; 3-methyl-pyridine; 3-ethyl-pyridine; trimethyl-pyrazine; tiglic acid; 2-hydroxy-3-methyl-2-cyclopenten-1-one; 2-ethyl-phenol; 4-ethyl-phenol; 3-ethyl-phenol; 1-(1H-pyrrol-2-yl)-ethanone; 2-methoxy-4-vinylphenol; 2-acetyl-5-methylfuran; benzoic acid; triacetin; and maltol.

In some embodiments, the aerosol further comprises 1 or more (e.g. 1, 2, 3, 4, 5, 6, 7, 8, 9, 10, 11, 12, 13, 14, 15, 16, 17, 18, 19, 20, 21 or 22) of the following compounds: 3-ethyl-2-hydroxy-2-cyclopenten-1-one; 3-methyl-pyridine; butanoic acid; 3-ethyl-pyridine; benzaldehyde; 2-hydroxy-3-methyl-2-cyclopenten-1-one; acetophenone; 2-ethyl-phenol; 2-methoxy-phenol; furaneol; 4-ethyl-phenol; 3-ethyl-phenol; 2-methoxy-4-vinylphenol; 4-ethyl-2-methoxy-phenol; octanoic acid; 2,6-dimethoxy-phenol; 2-methyl-propanoic acid; 3-methyl-butanoic acid; 1-(4-methylphenyl)-ethanone; vanillin; pentanoic acid; and benzeneacetic acid.

In some embodiments, the aerosol further comprises 1 or more (e.g. 1, 2, 3, 4, 5, 6, 7, 8, 9, 10, 11, 12 or 13) of the following compounds: 3-ethyl-2-hydroxy-2-cyclopenten-1-one; 3-methyl-pyridine; butanoic acid; 3-ethyl-pyridine; 2-hydroxy-3-methyl-2-cyclopenten-1-one; acetophenone; 2-ethyl-phenol; furaneol; 4-ethyl-phenol; 3-ethyl-phenol; 2-methoxy-4-vinylphenol; 4-ethyl-2-methoxy-phenol; and 2,6-dimethoxy-phenol.

In some embodiments, the aerosol further comprises 1 or more (e.g. 1, 2, 3, 4, 5, 6, 7 or 8) of the following compounds: 3-ethyl-2-hydroxy-2-cyclopenten-1-one; 3-methyl-pyridine; 3-ethyl-pyridine; 2-hydroxy-3-methyl-2-cyclopenten-1-one; 2-ethyl-phenol; 4-ethyl-phenol; 3-ethyl-phenol; and 2-methoxy-4-vinylphenol.

### Aerosol free delivery system

The composition as described herein may used in aerosol-free delivery systems that delivers the composition to a user orally, nasally, transdermally or in another way without forming an aerosol, including but not limited to, lozenges, gums, patches, articles comprising inhalable powders, and oral products (e.g. smokeless oral products) such as oral tobacco which includes snus or moist snuff, wherein the at least one substance may or may not comprise nicotine.

Thus, in some embodiments the composition is a composition for use in an aerosol-free delivery system. Where the composition is for use in an aerosol-free delivery system, it may or may not comprise the key smoke markers described herein. This is because it may not be necessary for the smoke markers to be present for an FMC-like experience to be obtained when using the composition in an aerosol-free delivery system.

In some embodiments the invention provides a smokeless oral product comprising the composition described herein.

As used herein, the terms "oral product" and "smokeless oral product" may denote any smokeless product designed to be placed in the oral cavity of a user for a limited period of time, during which there is contact between the user's saliva and the product. These terms do not include non-combustible aerosol provision systems, such as tobacco heated products.

In some embodiments the composition is a composition for oral delivery of the compounds present in the composition.

The composition (which may be for oral delivery) may be provided in the form of chews, soluble strips, lozenges, gums, snus or moist snuff.

As used herein, the term "snuff" generally describes a class of smokeless oral product which typically comprises solid particles having a mass median particle size measured by sieve analysis of between 0.01 and 5 mm, such as between 0.01 and 3 mm, such as between 0.01 and 1.0 mm.

In some embodiments, the moisture content of the composition is less than about 16 wt% of the total smokeless oral product, such as less than about 12 wt%, less than about 10 wt%, less than about 5 wt%, less than about 3 wt%, less than about 2 wt%, or less than about 1 wt% by weight of the total smokeless oral product.

In some embodiments, the moisture content of the composition is at least about 20 wt% by weight of the total smokeless oral product, such as at least about 25 wt%, at least about 30 wt%, at least about 35 wt%, at least about 40 wt%, at least about 45 wt%, at least about 50 wt%, or at least about 60 wt% by weight of the smokeless oral product.

The composition can be provided to the user (e.g. in a smokeless oral product) in a portioned or a non-portioned format. Portioning the composition can reduce or eliminate the handling of the composition by the user, which can offer advantages in terms of better hygiene, convenience and/or ease of use.

In some embodiments, the smokeless oral product can be provided to the user in a portioned format. In some known portioned smokeless oral products, material is surrounded by a pouch. For example, a common method of providing moist snuff is to seal the tobacco material in a permeable pouch. A pouch holds the tobacco material in place, while at the same time allowing substances such as flavours and nicotine to diffuse through the pouch and into the mouth of the user for absorption through the user's mucous membranes.

In some embodiments, the smokeless oral product can be provided to the user in a non-portioned format. In one embodiment, the smokeless oral product is packaged in loose form in a container, such as a can, sachet or tin.

The product may be suitable for buccal administration, i.e. the product is configured for insertion in the mouth of a user, i.e. a product having dimensions making it possible to insert and accommodate said product in the mouth, preferably between the gum and the upper lip.

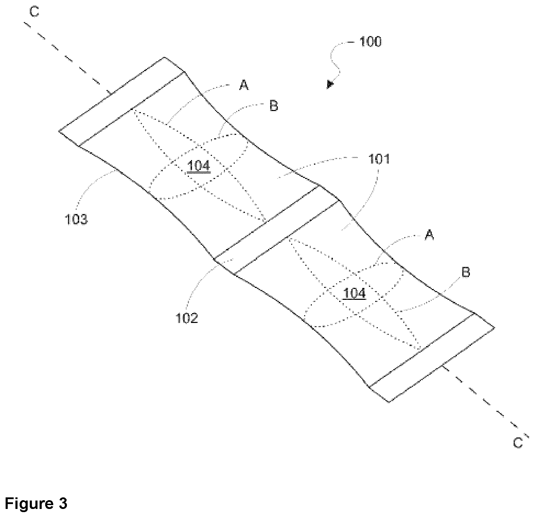

Figure 3 illustrates a perspective view of a smokeless oral product 100. The product 100 comprises at least two pouches 101, also referred to as pouch chambers, interconnected by means of a separation section 102. However, other products may comprise a single pouch, or may be a non-partitioned product.

Each of the pouches 101 encloses a composition according to the invention. Each of said pouches 101 has dimensions making it suitable for insertion into the mouth of the user (buccal administration), e.g. a length or width in the order of 1-4 cm. Further, two or more interconnected pouches 101 have combined dimensions likewise making them suitable for insertion into the mouth (buccal administration), such that the user may accommodate multiple interconnected pouches 101 in their mouth. For example, a single pouch 101 may contain an amount of a composition less than what would be commonly used, e.g. less than what causes an effect/medical response in most individuals. However, due to said pouches 101 being interconnected, the user may choose to dispense more than one pouch, e.g. two or three pouches, depending on their tolerance for the enclosed composition. Due to the interconnected nature, the pouches 101 may more easily be stored/accommodated in the mouth, e.g. without risk of losing/swallowing. In fact, a single pouch 101 may be too small to most individuals, whereas two or more interconnected pouches would constitute a preferred size.

The pouches 101 may be made of a water-permeable material 103 allowing liquid/water, or saliva of the mouth, to soak the composition enclosed by said pouches 101, thereby accelerating the release of components of said composition. Thus, the water-permeable material 103 allows water and components dissolved therein to pass through, such that said components may be dissolved in the liquid or absorbed by the user, e.g. into the blood vessels through the mucosa/gums. On the other hand, the water-permeable material 103 has a wet strength selected such that said material does not disintegrate once soaked with water/saliva, since such disintegration would cause the enclosed composition to enter the digestive system of the user, which may be harmful or not provide any effect at all. Preferably, the water-permeable material 103 is made of a textile or paper-like membrane.

A first cross-section A and a second cross-section B of the pouches 101 are highlighted by dashed lines, illustrating the inner volume/chamber 104 intended for accommodating the composition. Each of said pouches 101 are divided by a separation section 102, e.g. formed by welding opposing sidewalls of the water-permeable material 103 together, whereby said separation section 103 may be considered a welding section.

Dashed lines C indicate a possible continuation of the product 100, whereby said product 100 may comprise more than two interconnected pouches 101, and such that each of said pouches 101 are mutually interconnected to its neighbouring pouches by means of a separation section 102, and such that said pouches 101 extend in a row.

In some embodiments, the composition (which may be for oral delivery) further comprises one or more additives selected from sweeteners, taste modifiers, salts, buffering agents, colorants, oral care additives, preservatives, disintegration aids, emulsifiers, preservatives and antioxidants.

The aerosol-former material discussed above may be used as a humectant in the compositions which may be for oral delivery. The above disclosures (for example those regarding the compounds in Lists 1 to 4), in relation to the aerosol-generating material for use in aerosol generation apply equally to this aspect of the invention.

The above embodiments are to be understood as illustrative examples of the invention. It is to be understood that any feature described in relation to any one embodiment may be used alone, or in combination with other features described, and may also be used in combination with one or more features of any other of the embodiments, or any combination of any other of the embodiments. Furthermore, equivalents and modifications not described above may also be employed without departing from the scope of the invention, which is defined in the accompanying claims.

The various embodiments described herein are presented only to assist in understanding and teaching the claimed features. These embodiments are provided as a representative sample of embodiments only, and are not exhaustive and/or exclusive. It is to be understood that advantages, embodiments, examples, functions, features, structures, and/or other aspects described herein are not to be considered limitations on the scope of the invention as defined by the claims or limitations on equivalents to the claims, and that other embodiments may be utilised and modifications may be made without departing from the scope of the claimed invention. Various embodiments of the invention may suitably comprise, consist of, or consist essentially of, appropriate combinations of the disclosed elements, components, features, parts, steps, means, etc., other than those specifically described herein. In addition, this disclosure may include other inventions not presently claimed, but which may be claimed in future.

### Examples

### Example 1

The inventors have identified a range of compounds responsible for the overall sensory experience of smoking a traditional cigarette (sometimes called a factory made cigarette or FMC). The compounds were identified by collecting and analysing the smoke produced from burning cigarettes.

Analysis was performed using HTS-HRMS (High throughput screening coupled with high resolution mass spec) and GC-MS data. Specifically, compounds could be identified based on exact monoisotopic mass and isotopic pattern from HTS-HRMS data and from GC-MS mass spectra, as compared to reference MS libraries and reference compounds.

Human sensory assessment could be used to assign the individual chemicals to one of three categories or flavour dimensions, namely those responsible for the (1) sense, (2) taste, and (3) flavour of the cigarette smoke. Some compounds fall into more than one category or flavour dimension. For example, a single compound may be responsible for providing both a cigarette-like sense and taste.

For a true cigarette-like experience it is necessary for any substitute composition to contain compounds from each of these three categories. However, even compounds from one category can be enough to provide a composition having a cigarette-like sense, taste or flavour, or to increase the cigarette-like sense, taste or flavour of a given composition.

A machine learning model was created, trained and validated using more than 1,000 samples that were previously accessed by human sensory panels, to determine for each compound whether it would enhance one or more of the flavour dimensions. Using PLSR (partial least squares regression) models, compounds were found that noticeably contributed to each flavour dimension (i.e. (1) sense, (2) taste, and/or (3) flavour), in particular those compounds having a VIP (variable importance of projection) higher than 1. These compounds were termed as "chemical markers".

In total 324 chemical markers were identified as being associated with providing a cigarette-like experience. Over 250 chemical markers were found to enhance the cigarette-like sense, over 200 chemical markers were found to enhance the cigarette-like taste, and over 140 chemical markers were found to enhance the cigarette-like flavour.

Based on the chemical markers with the highest variable importance of projection scores, the 20 most important chemical markers for each flavour dimension were identified (termed the "key chemical markers"). These key chemical markers are listed in the table below.

**Table 4 - Key chemical markers for each flavour dimension**

| **Sense** | **Taste** | **Flavour** |
|---|---|---|
| Nicotine | Nicotine | 2-methoxy-phenol |
| 2-hydroxy-4-methyl-2-cyclopenten-1-one | Pseudooxynicotine and/or oxynicotine | p-Cresol |
| Quinic acid | Lipid peroxidation inhibitor 1 | Furaneol |
| Sucrose | Scopoletin | Indole |
| N-(1-deoxy-1-fructosyl)proline | N-(1-deoxy-1-fructosyl)proline | 3-methyl-pentanoic acid |
| 3,4-dimethyl-2(3H)-furanone | Sucrose | Butanoic acid |
| Lipid peroxidation inhibitor 1 | Type I sucrose ester - GV - I0 | 3-methyl-butanoic acid |
| Pseudooxynicotine and/or oxynicotine | Type I sucrose ester - GIV - I0 | D-limonene |
| 2-ethyl-quinoxaline | Chlorogenic acid | Acetophenone |
| 1,2,3,4,5,6-hexahydro-7H-cyclopenta[b]pyridin-7-one | 1,2,3,4,5,6-Hexahydro-7H-cyclopenta[b]pyridin-7-one | 1-(4-methylphenyl)-ethanone |
| 2-ethyl-pyridine | L-alpha-amino-1H-pyrrole-1-hexanoic acid | Vanillin |
| Scopoletin | Retinol | 2,6-dimethoxy-phenol |
| 2-ethyl-5-methyl-pyridine | Quinic acid | Benzaldehyde |
| Rutin | Type I sucrose ester - GIII - I0 | Benzeneacetic acid |
| Retinol | Rutin | 2-methyl-butanoic acid |
| 2-methyl-3-butyl-pyrazine | Megastigmatrienone | Pentanoic acid |
| Megastigmatrienone | 2-ethyl-5-methyl-pyridine | Hexanoic acid |
| 3,5-di(1,1-dimethylethyl)-4-hydroxy-benzaldehyde | 16-hydroxy-hexadecanoicacid | Acetic acid |
| L-alpha-amino-1H-pyrrole-1-hexanoic acid | Type III sucrose ester - GV - I0 | 2-methyl-propanoic acid |
| Solanesol | Ferulic acid | Octanoic acid |

### Example 2

Four different tobacco extracts were formed, as set out below. The extracts were formed from either flue-cured Virginia tobacco (hereinafter called C38 tobacco) or a blend of flue-cured Virginia, air-cured burley and sun-cured oriental tobacco (hereinafter called Paris tobacco), using the SFE (supercritical fluid extraction) or SDE (simultaneous distillation extraction) process described below.

### Simultaneous distillation extraction

300g of ground tobacco was mixed with 2800 mL of water. This solution, with a pH of 6, included glass beads to prevent bumping. The solution was heated to 120 °C and the volatile and semi-volatile compounds were collected in 15 mL of pentane: ethyl ether (2:1, v/v) solution maintained at -8 °C. After 6 hours of extraction, the pentane:ethyl ether solution containing volatile and semi-volatile compounds was collected and dried using an open package column containing sodium sulfate anhydrous, filtered throughout Whatman filter paper (n°2) and concentrated under controlled conditions (magnetic stirring: 200 rpm; water bath 40 °C) to prevent the loss of volatiles and exchange the pentane:ethyl ether solvent with ethanol.

### Supercritical fluid extraction

260g of ground tobacco was placed in a 1L vessel and placed in the equipment for extraction using supercritical CO₂ (1 kg/hr) as solvent. No co-solvent was used throughout the extraction process. The pressure of system was set at 100 bar and a first fraction extracted for a period of 1 hour (the volatile extract). The pressure was then increased to 300 bar and a second fraction extracted for a period of 1.5 hours (the non-volatile extract). The temperature was maintained at 60 °C throughout the extraction process. The two fractions or extracts were collected in two containers: one for the volatile fraction/extract and one for the non-volatile fraction/extract. Only the non-volatile fraction/extract was considered as the final extract. The (non-volatile) extract was collected in a bottle and kept in a refrigerated environment.

**Table 5 - Extracts**

| **Extract** | **Tobacco type** | **Extraction procedure** |
|---|---|---|
| 1 | C38 | SFE |
| 2 | Paris | SFE |
| 3 | C38 | SDE |
| 4 | Paris | SDE |

Each of these extracts was chemically analysed to determine which of the chemical markers identified in Example 1 was present. The results are set out below.

**Table 6 - Extract analysis**

| | **Number of chemical markers (% of the total number of chemical markers identified in Example 1)** | | |
|---|---|---|---|
| **Extract** | **sensory attributes** | **taste profile** | **flavour profile** |
| 1 (C38 SFE) | 110 (42%) | 110 (53%) | 40 (27%) |
| 2 (Paris SFE) | 82 (32%) | 83 (40%) | 36 (24%) |
| 3 (C38 SDE) | 51 (20%) | 61 (30%) | 22 (15%) |
| 4 (Paris SDE) | 59 (23%) | 68 (33%) | 28 (19%) |

The extracts were also analysed to determine whether the key chemical markers identified in Example 1 were present. The results are set out in the tables below.

**Table 7 - Extract analysis - key chemical markers (sense)**

| | **Present in Extract** | | | |
|---|---|---|---|---|
| **Compound (key sense marker)** | **1** | **2** | **3** | **4** |
| Nicotine | Yes | Yes | - | - |
| 2-hydroxy-4-methyl-2-cyclopenten-1-one | - | - | - | - |
| Quinic acid | Yes | - | - | - |
| Sucrose | Yes | - | - | - |
| N-(1-deoxy-1-fructosyl)proline | Yes | - | - | - |
| 3,4-dimethyl-2(3H)-furanone | Yes | Yes | Yes | Yes |
| Lipid peroxidation inhibitor 1 | - | - | - | - |
| Pseudooxynicotine and/or oxynicotine | Yes | Yes | Yes | Yes |
| 2-ethyl-quinoxaline | - | - | - | - |
| 1,2,3,4,5,6-hexahydro-7H-cyclopenta[b]pyridin-7-one | Yes | Yes | - | - |
| 2-ethyl-pyridine | Yes | Yes | - | - |
| Scopoletin | Yes | Yes | - | - |
| 2-ethyl-5-methyl-pyridine | Yes | Yes | - | - |
| Rutin | Yes | - | - | - |
| Retinol | - | Yes | Yes | Yes |
| 2-methyl-3-butyl-pyrazine | Yes | Yes | Yes | Yes |
| Megastigmatrienone | Yes | Yes | Yes | Yes |
| 3,5-di(1,1-dimethylethyl)-4-hydroxy-benzaldehyde | - | - | - | - |
| L-alpha-amino-1H-pyrrole-1-hexanoic acid | Yes | - | Yes | Yes |
| Solanesol | Yes | Yes | - | - |
| **Total** | **15** | **11** | **6** | **6** |

**Table 8 - Extract analysis - key chemical markers (taste)**

| | **Present in Extract** | | | |
|---|---|---|---|---|
| **Compound (key taste marker)** | **1** | **2** | **3** | **4** |
| Nicotine | Yes | Yes | - | - |
| Pseudooxynicotine and/or oxynicotine | Yes | Yes | Yes | Yes |
| Lipid peroxidation inhibitor 1 | - | - | - | - |
| Scopoletin | Yes | Yes | - | - |
| N-(1-deoxy-1-fructosyl)proline | Yes | - | - | - |
| Sucrose | Yes | - | - | - |
| Type I sucrose ester - GV - I0 | - | Yes | - | Yes |
| Type I sucrose ester - GIV - I0 | - | Yes | - | - |
| Chlorogenic acid | Yes | - | - | - |
| 1,2,3,4,5,6-Hexahydro-7H-cyclopenta[b]pyridin-7-one | Yes | Yes | - | - |
| L-alpha-amino-1H-pyrrole-1-hexanoic acid | Yes | - | Yes | Yes |
| Retinol | - | Yes | Yes | Yes |
| Quinic acid | Yes | - | - | - |
| Type I sucrose ester - GIII - I0 | - | Yes | - | - |
| Rutin | Yes | - | - | - |
| Megastigmatrienone | Yes | Yes | Yes | Yes |
| 2-ethyl-5-methyl-pyridine | Yes | Yes | - | - |
| 16-hydroxy-hexadecanoicacid | Yes | Yes | - | - |
| Type III sucrose ester - GV - I0 | - | Yes | Yes | Yes |
| Ferulic acid | Yes | - | - | - |
| **Total** | **14** | **12** | **5** | **6** |

**Table 9 - Extract analysis - key chemical markers (flavour)**

| | **Present in Extract** | | | |
|---|---|---|---|---|
| **Compound (key flavour marker)** | **1** | **2** | **3** | **4** |
| 2-methoxy-phenol | - | - | Yes | Yes |
| p-Cresol | - | - | - | - |
| Furaneol | - | - | - | - |
| Indole | - | - | - | - |
| 3-methyl-pentanoic acid | Yes | Yes | - | Yes |
| Butanoic acid | - | - | - | - |
| 3-methyl-butanoic acid | - | Yes | Yes | Yes |
| D-limonene | - | - | Yes | - |
| Acetophenone | - | - | - | - |
| 1-(4-methylphenyl)-ethanone | - | Yes | Yes | Yes |
| Vanillin | - | - | - | - |
| 2,6-dimethoxy-phenol | - | - | - | - |
| Benzaldehyde | - | - | Yes | Yes |
| Benzeneacetic acid | Yes | Yes | - | - |
| 2-methyl-butanoic acid | Yes | Yes | Yes | Yes |
| Pentanoic acid | Yes | Yes | - | Yes |
| Hexanoic acid | Yes | Yes | - | Yes |
| Acetic acid | Yes | Yes | Yes | Yes |
| 2-methyl-propanoic acid | - | Yes | - | Yes |
| Octanoic acid | - | - | Yes | Yes |
| **Total** | **6** | **9** | **8** | **11** |

**Table 10 - Extract analysis - key chemical markers (summary)**

| | **Extract 1 (C38 SFE)** | **Extract 2 (Paris SFE)** | **Extract 3 (C38 SDE)** | **Extract 4 (Paris SDE)** |
|---|---|---|---|---|
| Total number of key sensory markers | **15** | **11** | **6** | **6** |
| Total number of key taste markers | **14** | **12** | **5** | **6** |
| Total number of key flavour markers | **6** | **9** | **8** | **11** |
| Total number of key chemical markers | **35** | **32** | **19** | **23** |

As shown above, the SFE extracts were found to generally have higher numbers of both the overall chemical markers and the key chemical markers. These extracts would therefore be expected to provide a more cigarette-like experience than the SDE extracts, when used to provide the flavour of a composition, for example in an aerosol-generating composition which could be used in a non-combustible aerosol provision system. Of the two SFE extracts, the C38 extract (Extract 1) has a slightly higher number of the key chemical markers. However, the Paris extract (Extract 2) contains a higher number of key flavour markers. Flavour could be considered to be the most important flavour dimension in a non-combustible aerosol provision system (e.g. in a vaping device), due to the volatility of the chemical markers or compounds associated with this flavour dimension. As such, Extract 2 might be expected to provide an overall improved cigarette-like experience than Extract 1.

### Example 3

The extracts set out in Example 2 were then incorporated into a liquid composition and tested by five panellists, all of whom were current smokers.

The liquid formulations tested utilizing the extracts were as follows:
- Sample 1941 contained C38 SFE extract (2.46% w/w) in 60:40 base with 5% benzyl alcohol, and nicotine at 18.8mg/mL
- Sample 1942 contained Paris SFE extract (3.30% w/w) in 60:40 base with 5% benzyl alcohol, and nicotine at 18.9mg/mL
- Sample 1947 contained C38 SDE extract (1.67% w/w) in 60:40 base with 5% benzyl alcohol at 18.5mg/mL
- Sample 1948 contained Paris SDE extract (1.67% w/w) in 60:40 base with 5% benzyl alcohol at 18.5mg/mL

The liquids were assessed using a vaping device (Vuse Go) adapted with a cigarette-like mouthpiece, and compared to a benchmark commercially-available flavour (Creamy Tobacco) in the same vaping device (Vuse Go).

The panellists were asked to rate the cigarette-like flavour of the sample provided on a scale of 1 to 9, with a score of 1 meaning that the sample has a flavour nothing like a cigarette, 5 meaning that the sample has a flavour somewhat cigarette-like, and 9 meaning that the sample has a flavour identical to a cigarette. The average results were as follows:

**Table 11 - Cigarette-like flavour results**

| **Sample** | **Extract** | **Average result** |
|---|---|---|
| 1941 | 1 (C38 SFE) | 3.4 |
| 1942 | 2 (Paris SFE) | 5.2 |
| 1947 | 3 (C38 SDE) | 2.8 |
| 1948 | 4 (Paris SDE) | 3.6 |

The results are in good accordance with the predications made in Example 2. In particular, the flavor of Extract 2 was found to be the best. Extract 4 was found to outperform Extract 1 slightly. This may be due to the higher number of key flavour markers found in Extract 4 compared to Extract 1 (11 vs. 6), despite the total number of key chemical markers being less for Extract 4 (23 vs 35).

Participants were asked to rank all tested samples, including the control, on the attributes of "cigarette-like flavour", "cigarette-like sensation" and "cigarette-like product preference" after having tried the full set of samples individually.

**Table 12 - Sample rankings**

| **Sample** | **Cigarette-like flavour** | **Cigarette-like sensation** | **Cigarette-like product preference** |
|---|---|---|---|
| 1941 | 3.8 | 3.8 | 3.6 |
| 1942 | 4.4 | 4.4 | 4.4 |
| 1947 | 2.6 | 3.2 | 2.6 |
| 1948 | 3 | 2.2 | 3 |
| Control | 1.2 | 1.4 | 1.4 |

The results are in good accordance with the predications made in Example 2.

In particular, the SFE extracts would found to be preferred over the SDE extracts, with the Paris extract outperforming the C38 extract. Importantly, all of the test samples were significantly better than the control.

### Example 4

Each of the extracts in Table 5 was chemically analysed to determine all of the compounds present therein. Separately, cigarettes comprising the Paris and C38 tobacco were burnt, and the compounds present in the smoke identified by collecting and analysing the smoke.

Analysis was performed using HTS-HRMS (High throughput screening coupled with high resolution mass spec) and GC-MS data. Specifically, compounds could be identified based on exact monoisotopic mass and isotopic pattern from HTS-HRMS data and from GC-MS mass spectra, as compared to reference MS libraries and reference compounds.

For each extract over 100 compounds (smoke markers) were identified as being present in the smoke but not in the extract. Of these, certain specific compounds were identified as being acceptable for use under current toxicity regulations. These compounds (the "key smoke markers") are listed in the table below.

**Table 13 - Key smoke markers**

| | **Present in smoke but absent in extract** | | | |
|---|---|---|---|---|
| **Compound (chemical marker)** | **Extract 1** | **Extract 2** | **Extract 3** | **Extract 4** |
| 3-Ethyl-2-hydroxy-2-cyclopenten-1-one | Yes | Yes | Yes | Yes |
| 5-Methyl-2-furancarboxaldehyde | Yes | Yes | - | - |
| Propanoic acid | Yes | Yes | - | - |
| 3-Methyl-pyridine | Yes | Yes | Yes | Yes |
| Butanoic acid | Yes | Yes | Yes | Yes |
| 3-Ethyl-pyridine | Yes | Yes | Yes | Yes |
| Trimethyl-pyrazine | - | Yes | - | Yes |
| Benzaldehyde | Yes | Yes | - | - |
| Tiglic acid | - | Yes | Yes | Yes |
| 2-Hydroxy-3-methyl-2-cyclopenten-1-one | Yes | Yes | Yes | Yes |
| Acetophenone | Yes | Yes | Yes | Yes |
| 2-Ethyl-phenol | Yes | Yes | Yes | Yes |
| 2-Methoxy-phenol | Yes | Yes | - | - |
| Furaneol | Yes | Yes | Yes | Yes |
| 4-Ethyl-phenol | Yes | Yes | Yes | Yes |
| 3-Ethyl-phenol | Yes | Yes | Yes | Yes |
| 1-(1H-pyrrol-2-yl)-ethanone | - | Yes | Yes | Yes |
| 2-Methoxy-4-vinylphenol | Yes | Yes | Yes | Yes |
| 4-Ethyl-2-methoxy-phenol | Yes | Yes | Yes | Yes |
| Octanoic acid | Yes | Yes | - | - |
| 2,6-Dimethoxy-phenol | Yes | Yes | Yes | Yes |
| 2-Methyl-propanoic acid | Yes | - | Yes | - |
| 3-Methyl-butanoic acid | Yes | - | - | - |
| D-Limonene | Yes | - | - | - |
| 2-Acetyl-5-methylfuran | Yes | - | - | - |
| 1-(4-Methylphenyl)-ethanone | Yes | - | - | - |
| Vanillin | Yes | - | Yes | - |
| Pentanoic acid | - | - | Yes | - |
| Benzoic acid | - | - | Yes | Yes |
| Triacetin | - | - | Yes | Yes |
| Benzeneacetic acid | - | - | Yes | Yes |
| Maltol | - | - | Yes | Yes |
| Nicotine | - | - | Yes | Yes |

Some of the smoke markers are also key sense, taste and/or flavour markers. Where a marker is not present in the extract itself it may still be present in the smoke. For example, Extracts 1 and 2 contain nicotine (a key sense and taste marker) in the extract, but Extracts 3 and 4 did not. However, the smoke from Extracts 3 and 4 did contain nicotine. This further demonstrates that nicotine is a key marker, since it is either present in the extract or in the smoke, meaning that it will almost inevitably be present in any composition creating a cigarette-like experience.

Some of the other smoke markers are also similarly either present in each of the extracts (e.g. as a key flavour marker) or the smoke. For example, 2-methyl-propanoic acid is present in Extracts 2 and 4, but not Extracts 1 and 3. However, 2-methyl-propanoic acid is present in the smoke produced from the tobacco of Extracts 1 and 3.

## Claims

1. A composition comprising at least three of the following compounds: nicotine; 2-hydroxy-4-methyl-2-cyclopenten-1-one; quinic acid; sucrose; N-(1-deoxy-1-fructosyl)proline; 3,4-dimethyl-2(3H)-furanone; lipid peroxidation inhibitor 1; pseudooxynicotine and/or oxynicotine; 2-ethyl-quinoxaline; 1,2,3,4,5,6-hexahydro-7H-cyclopenta[b]pyridin-7-one; 2-ethyl-pyridine; scopoletin; 2-ethyl-5-methyl-pyridine; rutin; retinol; 2-methyl-3-butyl-pyrazine; megastigmatrienone; 3,5-di(1,1-dimethylethyl)-4-hydroxy-benzaldehyde; L-alpha-amino-1H-pyrrole-1-hexanoic acid; and solanesol.

2. The composition of claim 1, wherein the total number of chemical compounds present in the composition is less than about 200.

3. A composition comprising (i) a tobacco extract and (ii) at least one (e.g. at least 2 or at least 3) of the following compounds: nicotine; 2-hydroxy-4-methyl-2-cyclopenten-1-one; quinic acid; sucrose; N-(1-deoxy-1-fructosyl)proline; 3,4-dimethyl-2(3H)-furanone; lipid peroxidation inhibitor 1; pseudooxynicotine and/or oxynicotine; 2-ethyl-quinoxaline; 1,2,3,4,5,6-hexahydro-7H-cyclopenta[b]pyridin-7-one; 2-ethyl-pyridine; scopoletin; 2-ethyl-5-methyl-pyridine; rutin; retinol; 2-methyl-3-butyl-pyrazine; megastigmatrienone; 3,5-di(1,1-dimethylethyl)-4-hydroxybenzaldehyde; L-alpha-amino-1H-pyrrole-1-hexanoic acid; and solanesol.

4. The composition of any preceding claim, comprising at least 4, at least 5, at least 6, at least 8, at least 10, at least 15 or all of the listed compounds.

5. The composition of any preceding claim, wherein the composition comprises at least nicotine.

6. The composition of any preceding claim, wherein the composition comprises at least nicotine; 2-hydroxy-4-methyl-2-cyclopenten-1-one; and quinic acid.

7. The composition of any preceding claim, further comprising one or more of the following compounds: nicotine; pseudooxynicotine and/or oxynicotine; lipid peroxidation inhibitor 1; scopoletin; N-(1-deoxy-1-fructosyl)proline; sucrose; Type I sucrose ester - GV - I0; Type I sucrose ester - GIV - I0; chlorogenic acid; 1,2,3,4,5,6-Hexahydro-7H-cyclopenta[b]pyridin-7-one; L-alpha-amino-1H-pyrrole-1-hexanoic acid; retinol; quinic acid; Type I sucrose ester - GIII - I0; rutin; megastigmatrienone; 2-ethyl-5-methyl-pyridine; 16-hydroxy-hexadecanoicacid; Type III sucrose ester - GV - I0; and ferulic acid.

8. The composition of any preceding claim, further comprising one or more of the following compounds: 2-methoxy-phenol; p-cresol; furaneol; indole; 3-methyl-pentanoic acid; butanoic acid; 3-methyl-butanoic acid; D-limonene; acetophenone; 1-(4-methylphenyl)-ethanone; vanillin; 2,6-dimethoxy-phenol; benzaldehyde; benzeneacetic acid; 2-methyl-butanoic acid; pentanoic acid; hexanoic acid; acetic acid; 2-methyl-propanoic acid; and octanoic acid.

9. The composition of any preceding claim, further comprising one or more of the following compounds: 3-ethyl-2-hydroxy-2-cyclopenten-1-one; 5-methyl-2-furancarboxaldehyde; propanoic acid; 3-methyl-pyridine; butanoic acid; 3-ethyl-pyridine; trimethyl-pyrazine; benzaldehyde; tiglic acid; 2-hydroxy-3-methyl-2-cyclopenten-1-one; acetophenone; 2-ethyl-phenol; 2-methoxy-phenol; furaneol; 4-ethyl-phenol; 3-ethyl-phenol; 1-(1H-pyrrol-2-yl)-ethanone; 2-methoxy-4-vinylphenol; 4-ethyl-2-methoxy-phenol; octanoic acid; 2,6-dimethoxy-phenol; 2-methyl-propanoic acid; 3-methyl-butanoic acid; D-limonene; 2-acetyl-5-methylfuran; 1-(4-methylphenyl)-ethanone; vanillin; pentanoic acid; benzoic acid; triacetin; benzeneacetic acid; maltol; and nicotine.

10. The composition of any preceding claim, wherein at least one of the selected compounds is a purified compound; or wherein each of the compounds are purified compounds.

11. The composition of any preceding claim, wherein the composition comprises a total of from about 0.0001 to about 2 wt% of the compounds listed in claim 1.

12. The composition of any preceding claim, wherein the composition is an aerosol-generating composition; or wherein the composition is a composition for aerosol-free delivery of the compounds.

13. A consumable for use in a non-combustible aerosol provision device, the consumable comprising the composition of any preceding claim.

14. A non-combustible aerosol provision system comprising the consumable of claim 13 and a non-combustible aerosol provision device, the non-combustible aerosol provision device comprising an aerosol-generation device arranged to generate aerosol from the consumable when the consumable is used with the non-combustible aerosol provision device.

15. A method of forming the composition of any of claims 1-12, comprising combining the compounds and, if present, the tobacco extract.
